# FASCICULE DE BREVET EUROPEEN

(11) **EP 1 633 735 B1**
(45) Date de publication et mention de la délivrance du brevet: **30.11.2011**
(21) Numéro de dépôt: 04742661.4
(22) Date de dépôt: 06.05.2004
(51) Int. Cl.: C07D 401/04, C07D 401/14, C07D 471/04, C07D 491/04, C07D 495/04, C07D 295/12, A61K 31/436, A61K 31/435, A61K 31/506, A61K 31/44, A61K 31/495, A61P 25/00

(54) **DERIVES DE PIPERIDINYL- ET PIPERAZINYL-ALKYLCARBAMATES, LEURS PROCEDES DE PREPARATION ET LEUR APPLICATION EN THERAPEUTIQUE**
DERIVATE VON PIPERIDINYL- UND PIPERAZINYLALKYLCARBAMATEN, VERFAHREN ZU DEREN HERSTELLUNG UND DEREN VERWENDUNG IN THERAPEUTIKA
DERIVATIVES OF PIPERIDINYL- AND PIPERAZINYL-ALKYL CARBAMATES, PREPARATION METHODS THEREOF AND APPLICATION OF SAME IN THERAPEUTICS

(30) Priorité: 07.05.2003 FR 0305540
(43) Date de publication de la demande: 15.03.2006
(73) Titulaire: SANOFI, 75013 Paris (FR)
(72) Inventeur: ABOUABDELLAH, Ahmed, F-94320 Thiais (FR); BURNIER, Philippe, F-78600 Maisons-Laffitte (FR); HOORNAERT, Christian, F-92160 Antony (FR); JEUNESSE, Jean, F-75018 Paris (FR); PUECH, Frédéric, F-78170 La Celle Saint-Cloud (FR)
(74) Mandataire: Le Coupanec, Pascale A.M.P.
(86) Numéro de dépôt international: PCT/FR2004/001102
(87) Numéro de publication internationale: WO 2004/099176

(56) Documents cités:
- WO-A-01/64632
- WO-A-98/43636
- US-A- 4 539 323

## Description

### Dérivés de pipéridinyl- et pipérazinyl-alkylcarbamates, leur préparation et leur application en thérapeutique

L'invention a pour objet des dérivés de pipéridinyl- et pipérazinyl-alkylcarbamates, leur préparation et leur application en thérapeutique,

Les documents de l'art antérieur se rapportent à des composés présentant une haute affinité pour les récepteurs cannabinoïdes, particulièrement les récepteurs CB1. WO01/64632 décrit des dérivés d'azetidine comme antagonistes des récepteurs cannabinoïdes, WO98/43636 décrit des compositions pharmaceutiques contenant des dérivés de N-pipéridino-3-pyrazolecarboxamide utilisé comme antagonistes des récepteurs CB1. US 4,539,323 décrit des dérivés de pipéridine utiles comme vasodilatateurs et hypotensifs.

Les composés de l'invention répondent à la formule générale (I) : 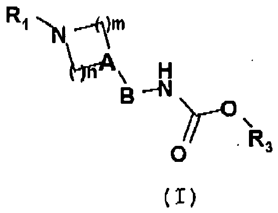 dans laquelle
A représente un atome d'azote ou un groupe CR₂ dans lequel R₂ représente un atome d'hydrogène, de fluor ou un groupe hydroxyle, cyano, trifluorométhyle, C₁₋₆-alkyle, C₁₋₆-alcoxy ;
n représente un nombre entier égal à 2 ou 3 et m représente un nombre entier égal à 2 lorsque A représente un atome d'azote ;
n représente un nombre entier égal à 1, 2 ou 3 et m un nombre entier égal à 1 ou 2 lorsque A représente un groupe CR₂ ;
B représente une liaison covalente ou un groupe C₁₋₈-alkylène R₁ représente un groupe choisi parmi un phényle, pyridinyle, pyridazinyle, pyrimidinyle, pyrazinyle, triazinyle, oxazolyle, isoxazolyle, thiazolyle, isothiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazopyrimidinyle, thiénopyrimidinyle, benzofuranyle, benzothiényle, benzimidàzolyle, benzoxazolyle, benzisoxazolyle, benzothiazolyle, benzisothiazolyle, indazolyle, pyrrolopyridinyle, furopyridinyle, dihydrofuropyridinyle, thiénopyridinyle, dihydrothiénopyridinyle, imidazopyridinyle, pyrazolopyridinyle, oxazolopyridinyle, isoxazolopyridinyle, thiazolopyridinyle ;
le groupe R₁ étant éventuellement substitué par un ou plusieurs groupes R' et/ou R" ;
R' représente un atome d'halogène ou un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃-7-cycloalkyl, C₃₋₇-cycloalkyle-C₁₋₆-alkylène, azétidinyle, pipéridinyle, pyrrolidinyle, morpholinyle, pipérazinyle, azépinyle, NH₂, NHR₆, NR₆R₇, NR₆COR₇, NR₆SO₂R₇, COR₆, CO₂R₆, SO₂R₆, SO₂NR₆R₇ ou -O-(C₁-₆-alkylène)-O- ;
R" représente un phényle, imidazolyle, pyridinyle, pyridazinyle, pyrazinyle ou pyrimidinyle;
le ou les groupes R" étant éventuellement substitués par un ou plusieurs groupes R' identiques ou différents l'un de l'autre ;
R₃ représente un groupe de formule générale CHR₄CONHR₅ dans laquelle
R₄ représente un atome d'hydrogène ou un groupe C₁₋₆-alkyle et
R₅ représente un atome d'hydrogène ou un groupe C₁₋₆-alkyle, C₃₋₇-cycloalkyle, C₃₋₇-cycloalkyle-C₁₋₆-alkylène ;
R₆ et R₇ représentent indépendamment l'un de l'autre un groupe C₁₋₆-alkyle.

Parmi les composés de formule générale (I), un premier sous-groupe de composés est constitué des composés pour lesquels :
A représente un atome d'azote ; et/ou
n représente un nombre entier égal à 2 ou 3 et m représente un nombre entier égal à 2 ; et/ou
B représente un groupe C₁₋₈-alkylène, plus
particulièrement un éthyle ou propyle ; et/ou
R₁ représente un groupe choisi parmi phényle, pyridinyle, pyrimidinyle, thiadiazolyle, naphtalènyle ;
le groupe R₁ étant éventuellement substitué par un ou plusieurs groupes R' et/ou R" ; et/ou
R' représente un atome d'halogène, plus particulièrement un chlore, ou un groupe nitro ou C₁₋₆-fluoroalkyle, plus particulièrement un trifluorométhyle ; et/ou
R" représente un phényle éventuellement substitué par un ou plusieurs groupes, identiques ou différents l'un de l'autre, choisis parmi un atome d'halogène, plus particulièrement un chlore, ou un groupe cyano, C₁₋₆-alcoxy, plus particulièrement un méthoxy, C₁₋₆-fluoroalcoxy, plus particulièrement un trifluorométhoxy ; et/ou R₃ représente un groupe de formule générale CHR₄CONHR₅ dans laquelle
R₄ représente un atome d'hydrogène et
R₅ représente un atome d'hydrogène ou un groupe C₁₋₆-alkyle, plus particulièrement un méthyle ou une éthyle, C₃₋₇-cycloalkyle, plus particulièrement un cyclopropyle, C₃₋₇-cycloalkyle-C₁₋₆-alkylène, plus particulièrement un cyclopropyle-méthylène.

Parmi les composés de formule générale (I), un second sous-groupe de composés est constitué des composés pour lesquels :
A représente un groupe CR₂ dans lequel R₂ représente un atome d'hydrogène, de fluor ou un groupe hydroxyle ;
et/ou
m représente un nombre entier égal à 1 ou 2 et n représente un nombre entier égal à 1 ou 2 ; et/ou
B représente une liaison covalente ou un groupe C₁₋₄-alkylène, plus particulièrement un méthyle, éthyle ou n-propyle ; et/ou
R₁ représente un groupe choisi parmi phényle, pyridinyle, pyridazinyle, pyrimidinyle, pyrazinyle, thiazolyle, quinolinyle, isoquinolinyle, phtalazinyle, quinazolinyle, quinoxalinyle, naphthyridinyle, furopyridinyle, thiénopyrimidinyle, imidazopyrimidinyle, benzothiazolyle, benzimidazolyle, benzoxazolyle ;
le groupe R₁ étant éventuellement substitué par un ou plusieurs groupes R' et/ou R" ; et/ou
R' représente un atome d'halogène, plus particulièrement un fluor, un chlore ou un brome, ou un groupe cyano, C₁₋₆-alkyle, plus particulièrement un méthyle, éthyle, n-propyle, isobutyle, un C₁₋₆-alcoxy, plus particulièrement un méthoxy, C₁₋₆-fluoroalkyle, plus particulièrement un trifluorométhyle, C₁₋₆-fluoroalcoxy, plus particulièrement un trifluorométhoxy, C₃₋₇-cycloalkyle, plus particulièrement un cyclopropyle ou cyclopentyle, pyrrolidinyle, NH₂, NR₆R₇, COR₆ ; et/ou
R" représente un phényle, imidazolyle, ou pyridinyle;
le ou les groupes R" étant éventuellement substitués par un ou plusieurs groupes R' identiques ou différents l'un de l'autre, plus particulièrement par un ou plusieurs atomes de chlore ou de fluor ; et/ou
R₃ représente un groupe de formule générale CHR₄CONHR₅ dans laquelle
R₄ représente un atome d'hydrogène ou un groupe C₁₋₆-alkyle, plus particulièrement un méthyle, et
R₅ représente un atome d'hydrogène ou un groupe C₁₋₆-alkyle, plus particulièrement un méthyle ou un éthyle, C₃₋₇-cycloalkyle, plus particulièrement un cyclopropyle, C₃₋₇-cycloalkyle-C₁₋₆-alkylène, plus particulièrement un cyclopropyle-méthylène ; et/ou
R₆ et R₇ représentent indépendamment l'un de l'autre un groupe C₁₋₆-alkyle, plus particulièrement un méthyle.

Parmi les composés de formule générale (I), un troisième sous-groupe de composés est constitué des composés pour lesquels :
A représente un groupe CR₂ dans lequel R₂ représente un atome d'hydrogène ; et/ou
m est égal à 2 et n est égal à 2 ; et/ou
B représente un groupe éthyle ; et/ou
R₁ représente un groupe choisi parmi phényle, pyridinyle, pyridazinyle, pyrimidinyle, pyrazinyle, thiazolyle, quinolinyle, isoquinolinyle, phtalazinyle, quinazolinyle, quinoxalinyle, naphthyridinyle, furopyridinyle, thiénopyrimidinyle, imidazopyrimidinyle, benzothiazolyle, benzimidazolyle, benzoxazolyle ;
le groupe R₁ étant éventuellement substitué par un ou plusieurs groupes R' et/ou R" ; et/ou
R' représente un atome d'halogène, plus particulièrement un fluor, un chlore ou un brome, ou un groupe cyano, C₁₋₆-alkyle, plus particulièrement un méthyle, éthyle, n-propyle, isobutyle, un C₁₋₆-alcoxy, plus particulièrement un méthoxy, C₁₋₆-fluoroalkyle, plus particulièrement un trifluorométhyle, C₁₋₆-fluoroalcoxy, plus particulièrement un trifluorométhoxy, C₃₋₇-cycloalkyle, plus
particulièrement un cyclopropyle ou cyclopentyle, pyrrolidinyle, NH₂, NR₆R₇, COR₆ ; et/ou
R" représente un phényle, imidazolyle, ou pyridinyle;
le ou les groupes R" étant éventuellement substitués par un ou plusieurs groupes R' identiques ou différents l'un de l'autre, plus particulièrement par un ou plusieurs atomes de chlore ou de fluor ; et/ou
R₃ représente un groupe de formule générale CHR₄CONHR₅ dans laquelle
R₄ représente un atome d'hydrogène et
R₅ représente un atome d'hydrogène ou un groupe C₁₋₆-alkyle, plus particulièrement un méthyle ou un éthyle ; et/ou
R₆ et R₇ représentent indépendamment l'un de l'autre un groupe C₁₋₆-alkyle, plus particulièrement un méthyle.

L'invention a également pour objet, parmi les composés de formule générale (I), des composés répondant à la formule générale (I') : 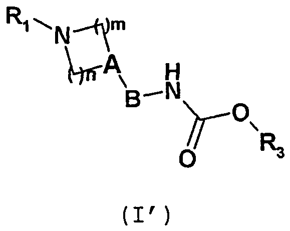 dans laquelle
A représente un atome d'azote ou un groupe CR₂ dans lequel R₂ représente un atome d'hydrogène, de fluor ou un groupe hydroxyle, cyano, trifluorométhyle, C₁₋₅-alkyle, C₁₋₅-alcoxy ;
n représente un nombre entier égal à 2 ou 3 et m représente un nombre entier égal à 2 lorsque A représente un atome d'azote ;
n représente un nombre entier égal à 1, 2 ou 3 et m un nombre entier égal à 1 ou 2 lorsque A représente un groupe CR₂ ;
B représente une liaison covalente ou un groupe C₁₋₈-alkyléne ;
R₁ représente un groupe choisi parmi un phényle, pyridinyle, pyridazinyle, pyrimidinyle, pyrazinyle, triazinyle, thiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazolylpyrimidinyle, thiénopyrimidinyle, benzofuranyle, benzothiényle, benzimidazolyle, benzoxazolyle, benzisoxazolyle, benzothiazolyle, benzisothiazolyle, indazolyle, pyrrolopyridinyle, furopyridinyle, dihydrofuropyridinyle, thiénopyridinyle, dihydrothiénopyridinyle, imidazopyridinyle, pyrazolopyridinyle, oxazolopyridinyle, isoxazolopyridinyle, thiazolopyridinyle,
éventuellement substitué par un ou plusieurs substituants choisis parmi un atome d'halogène, un groupe cyano, nitro, C₁₋₅-alkyle, C₁₋₃-alcoxy, C₁₋₅-thioalkyle, C₁₋₅-fluoroalkyle, C₁₋₃-fluoroalcoxy, C₁₋₃-fluorothioalkyle, C₃₋₅-cycloalkyle, C₃₋₅-cycloalkyle-C₁₋₃-alkylène, pipéridinyle, pyrrolidinyle, morpholinyle, NH₂, NHR₆, NR₆R₇, NHCOR₆, COR₆, CO₂R₆ SO₂R₆ -O-(C₁₋₃-alkylène)-O-, phényle, pyridinyle ou pyrimidinyle;
les groupes phényle pyridinyle et pyrimidinyle pouvant être substitués par un ou plusieurs substituants choisis parmi un atome d'halogène, un groupe cyano, nitro, C₁₋₅-alkyle, C₁₋₃-alcoxy, C₁₋₅-thioalkyle, C₁₋₅-fluoroalkyle, C₁₋₃-fluoroalcoxy, C₁₋₃-fluorothioalkyle, C₃₋₅-cycloalkyle, C₃₋₅-cycloalkyle-C₁₋₃-alkylène, pipéridinyle, pyrrolidinyle, morpholinyle, NH₂, NHR₆, NR₆R₇, NHCOR₆, COR₆, CO₂R₆, SO₂R₆, -O-(C₁₋₃-alkylène)-O-;
R₃ représente un groupe de formule générale CHR₄CONHR₅ dans laquelle
R₄ représente un atome d'hydrogène ou un groupe C₁₋₃-alkyle et
R₅ représente un atome d'hydrogène ou un groupe C₁₋₃-alkyle, C₃₋₅-cycloalkyle, C₃₋₅-cycloalkyle- C₁₋₃-alkylène
R₆ et R₇ représentent indépendamment l'un de l'autre un groupe C₁₋₃-alkyle.

Les composés de formule générale (I) peuvent comporter un ou plusieurs carbones asymétriques. Ils peuvent exister sous forme d'énantiomères ou de diastéréoisomères. Ces énantiomères et diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de l'invention, on entend par :
- C_{t-z} où t et z peuvent prendre les valeurs de 1 à 8, une chaîne carbonée pouvant avoir de t à z atomes de carbone, par exemple C₁₋₃ une chaîne carbonée qui peut avoir de 1 à 3 atomes de carbone,
- alkyle, un groupe aliphatique saturé, linéaire ou ramifié, par exemple un groupe C₁₋₃-alkyle représente une chaîne carbonée de 1 à 3 atomes de carbone, linéaire ou ramifiée, plus particulièrement un méthyle, éthyle, propyle, 1-méthyléthyle ,
- alkylène, un groupe alkyle divalent saturé, linéaire ou ramifié, par exemple un groupe C₁₋₃-alkylène représente une chaîne carbonée divalente de 1 à 3 atomes de carbone, linéaire ou ramifiée, plus particulièrement un méthylène, éthylène, 1-méthyléthylène, propylène ,
- cycloalkyle, un groupe alkyle cyclique, par exemple un groupe C₃₋₅-cycloalkyle représente un groupe carboné cyclique de 3 à 5 atomes de carbone, plus particulièrement un cyclopropyle, cyclobutyle, cyclopentyle ,
- alcoxy, un groupe -O-alkyle à chaîne aliphatique saturée, linéaire ou ramifiée,
- thioalkyle, un groupe -S-alkyle à chaîne aliphatique saturée, linéaire ou ramifiée ,
- fluoroalkyle, un groupe alkyle dont un ou plusieurs atomes d'hydrogène ont été substitués par un atome de fluor,
- fluoroalcoxy, un groupe alcoxy dont un ou plusieurs atomes d'hydrogène ont été substitués par un atome de fluor,
- fluorothioalkyle, un groupe thioalkyle dont un ou plusieurs atomes d'hydrogène ont été substitués par un atome de fluor
- atome d'halogène, un fluor, un chlore, un brome ou un iode.

Les composés de l'invention peuvent être préparés selon différentes méthodes, illustrées par les schémas qui suivent.

Ainsi une première méthode (schéma 1) consiste à faire réagir une amine de formule générale (II), dans laquelle A, B, R₁, n et m sont tels que définis ci-dessus, avec un carbonate de formule générale (IIIa) dans laquelle Z représente un atome d'hydrogène ou un groupe nitro, R₄ est tel que défini ci-dessus et R représente un groupe méthyle ou éthyle. Le carbamate-ester de formule générale (Ia) ainsi obtenu est ensuite transformé en composé de formule générale (I), par aminolyse au moyen d'une amine de formule générale R₅NH₂ dans laquelle R₅ est tel que défini ci-dessus. La réaction d'aminolyse peut être réalisée dans un solvant tel que le méthanol ou un mélange de solvants tels que le méthanol et le tétrahydrofurane ou le méthanol et le dioxane.

Une variante d'obtention des composés de formule générale (I) (schéma 1) consiste à faire réagir une amine de formule générale (II), telle que définie ci-dessus, avec un carbonate de formule générale (IIIb), dans laquelle Z représente un atome d'hydrogène ou un groupe nitro et R₄ et R₅ sont tels que définis ci-dessus, dans un solvant tel que le toluène ou le dichloroéthane, à une température comprise entre 0°C et 80°C.

Les carbonates de formule générale (IIIa) et (IIIb) peuvent être préparés selon toute méthode décrite dans la littérature, par exemple par réaction d'un alcool de formule générale HOCHR₄COOR où R représente un groupe méthyle ou éthyle, ou HOCHR₄CONHR₅ où R₄ et R₅ sont tels que définis ci-dessus, avec le chloroformiate de phényle ou de 4-nitrophényle, en présence d'une base telle que la triéthylamine ou la diisopropyléthylamine.

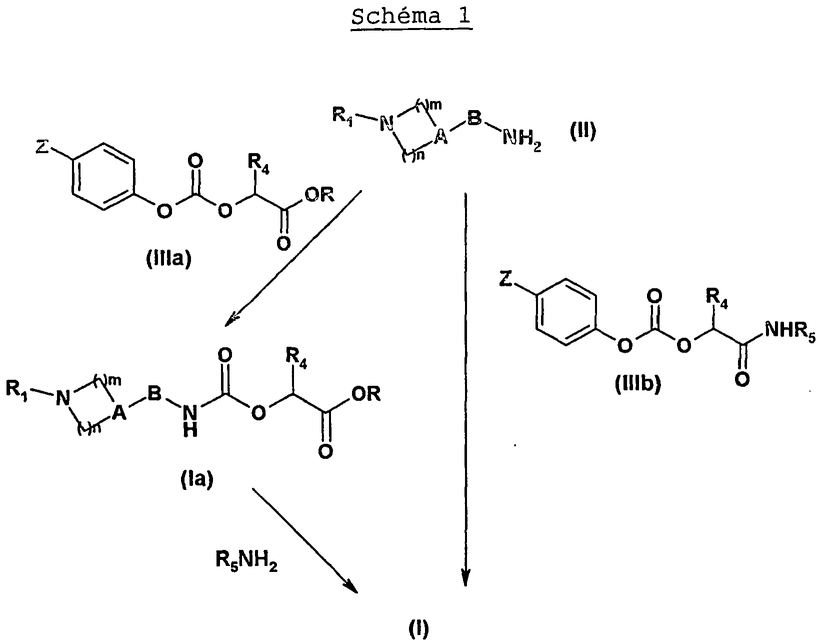

Une seconde méthode d'obtention des composés de formule générale (I) (schéma 2), consiste à faire réagir un dérivé de formule générale (IIa) dans laquelle Y représente un groupe hydroxyle, mésylate, tosylate, ou un atome de chlore, de brome ou d'iode, et A, B, R₁, n et m sont tels que définis ci-dessus, avec une oxazolidine-dione de formule générale (IV) dans laquelle R₄ est tel que défini ci-dessus, pour fournir le dérivé oxazolidine-dione de formule générale (V). Dans le cas où Y représente un groupe hydroxyle, la réaction peut être conduite suivant les conditions de Mitsunobu (Synthesis, 1981, 1-28), par exemple, par action d'azodicarboxylate de diéthyle ou de diisopropyle en présence de triphénylphosphine. Dans le cas où Y représente un atome de chlore, de brome, ou d'iode, ou un groupe mésylate ou tosylate, la réaction peut être conduite en présence d'une base telle que la 1,1,3,3-tétraméthylguanidine, l'hydrure de sodium ou le tert-butoxyde de sodium dans un solvant tel que le tétrahydrofurane, l'acétonitrile ou le diméthylformamide à une température comprise entre 0°C et la température de reflux du solvant. Le dérivé oxazolidine-dione de formule générale (V) ainsi obtenu est ensuite transformé en composé de formule générale (I), par aminolyse au moyen d'une amine de formule générale R₅NH₂ dans laquelle R₅ est tel que défini ci-dessus.

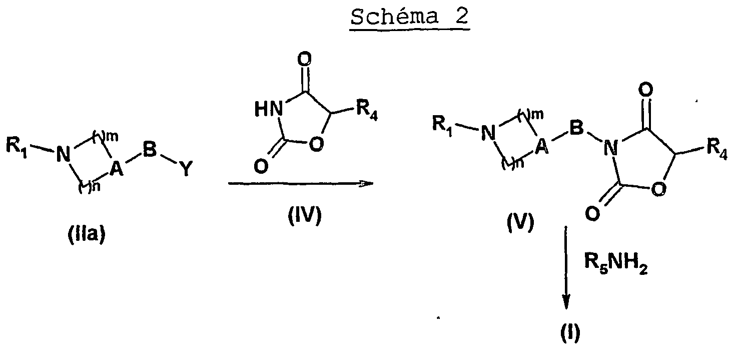

Les composés de formule générale (I), (Ia), (II), (IIa) et (V), dans laquelle R₁ est substitué par un groupe R", peuvent être également préparés par réaction des composés de formule générale (I), (Ia), (II), (IIa) et (V) correspondants, pour lesquels R₁ est substitué par un atome de chlore, de brome, d'iode ou par un groupement triflate dans la position où le groupe R" doit être introduit, avec un dérivé d'acide aryl- ou hétéroaryl-boronique suivant les conditions de réaction de Suzuki (Chem. Rev. 1995, 95, 2457-2483) ou avec un dérivé d'aryl- ou d'hétéroaryl-tri-alkylstannane suivant les conditions de réaction de Stille (Angew. Chem. Int. Ed. 1986, 25, 504-524).

Les composés de formules générales (II), (IIa) et (IV) quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les amines de formule générale R₅NH₂ sont disponibles dans le commerce .

L'invention, selon un autre de ses aspects, a également pour objet les composés de formule (Ia) dans laquelle n, m, A, B, R₁ et R₄ sont tels que définis ci-dessus, et R représente un groupe méthyle ou éthyle. Les composés de formule (Ia) sont utiles comme intermédiaires de synthèse pour la préparation des composés de formule (I).

L'invention, selon un autre de ses aspects, a également pour objet les composés de formule (V) dans laquelle n, m, A, B, R₁ et R₄ sont tels que définis ci-dessus, les composés suivants étant exclus :
* 3-[1-(4-amino-6,7-diméthoxy-2-quinazolinyl)-4-pipéridinyl]-2,4-oxazolidinedione
* 3-[1-(4-amino-6,7-diméthoxy-2-quinazolinyl)-4-pipéridinyl]-5-méthyl-2,4-oxazolidinedione
* 3-[1-(4-amino-6,7-diméthoxy-2-quinazolinyl)-4-pipéridinyl]-5-éthyl-2,4-oxazolidinedione
* 3-[1-(4-amino-6,7-diméthoxy-2-quinazolinyl)-4-pipéridinyl]-5-propyl-2,4-oxazolidinedione
* 3-[1-(4-amino-6,7-diméthoxy-2-quinazolinyl)-4-pipéridinyl]-5-(1-méthyléthyl)-2,4-oxazolidinedione.

Les composés de formule (V) sont utiles comme intermédiaires de synthèse pour la préparation des composés de formule (I).

Les exemples qui vont suivre illustrent la préparation de quelques composés de l'invention. Ces exemples ne sont pas limitatifs et ne font qu'illustrer l'invention. Les microanalyses, les spectres I.R. et R.M.N. et/ou la LC-MS (Liquid Chromatography coupled to Mass Spectroscopy) confirment les structures et les puretés des composés obtenus. PF(°C) représente le point de fusion en degrés Celsius. Les numéros indiqués entre parenthèses dans les titres des exemples correspondent à ceux de la 1ère colonne du tableau ci-après.

La nomenclature UICPA (Union Internationale de Chimie Pure et Appliquée - IUPAC en anglais) a été utilisée pour la dénomination des composés dans les exemples suivants.

### Exemple 1 (Composé N°38)

### Chlorhydrate de 2-[4-(1-naphthyl)-1-pipérazinyl]éthyl-carbamate de 2-(méthylamino)-2-oxoéthyle

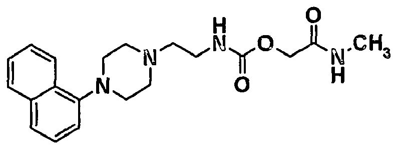

### 1.1. 2-{2-[4-(1-naphthyl)-1-pipérazinyl]éthyl}-1H-isoindole-1,3(2H)-dione

On chauffe à 90°C pendant deux heures une suspension de 1,17 g (5,52 mmoles) de 1-(1-naphthyl)pipérazine (Tetrahedron Letters, 1998, 39(15), 2219-2222), de 0,99 g (7,15 mmoles) de carbonate de potassium et de 1,68 g (6,62 mmoles) de 2-(2-bromoéthyl)-1H-isoindole-1,3(2*H*)-dione, dans 15 ml de *N*;*N*-diméthylformamide.

On laisse revenir à température ambiante et on concentre sous pression réduite. On reprend le résidu par de l'acétate d'éthyle et de l'eau, on sépare la phase aqueuse, on l'extrait trois fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 40/60 d'acétate d'éthyle et de cyclohexane. On obtient ainsi 1,14 g de produit pur sous forme d'huile.

### 1.2. 2-[4-(1-naphthyl)-1-pipérazinyl]éthanamine

A une solution de 1,14 g (2,96 mmoles) de 2-{2-[4-(1-naphthyl)-1-pipérazinyl]éthyl}-1*H*-isoindole-1,3(2*H*)-dione, préparé à l'étape 1.1., dans 15 ml d'éthanol, on ajoute lentement à température ambiante 0,17 ml (3,55 mmoles) d'hydrazine monohydrate. On porte ensuite le mélange réactionnel au reflux pendant une heure.

On laisse revenir à température ambiante, on sépare l'insoluble par filtration et on concentre le filtrat sous pression réduite. On reprend le résidu par 20 ml d'éther et on laisse sous agitation à température ambiante pendant vingt minutes. On sépare de nouveau l'insoluble et on concentre le filtrat sous pression réduite. On purifie le résidu obtenu par chromatographie sur gel de silice en éluant avec un mélange 90/10/1 de dichlorométhane, de méthanol et d'ammoniaque à 28 %.

On obtient ainsi 0,45 g d'amine sous forme d'huile incolore.

### 1.3.[(phényloxycarbonyl)oxy]acétate d'éthyle

A une solution de 25 g (240 mmoles) de glycolate d'éthyle et de 55 ml (315 mmoles) de diisopropyléthylamine dans 500 ml de toluène, on ajoute lentement à température ambiante 32 ml (256 mmoles) de chloroformiate de phényle. On poursuit l'agitation à température ambiante pendant 2 heures.

On sépare le sel formé et on concentre le filtrat sous pression réduite.

On obtient 53,7 g de produit huileux que l'on utilise tel quel dans l'étape suivante.

### 1.4. [({2-4-(1-naphthyl)-1-pipérazinyl]éthyl}amino)carbonyl] oxyacétate d'éthyle

On chauffe à 50°C pendant 6 heures, une solution de 0,45 g (1,76 mmoles) de 2-[4-(1-naphthyl)-1-pipérazinyl]éthanamine, préparé à l'étape 1.2., et de 0,48 g (2,16 mmoles) de [(phényloxycarbonyl)oxy]acétate d'éthyle, obtenu à l'étape 1.3., dans 15 ml de toluène.

On laisse revenir à température ambiante, on sépare l'insoluble par filtration et on concentre le filtrat sous pression réduite. On reprend le résidu par du dichlorométhane et de l'eau, on sépare la phase aqueuse, on l'extrait trois fois avec du dichlorométhane, on lave les phases organiques réunies avec une solution aqueuse saturée en chlorure de sodium et on les sèche sur sulfate de sodium. Après évaporation du solvant, on purifie le résidu obtenu par chromatographie sur gel de silice en éluant avec un mélange 97/3 puis 93/7 de dichlorométhane et de méthanol.

On obtient ainsi 0,49 g de produit pur sous forme d'huile.

### 1.5. 2[4-(1-naphthyl)-1-pipérazinyl)éthylcarbamate de 2-(méthylamino)-2-oxoéthyle

A une solution de 0,48 g (1,25 mmoles) de [({2-4-(1-naphthyl)-1-pipérazinyl]éthyl}amino)carbonyl]oxyacétate d'éthyle, préparé à l'étape 1.4., dans 5 ml de méthanol, on ajoute 1,9 ml (3,75 mmoles) d'une solution de méthylamine (2M) dans le tétrahydrofurane. On poursuit l'agitation à température ambiante pendant 1 heure.

Après concentration sous pression réduite, on purifie le résidu obtenu par chromatographie sur gel de silice en éluant avec un mélange 97/3 de dichlorométhane et de méthanol. On obtient un résidu huileux qu'on reprend avec une solution d'acide chlorhydrique (5N) dans l'isopropanol. On concentre à sec puis on recristallise le sel obtenu dans un mélange 1/1 d'acétone et de diisopropyléther.

On obtient ainsi 0,23 g de produit pur, monochlorhydrate, sous forme de solide blanc.

LC-MS . M+H = 371

PF(°C) : 166°C

RMN¹H (DMSO) δ (ppm) : 2,55 (d, 3H) ; 3,20-3,75 (massif, 12H) ; 4,40 (s, 2H) ; 7,10 (d, 1H) ; 7,35-7,70 (massif, 4H)

; 7,80-8,05 (massif, 2H) ; 8,15 (dd, 1H) ; 10,90 (large s, 1H) .

### Exemple 2 (Composé N°157)

### Chlorhydrate de 3-[1-(1-isoquinolinyl)-4-pipéridinyl]-propylcarbamate de 2-(méthylamino)-2-oxoéthyle

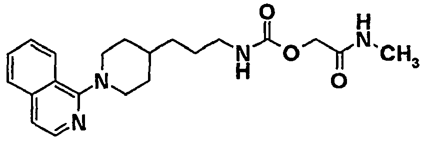

### 2.1. 2-[1-(1-isoquinolinyl)-4-pipéridinyl]éthanol

Sous atmosphère inerte, on introduit 4,17 g (25,50 mmoles) de 1-chloroisoquinoline, 3,62 g (28 mmoles) de 2-(4-pipéridinyl)éthanol, 5,90 g (61,20 mmoles) de tert-butylate de sodium et 0,476 g (0,765 mmole) de BINAP (2,2'-bis(diphénylphosphino)-1,1'-binaphthyle) en suspension dans 50 ml de toluène. On ajoute ensuite 0,233 g (0,255 mmole) de dipalladium tris(dibenzylidèneacétone). On porte ensuite le mélange réactionnel au reflux pendant 12 heures.

On sépare les sels par filtration sur célite, puis on concentre le filtrat sous pression réduite. On reprend le résidu avec de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 97/3 de dichlorométhane et de méthanol.

On obtient 3,34 g de produit sous forme de pâte grise.

### 2.2. 1-[4-(2-chloroéthyl)-1-pipéridinyl]isoquinoline

A une solution de 3,09 g (12,10 mmoles) de 2-[1-(1-isoquinolinyl)-4-pipéridinyl]éthanol, préparé à l'étape 2.1., dans 30 ml de dichlorométhane, on additionne goutte à goutte 2,20 ml (30,10 mmoles) de chlorure de thionyle. On porte ensuite le mélange réactionnel au reflux pendant 5 heures.

On concentre à sec sous pression réduite. On reprend le résidu dans 40 ml de dichlorométhane et 30 ml d'une solution aqueuse d'hydroxyde de sodium (1M). On sépare la phase aqueuse, on l'extrait deux fois avec du dichlorométhane, on lave les phases organiques réunies avec une solution aqueuse saturée en chlorure de sodium, on les sèche sur sulfate de sodium et on concentre le filtrat sous pression réduite. On obtient 2,70 g de produit sous forme de pâte brune utilisé tel quel dans l'étape suivante.

### 2.3. 3-[1-(1-isoquinolinyl)-4-pipéridinyl]propanenitrile

A une suspension de 2,63 g (9,57 mmoles) de 1-[4-(2-chloroéthyl)-1-pipéridinyl]isoquinoline, préparé à l'étape 2.2., et de 0,048 g (0,29 mmole) d'iodure de potassium dans 30 ml de diméthylsulfoxyde, on ajoute par petites portions 0,63 g (9,57 mmoles) de cyanure de potassium. On porte ensuite le mélange réactionnel à environ 120°C pendant 16 heures.

On laisse revenir à température ambiante puis on ajoute 90 ml d'eau et de l'acétate d'éthyle. On sépare la phase aqueuse, on l'extrait trois fois avec de l'acétate d'éthyle, on lave les phases organiques réunies avec une solution aqueuse saturée en chlorure de sodium, on les sèche sur sulfate de sodium et on concentre le filtrat sous pression réduite. On purifie le résidu ainsi obtenu par chromatographie sur gel de silice en éluant avec un mélange 30/70 d'acétate d'éthyle et de cyclohexane.

On obtient 0,77 g de produit sous forme de solide jaune. PF (°C) : 141-143°C

### 2.4. 3-[1-(1-isoquinolinyl)-4-pipéridinyl]propanamine

A une suspension de 0,22 g (5,80 mmoles) d'hydrure de lithium et d'aluminium dans 8 ml de tétrahydrofurane, on additionne goutte à goutte une solution de 0,77 g (2,90 mmoles) de 3-[1-(1-isoquinolinyl)-4-pipéridinyl]-propanenitrile, préparé à l'étape 2.3., dans 16 ml de tétrahydrofurane. On porte ensuite le mélange réactionnel au reflux pendant 5 heures.

On refroidit le milieu réactionnel à environ 0°C puis on additionne lentement 15 ml d'une solution aqueuse d'hydroxyde de sodium (1M). On laisse sous agitation à température ambiante pendant 30 minutes puis on additionne par petites portions du sulfate de sodium humide. On sépare les sels par filtration sur papier puis on laisse décanter. On extrait la phase aqueuse trois fois avec de l'acétate d'éthyle, on sèche les phases organiques réunies sur sulfate de sodium et on concentre le filtrat sous pression réduite. On purifie le résidu ainsi obtenu par chromatographie sur gel de silice en éluant avec un mélange 93/7/0,7 de dichlorométhane, de méthanol et d'ammoniaque à 28 %.

On obtient 0,329 g de produit sous forme d'huile jaune qui cristallise à température ambiante.

### 2.5. [{3-[1-(1-isoquinolinyl)-4-pipéridinyl]propyl}amino) carbonyl]oxyacétate d'éthyle

On procède comme décrit dans l'exemple 1 (étape 1.4.). A partir de 0,350 g (1,30 mmoles) de 3-[1-(1-isoquinolinyl)-4-pipéridinyl]propanamine, obtenu à l'étape 2.4., et de 0,32 g (1,43 mmoles) de [(phényloxycarbonyl)oxy]acétate d'éthyle, préparé à l'étape 1.3. de l'exemple 1, et après chromatographie sur gel de silice en éluant avec un mélange 20/80 puis 30/70 d'acétate d'éthyle et de cyclohexane, on obtient 0,383 g de produit sous forme de pâte jaune.

### 2.6. 3-[1-(1-isoquinolinyl)-4-pipéridinyl]propylcarbamate de 2-(méthylamino)-2-oxoéthyle

On procède comme décrit dans l'exemple 1 (étape 1.5.). A partir de 0,380 g (0,95 mmole) de [{3-[1-(1-isoquinolinyl)-4-pipéridinyl]propyl}amino)carbonyl]oxyacétate d'éthyle, obtenu à l'étape 2.5., et de 4,8 ml (9,51 mmoles) d'une solution de méthylamine (2M) dans le tétrahydrofurane, et après chromatographie sur gel de silice en éluant avec un mélange 98/2 puis 95/5 de dichlorométhane et de méthanol, on obtient 0,277 g de produit sous forme d'huile. On reprend ensuite ce résidu huileux avec une solution d'acide chlorhydrique (5N) dans l'isopropanol, on filtre le sel formé puis on le lave avec de l'acétate d'éthyle.

On obtient, après séchage sous vide à environ 40°C, 0,204 g de chlorhydrate sous forme de solide blanc amorphe.

LC-MS _{:} M+H = 385

RMN ¹H (DMSO+D₂O) δ (ppm) : 1,30 (m, 2H) ; 1,40-1,80 (massif, 5H) ; 1,90 (large d, 2H) ; 2,60 (s, 3H) ; 3,05 (m, 2H) ; 3,40 (t, 2H) ; 4,05 (large d, 2H) ; 4,30 (s, 2H) ; 7,50 (d, 1H) ; 7,80 (m, 2H) ; 7,95 (t, 1H) ; 8,05 (d, 1H) 8,20 (d, 1H).

### Exemple 3 (Composé N°44)

### 2-{1-[3-(trifluorométhyl)phényl]-4-pipéridinyl}éthylcarbamate de 2-amino-2-oxoéthyle

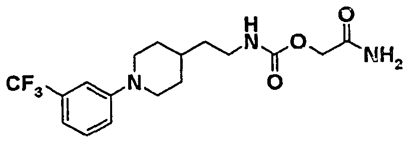

### 3.1. 2-{1-[3-(trifluorométhyl)phényl]-4-pipéridinyl}éthanol

On procède comme décrit dans l'exemple 2 (étape 2.1.). A partir de 25,6 g (113,90 mmoles) de 1-bromo-3-(trifluorométhyl)-benzène, de 17,66 g (136,60 mmoles) de 2-(4-pipéridinyl)-éthanol, de 26,24 g (273 mmoles) de tert-butylate de sodium, de 2,12 g (3,41 mmoles) de BINAP et de 1,04 g (1,14 mmoles) de dipalladium tris(dibenzylidène-acétone), et après chromatographie sur gel de silice en éluant avec un mélange 25/75 d'acétate d'éthyle et de cyclohexane, on obtient 17,90 g d'un résidu huileux orangé. On reprend ensuite ce résidu avec 100 ml de méthanol puis on additionne une solution de 4,24 g d'hydroxyde de potassium dans 15 ml de méthanol et on poursuit l'agitation à température ambiante pendant 1 heure. On concentre sous pression réduite puis on reprend le résidu par du chloroforme et une solution aqueuse d'acide chlorhydrique (1N). On sépare la phase organique, on la sèche sur sulfate de sodium et on concentre le filtrat sous pression réduite. On obtient 14 g de produit sous forme d'huile jaune foncée utilisé tel quel dans l'étape suivante.

### 3.2. méthanesulfonate de 2-{1-[3-(trifluorométhyl)phényl]-4-pipéridinyl}éthyle

A une solution de 2 g (7,32 mmoles) de 2-{1-[3-(trifluorométhyl)phényl]-4-pipéridinyl}éthanol, obtenu à l'étape 3.1., et de 1,53 ml (10,98 mmoles) de triéthylamine dans 40 ml de dichlorométhane, refroidie à environ 0°C, on ajoute goutte à goutte, sous atmosphère inerte, une solution de 1 g (8,78 mmoles) de chlorure de mésyle dans 5 ml de dichlorométhane. On poursuit l'agitation à 0°C pendant une heure puis à température ambiante pendant deux heures.

On ajoute de l'eau au milieu réactionnel, on sépare la phase aqueuse, on l'extrait trois fois avec du dichlorométhane, on lave les phases organiques réunies avec une solution aqueuse saturée en chlorure de sodium, on les sèche sur sulfate de sodium et on concentre le filtrat sous pression réduite. On obtient ainsi 2,5 g de produit sous forme d'huile utilisé tel quel dans l'étape suivante.

### 3.3. 3-(2-{1-[3-(trifluorométhyl)phényl]-4-pipéridinyl}-éthyl)-1,3-oxazolidine-2,4-dione

On porte au reflux pendant 12 heures, sous atmosphère inerte, une solution de 2,3 g (6,545 mmoles) de méthane sulfonate de 2-{1-[3-(trifluorométhyl)phényl]-4-pipéridinyl}éthyle, préparé à l'étape 3.2., de 0,694 g (6,87 mmoles) de 1,3-oxazolidin-2,4-dione (J. Med. Chem., 1991, 34, 1538-1544) et de 1,5 g (13,09 mmoles) de 1,1,3,3-tétraméthylguanidine, dans 30 ml de tétrahydrofurane.

On concentre sous pression réduite. On reprend le résidu par du dichlorométhane et de l'eau, on sépare la phase aqueuse, on l'extrait deux fois avec du dichlorométhane, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 20/80 puis 40/60 d'acétate d'éthyle et de cyclohexane.

On obtient 1,61 g de produit pur sous forme d'huile.

### 3.4. 2-{1-[3-(trifluorométhyl)phényl]-4-pipéridinyl}éthylcarbamate de 2-amino-2-oxoéthyle

A une solution de 0,77 g (2,16 mmoles) de 3-(2-{1-[3-(trifluorométhyl)phényl]-4-pipéridinyl}éthyl)-1,3-oxazolidine-2,4-dione, obtenu à l'étape 3.3., dans 10 ml d'un mélange 1/1 de méthanol et de tétrahydrofurane, on ajoute 9,3 ml (64,82 mmoles) d'une solution d'ammoniaque (7N) dans le méthanol. On poursuit l'agitation à température ambiante pendant 24 heures.

Après concentration sous pression réduite, on purifie le résidu obtenu par chromatographie sur gel de silice en éluant avec un mélange 97/3 puis 95/5 de dichlorométhane et de méthanol, suivie d'une recristallisation dans un mélange d'acétate d'éthyle et de diisopropyléther.

On obtient 0,370 g de produit pur sous forme de solide blanc.

LC-MS : M+H = 374

PF(°C) : 140-142°C

RMN ¹H (CDCl₃) δ (ppm) : 1,30-1,55 (massif, 5H) ; 1,90 (large d, 2H) ; 2,80 (t, 2H) ; 3,35 (q, 2H) ; 3,80 (large d, 2H) ; 4,60 (s, 2H) ; 4,90 (large s, 1H) ; 5,55 (large s, 1H) 6,05 (large s, 1H) ; 7,10 (m, 3H) ; 7,35 (t, 1H) .

### Exemple 4 (Composé N°47)

### Chlorhydrate de 2-[1-(6-méthyl-2-pyridinyl)-4-pipéridinyl]-éthylcarbamate de 2-(méthylamino)-2-oxoéthyle

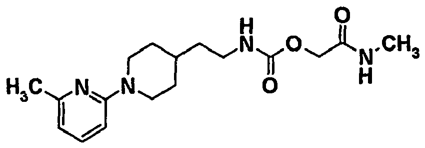

### 4.1. 2-[1-(6-méthyl-2-pyridinyl)-4-pipéridinyl]éthanol

Dans un autoclave, on introduit 1 g (7,74 mmoles) de 2-(4-pipéridinyl)éthanol et 0,987 g (7,74 mmoles) de 2-chloro-6-méthylpyridine. On chauffe ensuite à 130°C pendant 17 heures.

On laisse revenir à température ambiante puis on reprend le mélange réactionnel par du chloroforme et une solution aqueuse saturée en hydrogénocarbonate de sodium. On sépare la phase aqueuse, on l'extrait deux fois avec du chloroforme, on lave les phases organiques réunies avec une solution aqueuse saturée en chlorure de sodium, on les sèche sur sulfate de sodium et on concentre le filtrat sous pression réduite.

On obtient ainsi 1,21 g de produit sous forme d'un liquide orange utilisé tel quel dans l'étape suivante.

### 4.2. méthanesulfonate de 2-[1-(6-méthyl-2-pyridinyl)-4-pipéridinyl]éthyle

On procède comme décrit dans l'exemple 3 (étape 3.2.). A partir de 0,661 g (3 mmoles) de 2-[1-(6-méthyl-2-pyridinyl)-4-pipéridinyl]éthanol, obtenu à l'étape 4.1., de 0,378 g (3,30 mmoles) de chlorure de mésyle et de 0,63 ml (4,50 mmoles) de triéthylamine, on obtient 0,779 g de produit sous forme d'huile orange utilisé tel quel dans l'étape suivante.

### 4.3. 3-{2-[1-(6-méthyl-2-pyridinyl)-4-pipéridinyl]éthyl}-1,3-oxazolidine-2,4-dione

On procède suivant la méthode décrite dans l'exemple 3 (étape 3.3.). A partir de 0,776 g (2,60 mmoles) de méthanesulfonate de 2-[1-(6-méthyl-2-pyridinyl)-4-pipéridinyl]éthyle, obtenu à l'étape 4.2., de 0,315 g (3,12 mmoles) de 1,3-oxazolidin-2,4-dione et de 0,65 ml (5,20 mmoles) de 1,1,3,3-tétraméthylguanidine, et après chromatographie sur gel de silice en éluant avec un mélange 30/70 d'acétate d'éthyle et de cyclohexane, on obtient 0,76 g de produit pur sous forme d'huile jaune.

### 4.4. 2-[1-(6-méthyl-2-pyridinyl)-4-pipéridinyl]éthylcarbamate de 2-(méthylamino)-2-oxoéthyle

On procède suivant le mode opératoire décrit dans l'exemple 1 (étape 1.5.). A partir de 0,841 g (2,77 mmoles) de 3-{2-[1-(6-méthyl-2-pyridinyl)-4-pipéridinyl]éthyl}-1,3-oxazolidine-2,4-dione, obtenu à l'étape 4.3., et de 6,9 ml (13,86 mmoles) d'une solution de méthylamine (2M) dans le tétrahydrofurane, on obtient 0,598 g de produit pur sous forme d'huile, après chromatographie sur gel de silice en éluant avec un mélange 95/5 de dichlorométhane et de méthanol. On reprend ensuite ce résidu huileux avec une solution d'acide chlorhydrique (5N) dans l'isopropanol, on filtre le sel formé puis on le lave successivement avec de l'acétone puis de l'éther.

On obtient, après séchage sous vide à environ 80°C, 0,492 g de chlorhydrate sous forme de poudre blanche.

LC-MS : M+H = 335

PF(°C) . 95-100°C

RMN ¹H (DMSO+D₂O) δ (ppm) : 0,95-1,45 (massif, 4H) ; 1,60 (m, 1H) ; 1,80 (large d, 2H) ; 2,40 (s, 3H) ; 2,60 (s, 3H) ; 2,90-3,20 (massif, 4H) ; 4,10 (large d, 2H) ; 4,30 (s, 2H) ; 6,70 (d, 1H) ; 7,10 (d, 1H) ; 7,75 (dd, 1H) .

### Exemple 5 (Composé N°154)

### Chlorhydrate de [1-(1-isoquinolinyl)-4-pipéridinyl]méthylcarbamate de 2-(méthylamino)-2-oxoéthyle

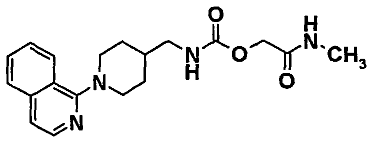

### 5.1. [1-(1-isoquinolinyl)-4-pipéridinyl]méthanol

On procède comme décrit dans l'exemple 2 (étape 2.1.). A partir de 2,50 g (15,28 mmoles) de 1-chloroisoquinoline, de 1,94 g (136,6 mmoles) de 4-pipéridinylméthanol, de 3,53 g (36,67 mmoles) de tert-butylate de sodium, de 0,285 g (0,46 mmole) de BINAP et de 0,140 g (0,15 mmole) de dipalladium tris(dibenzylidèneacétone), et après chromatographie sur gel de silice en éluant avec un mélange 98/2/0,2 puis 95/5/0,5 de dichlorométhane, de méthanol et d'ammoniaque à 28 %, on obtient 2,40 g de produit pur sous forme d'huile orange visqueuse.

### 5.2. 3-{[1-(1-isoquinolinyl)-4-pipéridinyl]méthyl}-1,3-oxazolidine-2,4-dione

A une solution de 2,4 g (9,95 mmoles) de [1-(1-isoquinolinyl)-4-pipéridinyl]méthanol, préparé à l'étape 5.1., de 2,87 g (10,94 mmoles) de triphénylphosphine et de 1,21 g (11,93 mmoles) de 1,3-oxazolidin-2,4-dione dans 40 ml de tétrahydrofurane, refroidie à environ -10°C, on ajoute goutte à goutte, sous atmosphère inerte, une solution de 2,01 g (9,95 mmoles) de diisopropylazodicarboxylate (DIAD) dans 5 ml de tétrahydrofurane tout en maintenant la température du milieu réactionnel entre -10°C et 0°C. On poursuit l'agitation à 0°C pendant 1 heure, puis à 25°C pendant 18 heures.

On concentre sous pression réduite, on reprend le résidu avec du dichlorométhane et 10 ml d'une solution aqueuse d'hydroxyde de sodium à 5 %. On sépare la phase aqueuse puis on l'extrait 2 fois avec du dichlorométhane. On réunit les phases organiques et on les lave successivement avec une solution aqueuse d'acide chlorhydrique (1N), puis une solution aqueuse saturée d'hydrogénocarbonate de sodium et une solution aqueuse saturée de chlorure de sodium. On sèche la phase organique sur sulfate de sodium et on concentre le filtrat sous pression réduite. On purifie le résidu ainsi obtenu par chromatographie sur gel de silice en éluant avec un mélange 99/1/0,1 puis 98/2/0,2 de dichlorométhane, de méthanol et d'ammoniaque à 28 %.

On obtient ainsi 3,57 g d'oxazolidine-dione sous forme de pâte orange.

### 5.3. [1-(1-isoquinolinyl)-4-pipéridinyl]méthylcarbamate de 2-(méthylamino)-2-oxoéthyle

On procède suivant le mode opératoire décrit dans l'exemple 1 (étape 1.5.). A partir de 3,57 g (10,97 mmoles) de 3-{[1-(1-isoquinolinyl)-4-pipéridinyl]méthyl}-1,3-oxazolidine-2,4-dione, obtenu à l'étape 5.2., et de 27 ml (54,86 mmoles) d'une solution de méthylamine (2M) dans le tétrahydrofurane, et après chromatographie sur gel de silice en éluant avec un mélange 95/5/0,5 de dichlorométhane, de méthanol et d'ammoniaque à 28 %, on obtient 0,90 g de produit pur sous forme de pâte jaune. On reprend ensuite ce résidu avec une solution d'acide chlorhydrique (5N) dans l'isopropanol, on filtre le sel formé puis on le lave avec de l'acétone.

On obtient, après séchage sous vide à environ 80°C, 0,728 g de chlorhydrate sous forme de solide jaune pâle.

LC-MS : M+H = 357

PF(°C) : 208-212°C (décomposition)

RMN ¹H (D₂O) δ (ppm) : 1,55 (m, 2H) ; 1,95 (m, 3H) ; 2,70 (s, 3H) ; 3,20 (large d, 2H) ; 3,45 (t, 2H) ; 4,10 (large d, 2H) ; 4,50 (s, 2H) ; 7,35 (d, 1H) ; 7,55 (d, 1H) ; 7,70 (m, 1H) ; 7,90 (d, 2H) ; 8,20 (d, 1H) .

### Exemple 6 (Composé N°158)

### 2-[1-(6-fluoro-1-isoquinolinyl)-4-pipéridinyl]éthylcarbamate de 2-amino-2-oxoéthyle

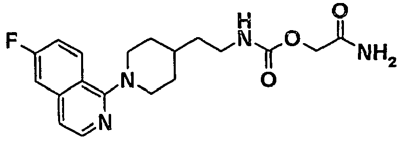

### 6.1. 2-[1-(6-fluoro-1-isoquinolinyl)-4-pipéridinyl]éthanol

On procède comme décrit dans l'exemple 4 (étape 4.1.). A partir de 1,52 g (8,39 mmoles) de 1-chloro-6-fluoroisoquinoline et de 1,20 g (9,23 mmoles) de 2-(4-pipéridinyl)éthanol, et après chromatographie sur gel de silice en éluant avec un mélange 95/5/0,5 de dichlorométhane, de méthanol et d'ammoniaque à 28 %, on obtient 0,90 g de produit pur sous forme de pâte jaune.

### 6.2. méthanesulfonate de 2-[1-(6-fluoro-1-isoquinolinyl)-4-pipéridinyl]éthyle

On procède comme décrit dans l'exemple 3 (étape 3.2.). A partir de 1,47 g (5,36 mmoles) de 2-[1-(6-fluoro-1-isoquinolinyl)-4-pipéridinyl]éthanol, obtenu à l'étape 6.1., de 0,675 g (5,89 mmoles) de chlorure de mésyle et de 1,13 ml (8,04 mmoles) de triéthylamine, on obtient 1,80 g de produit sous forme d'huile utilisé tel quel dans l'étape suivante.

### 6.3. 3-{2-[1-(6-fluoro-1-isoquinolinyl)-4-pipéridinyl]éthyl} 1,3-oxazolidine-2,4-dione

On procède suivant la méthode décrite dans l'exemple 3 (étape 3.3.). A partir de 1,80 g (5,10 mmoles) de méthanesulfonate de 2-[1-(6-fluoro-1-isoquinolinyl)-4-pipéridinyl]éthyle, obtenu à l'étape 6.2., de 0,62 g (6,13 mmoles) de 1,3-oxazolidin-2,4-dione et de 1,30 ml (10,21 mmoles) de 1,1,3,3-tétraméthylguanidine, et après chromatographie sur gel de silice en éluant avec un mélange 40/60 d'acétate d'éthyle et de cyclohexane, on obtient 1,34 g de produit pur sous forme de solide amorphe blanc.

### 6.4. 2-[1-(6-fluoro-1-isoquinolinyl)-4-pipéridinyl) éthylcarbamate de 2-amino-2-oxoéthyle

On procède suivant le mode opératoire décrit dans l'exemple 3 (étape 3.4.). A partir de 0,597 g (1,67 mmoles) de 3-{2-[1-(6-fluoro-1-isoquinolinyl)-4-pipéridinyl]éthyl}1,3-oxazolidine-2,4-dione, obtenu à l'étape 6.3., et de 14,30 ml (100,20 mmoles) d'une solution d'ammoniaque (7M) dans le méthanol, et après chromatographie sur gel de silice en éluant avec un mélange 95/5 de dichlorométhane et de méthanol, suivie d'une recristallisation dans le diisopropyléther, on obtient 0,168 g de produit pur sous forme de solide blanc.

LC-MS : M+H = 375

PF(°C) . 135-139°C

RMN ¹H (DMSO) δ (ppm) : 1,20-1,70 (massif, 5H) ; 1,80 (large d, 2H) ; 2,85 (t, 2H) ; 3,10 (large d, 2H) ; 3,70 (large d, 2H) ; 4,30 (s, 2H) ; 6,95-7,20 (massif, 3H) ; 7,30 (d, 1H) ; 7,45 (td, 1H) ; 7,60 (dd, 1H) ; 8,10 (m, 2H) .

### Exemple 7 (Composé N°172)

### Chlorhydrate de 2-[1-(4-isoquinolinyl)-4-pipéridinyl]éthylcarbamate de 2-(méthylamino)-2-oxoéthyle

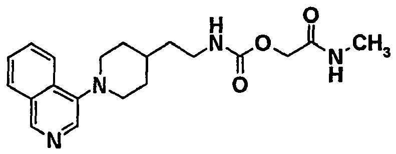

### 7.1. 2-[1-(4-isoquinolinyl)-4-pipéridinyl]éthanol

On procède suivant le protocole décrit dans l'exemple 2 (étape 2.1.). A partir de 1 g (4,81 mmoles) de 4-bromoisoquinoline, de 0,683 g (5,29 mmoles) de 2-(4-pipéridinyl) éthanol, de 1,11 g (11,50 mmoles) de tert-butylate de sodium, de 0,090 g (0,144 mmole) de BINAP et de 0,044 g (0,048 mmole) de dipalladium tris(dibenzylidèneacétone), et après chromatographie sur gel de silice en éluant avec un mélange 97/3 puis 95/5 de dichlorométhane et de méthanol, on obtient 0,810 g de produit sous forme d'un liquide vert visqueux.

### 7.2. 3-{2-[1-(4-isoquinolinyl)-4-pipéridinyl] éthyl}1,3-oxazolidine-2,4-dione

On utilise le mode opératoire décrit dans l'exemple 5 (étape 5.2.). A partir de 0,801 g (3,12 mmoles) de 2-[1-(4-isoquinolinyl)-4-pipéridinyl]éthanol, préparé à l'étape 7.1., de 0,902 g (3,44 mmoles) de triphénylphosphine, de 0,379 g (3,75 mmoles) de 1,3-oxazolidin-2,4-dione, et de 0,632 g (3,12 mmoles) de diisopropylazodicarboxylate (DIAD), et après chromatographie sur gel de silice en éluant avec un mélange 98/2 de dichlorométhane et de méthanol, on obtient 1 g de produit sous forme de pâte verte.

### 7.3. 2-[1-(4-isoquinolinyl)-4-pipéridinyl]éthylcarbamate de 2-(méthylamino)-2-oxoéthyle

On utilise le mode opératoire décrit dans l'exemple 1 (étape 1.5.). A partir de 1 g (2,95 mmoles) de 3-{2-[1-(4-isoquinolinyl)-4-pipéridinyl]éthyl}1,3-oxazolidine-2,4-dione, obtenu à l'étape 7.2., et de 7,40 ml (14,73 mmoles) d'une solution de méthylamine (2M) dans le tétrahydrofurane, et après chromatographie sur gel de silice en éluant avec un mélange 95/5 de dichlorométhane et de méthanol, on obtient 0,410 g de produit pur sous forme de solide amorphe blanc. On reprend ensuite ce résidu huileux avec une solution d'acide chlorhydrique (5N) dans l'isopropanol, on filtre le sel formé puis on le lave successivement avec de l'acétone puis du diisopropyléther.

On obtient, après séchage sous vide à environ 90°C, 0,359 g de chlorhydrate sous forme de solide jaune.

LC-MS _{:} M+H = 371

PF(°C) : 205-210°C

RMN ¹H (DMSO) δ (ppm) : 1,30-1,70 (massif, 5H) ; 1,90 (large d, 2H) ; 2,60 (d, 3H) ; 2,90 (t, 2H) ; 3,15 (m, 2H) ; 3,50 (large d, 2H) ; 4,35 (s, 2H) ; 7,25 (large t, 1H) ; 7,80 (large s, 1H) ; 8,0 (dd, 1H) ; 8,05-8,30 (massif, 3H) ; 8,45 (d, 1H) ; 9,45 (s, 1H) .

### Exemple 8 (Composé N° 126)

### 2-[1-(2-quinolinyl)-4-pipéridinyl]éthylcarbamate de 2-(méthylamino)2-oxoéthyle

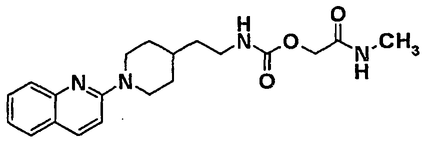

### 8.1. 2-[1-(2-quinolinyl)-4-pipéridinyl]éthanol

On procède comme décrit dans l'exemple 4 (étape 4.1.). A partir de 2 g (12,20 mmoles) de 2-chloroquinoline et de 1,58 g (12,20 mmoles) de 2-(4-pipéridinyl)éthanol, et après chromatographie sur gel de silice en éluant avec un mélange 98/2/0,2 puis 95/5/0,5 de dichlorométhane, de méthanol et d'ammoniaque à 28 %, on obtient 2,36 g de produit pur sous forme d'huile jaune pâle qui cristallise à température ambiante.

### 8.2. 3{2-[1-(2-quinolinyl)-4-pipéridinyl]éthyl-1,3-oxazolidine-2,4-dione

On procède suivant le mode opératoire décrit dans l'exemple 5 (étape 5.2.). A partir de 2,22 g (8,65 mmoles) de 2-[1-(2-quinolinyl)-4-pipéridinyl]éthanol, préparé à l'étape 8.1., de 2,50 g (9,52 mmoles) de triphénylphosphine, de 1,05 g (10,38 mmoles) de 1,3-oxazolidin-2,4-dione, et de 1,75 g (8,65 mmoles) de diisopropylazodicarboxylate (DIAD), et après chromatographie sur gel de silice en éluant avec un mélange 30/70 puis 40/60 d'acétate d'éthyle et de cyclàhexane, on obtient 2,63 g de produit sous forme de solide amorphe blanc.

### 8.3. 2-[1-(2-quinolinyl)-4-pipéridinyl]éthylcarbamate de 2-(méthylamino)2-oxoéthyle

On procède suivant le mode opératoire décrit dans l'exemple 1 (étape 1.5.). A partir de 1,5 g (4,42 mmoles) de 3{2-[1-(2-quinolinyl)-4-pipéridinyl]éthyl}-1,3-oxazolidine-2,4-dione, obtenu à l'étape 8.2., et de 11 ml (22,10 mmoles) d'une solution de méthylamine (2M) dans le tétrahydrofurane, et après chromatographie sur gel de silice en éluant avec un mélange 98/2/0,2 puis 95/5/0,5 de dichlorométhane, de méthanol et d'ammoniaque à 28 %, suivie d'une cristallisation dans l'acétate d'éthyle, on obtient 0,405 g de produit pur sous forme de solide blanc.

LC-MS : M+H = 371

PF(°C) : 126-128°C

RMN ¹H (CDCl₃) δ (ppm) : 1,20-1,60 (massif, 5H) ; 1,85 (large d, 2H) ; 2,85 (d, 3H) ; 3,0 (large t, 2H) ; 3,30 (large q, 2H) ; 4,55 (large d, 2H) ; 4,60 (s, 2H) ; 4,85 (large s, 1H) ; 6,10 (large s, 1H) ; 7,0 (d, 1H) ; 7,20 (t, 1H) ; 7, 55 (m, 2H) ; 7,70 (d, 1H) ; 7,90 (d, 1H) .

### Exemple 9 (Composé N°127)

### 2-[1-(2-quinolinyl)-4-pipéridinyl]éthylcarbamate de 2-amino-2-oxoéthyle

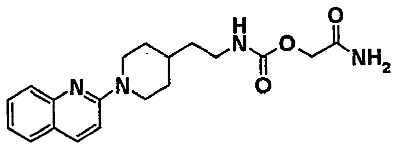

On procède suivant le mode opératoire décrit dans l'exemple 3 (étape 3.4.). A partir de 1,14 g (3,36 mmoles) de 3(2-[1-(2-quinolinyl)-4-pipéridinyl]éthyl}-1,3-oxazolidine-2,4-dione, décrit dans l'exemple 8 (étape 8.2.), et de 9,60 ml (67,20 mmoles) d'une solution d'ammoniaque (7M) dans le méthanol, et après chromatographie sur gel de silice en éluant avec un mélange 95/5 de dichlorométhane et de méthanol, suivie d'une recristallisation dans l'acétate d'éthyle, on obtient 0,360 g de produit pur sous forme de solide blanc.

LC-MS . M+H = 357

PF(°C) : 135-137°C

RMN ¹H (CDCl₃) δ (ppm) : 1,15-1,70 (massif, 5H) ; 1,85 (large d, 2H) ; 2,95 (t, 2H) ; 3,35 (q, 2H) ; 4,55 (large d, 2H) ; 4, 60 (s, 2H) ; 4,85 (large s, 1H) ; 5,55 (large s, 1H) ; 6,05 (large s, 1H) ; 7,0 (d, 1H) ; 7,20 (t, 1H) ; 7,55 (t, 1H) ; 7,60 (d, 1H) ; 7,70 (d, 1H) ; 7,90 (d, 1H).

### Exemple 10 (Composé n°137)

### 2-[1-(6-chloro-2-quinolinyl)-4-pipéridinyl]éthylcarbamate de 2-amino-2-oxoéthyle

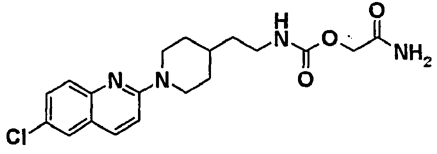

10.1. 2-[1-(6-chloro-2-quinolinyl)-4-pipéridinyl]éthanol On procède comme décrit dans l'exemple 4 (étape 4.1.). A partir de 2 g (10,10 mmoles) de 2,6-dichloroquinoline et de 1,44 g (11, 10 mmoles) de 2-(4-pipéridinyl)éthanol, et après chromatographie sur gel de silice en éluant avec un mélange 98/2 de dichlorométhane et de méthanol, on obtient 2,54 g de produit pur sous forme de solide blanc.

### 10.2. méthanesulfonate de 2-[1-(6-chloro-2-quinolinyl)-4-pipéridinyl]éthyle

On procède comme décrit dans l'exemple 3 (étape 3.2.). A partir de 2,49 g (8,56 mmoles) de 2-[1-(6-chloro-2-quinolinyl)-4-pipéridinyl]éthanol, obtenu à l'étape 10.1., de 1,08 g (9,42 mmoles) de chlorure de mésyle et de 1,81 ml (12,84 mmoles) de triéthylamine, on obtient 3,10 g de produit sous forme d'huile utilisé tel quel dans l'étape suivante.

### 10.3. 3-{2-[1-(6-chloro-2-quinolinyl)-4-pipéridinyl]éthyl}-1,3-oxazolidine-2,4-dione

On procède suivant la méthode décrite dans l'exemple 3 (étape 3.3.). A partir de 3 g (8,13 mmoles) de méthanesulfonate de 2-[1-(6-chloro-2-quinolinyl)-4-pipéridinyl]éthyle, obtenu à l'étape 10.2., de 1,09 g (10,8 mmoles) de 1,3-oxazolidin-2,4-dione et de 2,30 ml (18 mmoles) de 1,1,3,3-tétraméthylguanidine, et après chromatographie sur gel de silice en éluant avec un mélange 98/2 de dichlorométhane et de méthanol, , on obtient 2,57 g de produit.

### 10.4. 2-[1-(6-chloro-2-quinolinyl)-4-pipéridinyl]éthylcarbamate de 2-amino-2-oxoéthyle

On procède suivant le mode opératoire décrit dans l'exemple 3 (étape 3.4.). A partir de 1,28 g (3,42 mmoles) de 3-{2-[1-(6-chloro-2-quinolinyl)-4-pipéridinyl]éthyl}1,3-oxazolidine-2,4-dione, obtenu à l'étape 10.3., et de 22,10 ml (154,08 mmoles) d'une solution d'ammoniaque (7M) dans le méthanol, et après cristallisation, dans l'éthanol, on obtient 0,64 g de produit pur sous forme de solide blanc.

LC-MS _{:} M+H = 391

PF(°C) : 189-191°C

RMN ¹H (DMSO) δ (ppm) : 1,10 (m, 2H) ; 1,40 (m, 2H) ; 1,60 (m, 1H) ; 1,80 (large d, 2H) ; 2,90 (large t, 2H) ; 3,05 (m, 2H) ; 4,30 (s, 2H) ; 4,50 (large d, 2H) ; 7,15 (m, 3H) ; 7,25 (d, 1H) ; 7,50 (m, 2H) ; 7,75 (d, 1H) ; 7,95 (d, 1H) .

### Exemple 11 (Composé N°166)

### 2-[1-(3-isoquinolinyl)-4-pipéridinyl]éthylcarbamate de 2-(méthylamino)-2-oxoéthyle

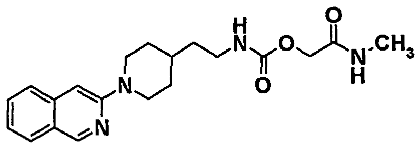

### 11.1. -[1-(3-isoquinolinyl)-4-pipéridinyl]éthanol

On procède comme décrit dans l'exemple 4 (étape 4.1.). A partir de 1 g (6,11 mmoles) de 3-chloroisoquinoline et de 0,869 g (6,72 mmoles) de 2-(4-pipéridinyl)éthanol, et après chromatographie sur gel de silice en éluant avec un mélange 98/2 de dichlorométhane et de méthanol, on obtient 0,34 g de produit pur sous forme d'huile.

### 11.2. méthanesulfonate de 2-[1-(3-isoquinolinyl)-4-pipéridinyl]éthyle

On procède comme décrit dans l'exemple 3 (étape 3.2.). A partir de 0,34 g (1,33 mmoles) de 2-[1-(3-isoquinolinyl)-4-pipéridinyl]éthanol, obtenu à l'étape 11.1., de 0,18 g (1,59 mmoles) de chlorure de mésyle et de 0,30 ml (1,99 mmoles) de triéthylamine, on obtient 0,44 g de produit sous forme d'huile utilisé tel quel dans l'étape suivante.

### 11.3. 3-{2-[1-(3-isoquinolinyl)-4-pipéridinyl]éthyl}-1,3-oxazolidine-2,4-dione

On procède suivant la méthode décrite dans l'exemple 3 (étape 3.3.). A partir de 0,44 g (1,32 mmoles) de méthanesulfonate de 2-[1-(3-isoquinolinyl)-4-pipéridinyl]éthyle, obtenu à l'étape 11.2., de 0,16 g (1,58 mmoles) de 1,3-oxazolidin-2,4-dione et de 0,30 g (2,63 mmoles) de 1,1,3,3-tétraméthylguanidine, et après chromatographie sur gel de silice en éluant avec un mélange 98/2 de dichlorométhane et de méthanol, on obtient 0,25 g de produit.

### 11.4. 2-[1-(3-isoquinolinyl)-4-pipéridinyl]éthylcarbamate de 2-(méthylamino)-2-oxoéthyle

On procède suivant le mode opératoire décrit dans l'exemple 1 (étape 1.5.). A partir de 0,24g (0,71 mmole) de 3-12-[l-(3-isoquinolinyl)-4-pipéridinyl]éthyl}-1,3-oxazolidine-2,4-dione, obtenu à l'étape 11.3., et de 1,8 ml (3,53 mmoles) d'une solution de méthylamine (2M) dans le tétrahydrofurane, et après chromatographie sur gel de silice en éluant avec un mélange 98/2 puis 96/4 de dichlorométhane et de méthanol, suivie d'une cristallisation dans le diisopropyléther, on obtient 0,16 g de produit pur sous forme de solide blanc.

LC-MS : M+H = 371

PF(°C) : 156-158°C

RMN ¹H (CDCl₃) δ (ppm) : 1,20-1,70 (massif, 5H) ; 1,85 (d, 2H) ; 2,90 (m, 5H) ; 3,30 (q, 2H) ; 4,40 (d, 2H) ; 4,60 (s, 2H) ; 4,85 (large s, 1H) ; 6,10 (large s, 1H) ; 6,80 (s, 1H) ; 7,30 (m, 1H) ; 7,60 (m, 2H) ; 7,80 (d, 1H) ; 8,95 (s, 1H).

### Exemple 12 (Composé N°128)

### 2-[4-fluoro-l-(2-quinolinyl)-4-pipéridinyl]éthylcarbamate de 2-(méthylamino)-2-oxoéthyle

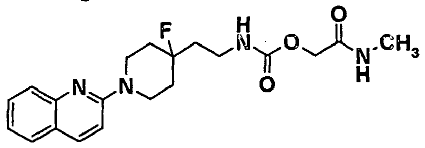

### 12.1. 4-hydroxy-4-(2-hydroxyéthyl)-1-pipéridine carboxylate de tert-butyle

A une suspension de 4,12 g (108,50 mmoles) d'hydrure de lithium et d'aluminium dans 150 ml de tétrahydrofurane, on additionne goutte à goutte une solution de 31,20 g (108,50 mmoles) de 4-(2-éthoxy-2-oxoéthyl)-4-hydroxy-1-pipéridinecarboxylate de *tert*-butyle (WO0216352) dans 150 ml de tétrahydrofurane. On laisse sous agitation à température ambiante pendant 2 heures puis on procède comme décrit dans l'exemple 2 (étape 2.4.).

On obtient 26 g de produit sous forme d'huile jaune utilisé tel quel dans l'étape suivante.

### 12.2. 4-(2-{[tert-butyl(diphényl)silyl]oxy}éthyl)-4-hydroxy-1-pipéridinecarboxylate de tert-butyle

A une solution de 18,4 g (75 mmoles) de 4-hydroxy-4-(2-hydroxyéthyl)-1-pipéridinecarboxylate de *tert*-butyle, obtenu à l'étape 12.1., et de 11,60 ml (82,50 mmoles) de triéthylamine dans 100 ml de dichlorométhane, refroidie à environ 0°C, on ajoute goutte à goutte, sous atmosphère inerte, une solution de 21,50 ml (82,50 mmoles) de chlorure de tert-butyl(diphényl)silyle dans 15 ml de dichlorométhane. On laisse revenir à température ambiante puis on poursuit l'agitation pendant 12 heures. On ajoute au milieu réactionnel une solution aqueuse saturée en chlorure d'ammonium. On sépare la phase aqueuse, on l'extrait deux fois avec du dichlorométhane, on lave les phases organiques réunies avec une solution aqueuse saturée en chlorure de sodium, on les sèche sur sulfate de sodium et on concentre le filtrat sous pression réduite. On purifie le résidu ainsi obtenu par chromatographie sur gel de silice en éluant avec un mélange 10/90 puis 20/80 d'acétate d'éthyle et de cyclohexane.

On obtient ainsi 33,48 g de produit sous forme d'huile jaune.

### 12.3. 4-(2-{[tert-butyl(diphényl)silyl]oxy}éthyl)-4-fluoro-1-pipéridinecarboxylate de tert-butyle

A une solution de 5 g (10,34 mmoles) de 4-(2-{[*tert*-butyl-(diphényl)silyl]oxy}éthyl)-4-hydroxy-1-pipéridine carboxylate de *tert*-butyle obtenu à l'étape 12.2., dans 100 ml de dichlorométhane, refroidie à environ 0°C, on ajoute goutte à goutte, sous atmosphère inerte, une solution de 1,70ml (13,40 mmoles) de 1,1'-[(trifluoro-λ⁴-sulfanyl)imino]-diéthane (DAST) dans 10 ml de dichlorométhane. On laisse revenir à température ambiante puis on poursuit l'agitation pendant 12 heures. On ajoute au milieu réactionnel une solution aqueuse saturée en hydrogénocarbonate de sodium. On sépare la phase aqueuse, on l'extrait trois fois avec du dichlorométhane, on lave les phases organiques réunies avec une solution aqueuse saturée en chlorure de sodium et on les sèche sur sulfate de sodium. On concentre le filtrat sous pression réduite puis on purifie le résidu ainsi obtenu par chromatographie sur gel de silice en éluant avec un mélange 10/90 d'acétate d'éthyle et de cyclohexane. On obtient ainsi 4,65 g de produit sous forme d'huile orange. A une solution de 4,50 g de ce résidu huileux, de 1,25 g (10,7 mmoles) de l'oxyde de *N*-méthyl-morpholine (NMO) dans un mélange de 8 ml d'acétone et de 6 ml d'eau, on ajoute à température ambiante 0,60 ml d'une solution de tetroxyde d'osmium (2,5 %) dans le tert-butanol. On poursuit l'agitation pendant 21 heures. On reprend le résidu avec de l'acétate d'éthyle et de l'eau, on sépare la phase aqueuse, on l'extrait deux fois avec de l'acétate d'éthyle, on lave les phases organiques réunies avec une solution aqueuse saturée en chlorure de sodium et on les sèche sur sulfate de sodium. Après évaporation du solvant, le résidu obtenu est purifié par chromatographie sur gel de silice en éluant avec un mélange 6/94 d'acétate d'éthyle et de cyclohexane.

On obtient ainsi 3,40 g de produit sous forme d'huile jaune pâle.

### 12.4. 4-(2-{[tert-butyl(diphényl)silyl]oxy}éthyl)-4-fluoropipéridine

A une solution de 3 g (6,17 mmoles) de 4-(2-{[*tert*-butyl-(diphényl)silyl]oxy}éthyl)-4-fluoro-1-pipéridinecarboxylate de *tert*-butyle, obtenu à l'étape 12.3., dans 20 ml de dichlorométhane, on ajoute lentement 2,80 ml (37,06 mmoles) d'acide trifluoroacétique. On poursuit l'agitation à température ambiante pendant 5 heures. On verse le mélange réactionnel dans un mélange d'eau glacée et d'ammoniaque à 28 %. On décante, on extrait deux fois la phase aqueuse par du dichlorométhane, on lave les phases organiques réunies avec une solution aqueuse saturée en chlorure de sodium, on les sèche sur sulfate de sodium et on concentre sous pression réduite.

On obtient 2,30 g de produit sous forme d'huile jaune utilisé tel quel dans l'étape suivante.

### 12.5. 2-[4-(2-{[tert-butyl(diphényl)silyl]oxy}éthyl)-4-fluoro-1-pipéridinyl]quinoline

On procède comme décrit dans l'exemple 2 (étape 2.1.). A partir de 1,18 g (5,68 mmoles) de 2-bromoquinoline (Eur. J. Org. Chem. 2002, 4181-4184), de 2,30 g (5,98 mmoles) de 4-(2-{[*tert*-butyl(diphényl)silyl]oxy)éthyl)-4-fluoropipéridine, obtenu à l'étape 12.4., de 0,66 g (6,81 mmoles) de tert-butylate de sodium, de 0,149 g (0,239 mmole) de BINAP et de 0,074 g (0, 081 mmole) de dipalladium tris (dibenzylidène-acétone), et après chromatographie sur gel de silice en éluant avec un mélange 10/90 d'acétate d'éthyle et de cyclohexane, on obtient 2,15 g de produit pur sous forme d'huile orange.

### 12.6. 2-[4-fluoro-1-(2-quinolinyl)-4-pipéridinyl]éthanol

A une solution de 2,15 g (4,19 mmoles) de 2-[4-(2-{[*tert-*butyl(diphényl)silyl]oxy}éthyl)-4-fluoro-1-pipéridinyl] quinoline, obtenu à l'étape 12.5., dans 20 ml de tétrahydrofurane, on ajoute 0,40 g (1,26 mmoles) de fluorure de n-tétrabutylammonium trihydrate. On poursuit l'agitation à température ambiante pendant 4 heures. On concentre à sec puis on purifie le résidu obtenu par chromatographie sur gel de silice en éluant avec un mélange 35/65 puis 40/60 d'acétate d'éthyle et de cyclohexane.

On obtient 0,61 g de produit sous forme d'huile orange.

### 12.7. méthanesulfonate de 2-[4-fluoro-1-(2-quinolinyl)-4-pipéridinyl]éthyle

On procède comme décrit dans l'exemple 3 (étape 3.2.). A partir de 0,61 g (2,22 mmoles) de 2-[4-fluoro-1-(2-quin linyl)-4-pipéridinyl]éthanol, obtenu à l'étape 12.6., de 0,280 g (2,45 mmoles) de chlorure de mésyle et de 0,35 ml (2,45 mmoles) de triéthylamine, on obtient 0,80 g de produit sous forme d'huile orange utilisé tel quel dans l'étape suivante.

### 12.8. 3-{2-[4-fluoro-1-(2-quinolinyl)-4-pipéridinyl]éthyl}-1,3-oxazolidine-2,4-dione

On procède suivant la méthode décrite dans l'exemple 3 (étape 3.3.). A partir de 0,780 g (2,22 mmoles) de méthanesulfonate de 2-[4-fluoro-1-(2-quinolinyl)-4-pipéridinyl]éthyle, obtenu à l'étape 12.7., de 0,27 g (2,66 mmoles) de 1,3-oxazolidin-2,4-dione et de 0,51 g (4,43 mmoles) de 1,1,3,3-tétraméthylguanidine, et après chromatographie sur gel de silice en éluant avec un mélange 99/1 de dichlorométhane et de méthanol, on obtient 0,520 g de produit pur sous forme de solide beige.

### 12.9: 2-[4-fluoro-1-(2-quinolinyl)-4-pipéridinyl]éthylcarbamate de 2-(méthylamino)-2-oxoéthyle

On procède suivant le mode opératoire décrit dans l'exemple 1 (étape 1.5.). A partir de 0,52 g (1,46 mmoles) de 3-{2-[4-fluoro-1-(2-quinolinyl)-4-pipéridinyl]éthyl}-1,3-oxazolidine-2,4-dione, obtenu à l'étape 12.8., et de 3,6 ml (7,28 mmoles) d'une solution de méthylamine (2M) dans le tétrahydrofurane, et après chromatographie sur gel de silice en éluant avec un mélange 99/1 d'acétate d'éthyle et de méthanol, suivie d'une cristallisation dans le diéthyléther, on obtient 0,390 g de produit pur sous forme de solide blanc.

LC-MS : M+H = 389

PF(°C) : 147-149°C

RMN ¹H (CDCl₃) δ (ppm) : 1,70-2,10 (massif, 6H) ; 2,90 (d, 3H) ; 3,40 (large t, 2H) ; 3,50 (q, 2H) ; 4,40 (large d, 2H) ; 4,60 (s, 2H) ; 5,15 (large s, 1H) ; 6,15 (large s, 1H) ; 7,05 (d, 1H) ; 7,25 (t, 1H) ; 7,55 (t, 1H) ; 7,65 (d, 1H) ; 7,75 (d, 1H) ; 7,95 (d, 1H) .

### Exemple 13 (Composé N°49)

### 2-[1-(6-isobutyl-2-pyridinyl)-4-pipéridinyl]éthylcarbamate de 2-(méthylamino)-2-oxoéthyle

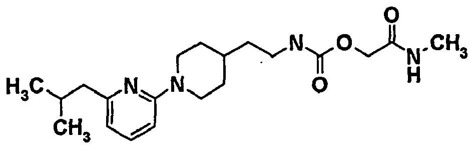

### 13.1. 2-[1-(6-bromo-2-pyridinyl)-4-piperidinyl]ethanol

On procède comme décrit dans l'exemple 4 (étape 4.1.). A partir de 30,20 g (127 mmoles) de 2,6-dibromopyridine et de 16,45 g (127 mmoles) de 2-(4-pipéridinyl)éthanol, et après chromatographie sur gel de silice en éluant avec un mélange 30/70 d'acétate d'éthyle et de cyclohexane, on obtient 7 g de produit pur sous forme d'huile,.

### 13.2. méthanesulfonate de 2-[1-(6-bromo-2-pyridinyl)-4-pipéridinyl]éthyle

On procède comme décrit dans l'exemple 3 (étape 3.2.). A partir de 7 g (24,50 mmoles) de 2-[1-(6-bromo-2-pyridinyl)-4-pipéridinyl]éthanol, obtenu à l'étape 13.1., de 2,50 ml (26,90 mmoles) de chlorure de mésyle et de 3,80 ml (26,90 mmoles) de triéthylamine, on obtient 8,68 g de produit sous forme d'huile utilisé tel quel dans l'étape suivante.

### 13.3. 3-{2-[1-(6-bromo-2-pyridinyl)-4-pipéridinyl]éthyl}-1,3-oxazolidine-2,4-dione

On procède suivant la méthode décrite dans l'exemple 3 (étape 3.3.). A partir de 8,68 g (23,80 mmoles) de méthanesulfonate de 2-[1-(6-bromo-2-pyridinyl)-4-pipéridinyl]éthyle, obtenu à l'étape 13.2., de 2,90 g (28,60 mmoles) de 1,3-oxazolidin-2,4-dione et de 6 ml (47,60 mmoles) de 1,1,3,3-tétraméthylguanidine, et après chromatographie sur gel de silice en éluant avec un mélange 97/3 de dichlorométhane et de méthanol, on obtient 4,52 g de produit sous forme d'huile.

### 13.4. 3-{2-[1-(6-isobutyl-2-pyridinyl)-4-pipéridinyl]éthyl}-1,3-oxazolidine-2,4-dione

Sous atmosphère inerte, on introduit 2 g (5,43 mmoles) de 3-{2-[1-(6-bromo-2-pyridinyl)-4-pipéridinyl]éthyl}-1,3-oxazolidine-2,4-dione, préparé à l'étape 13.3., et 0,20 g (0,271 mmole) de palladium dichloro bis(triphénylphosphine) (Pd(PPh₃)₂Cl₂) en suspension dans 10 ml de tétrahydrofurane. On ajoute ensuite 22 ml (10,80 mmoles) d'une solution de bromo(isobutyl)zinc (0,5M) dans le tétrahydrofurane. On poursuit l'agitation à température ambiante pendant 17 heures. On verse le mélange réactionnel dans de l'eau et de l'acétate d'éthyle. On décante, on extrait deux fois la phase aqueuse par de l'acétate d'éthyle, on sèche les phases organiques réunies sur sulfate de sodium et on concentre le filtrat sous pression réduite. On purifie le résidu ainsi obtenu par chromatographie sur gel de silice en éluant avec un mélange 20/80 d'acétate d'éthyle et de cyclohexane.

On obtient 1,41 g de produit sous forme de solide blanc. PF (°C) _{:} 94-96°C

### 13.5. 2-[1-(6-isobutyl-2-pyridinyl)-4-pipéridinyl]éthylcarbamate de 2-(méthylamino)-2-oxoéthyle

On procède suivant le mode opératoire décrit dans l'exemple 1 (étape 1.5.). A partir de 0,72 g (2,08 mmoles) de 3-{2-[1-(6-isobutyl-2-pyridinyl)-4-pipéridinyl]éthyl}-1,3-oxazolidine-2,4-dione, obtenu à l'étape 13.4., et de 5,20 ml (10,40 mmoles) d'une solution de méthylamine (2M) dans le tétrahydrofurane, et après chromatographie sur gel de silice en éluant avec un mélange 95/5/0, 5 de dichlorométhane, de méthanol et d'ammoniaque à 28 %, suivie d'une cristallisation dans le diisopropyléther, on obtient 0,540 g de produit pur sous forme de solide blanc.

LC-MS : M+H = 377

PF(°C) : 97-99°C

RMN ¹H (DMSO) δ (ppm) : 0,85 (d, 6H) ; 1,05 (m, 2H) ; 1,20-1,60 (massif, 3H) ; 1,70 (large d, 2H) ; 2,0 (m, 1H) ; 2,40 (d, 2H) ; 2,55 (d, 3H) ; 2,70 (large t, 2H) ; 3,05 (large q, 2H) ; 4,20 (large d, 2H) ; 4,30 (s, 2H) ; 6,35 (d, 1H) ; 6,55 (d, 1H) ; 7,15 (large t, 1H) ; 7,40 (dd, 1H) ; 7,75 (large s, 1H).

### Exemple 14 (Composé N°58)

### 2-[1-(6-phényl-2-pyridinyl)-4-pipéridinyl]méthylcarbamate de 2-amino-2-oxoéthyle

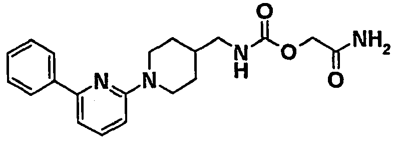

### 14.1. 3-{2-[1-(6-phényl-2-pyridinyl)-4-pipéridinyl]-méthyl}-1,3-oxazolidine-2,4-dione

Sous atmosphère inerte, on introduit 0,20 g (0,56 mmole) de 3-{2-[1-(6-bromo-2-pyridinyl)-4-pipéridinyl]méthyl}-1,3-oxazolidine-2,4-dione, préparé selon le mode opératoire décrit dans l'exemple 13 (étapes 13.1., 13.2. et 13.3.), 0,089 g (0,73 mmoles) d'acide phénylboronique et 0,480 g (2,25 mmoles) de phosphate de potassium hydraté en suspension dans 3 ml de 1,2-diméthoxyéthane. On ajoute ensuite 0,040 g (0,0346 mmole) de palladium tétrakis(triphénylphosphine). On porte ensuite le mélange réactionnel à environ 85°C pendant 16 heures. On concentre sous pression réduite. On reprend le résidu avec de l'acétate d'éthyle et de l'eau, on sépare la phase aqueuse, on l'extrait 2 fois avec de l'acétate d'éthyle, on sèche les phases organiques réunies sur sulfate de sodium et on concentre le filtrat sous pression réduite. On purifie le résidu ainsi obtenu par chromatographie sur gel de silice en éluant avec un mélange 40/60 d'acétate d'éthyle et de cyclohexane.

On obtient 0,175 g de produit.

### 14.2. 2-[1-(6-phényl-2-pyridinyl)-4-pipéridinyl]méthylcarbamate de 2-amino-2-oxoéthyle

On procède suivant le mode opératoire décrit dans l'exemple 3 (étape 3.4.). A partir de 0,175 g (0,499 mmole) de 3-{2-[1-(6-phényl-2-pyridinyl)-4-pipéridinyl]méthyl}-1,3-oxazolidine-2,4-dione, obtenu à l'étape 14.1., et de 2,5 ml (17, 45 mmoles) d'une solution d'ammoniaque (7M) dans le méthanol, on obtient 0,070 g de produit pur sous forme de solide blanc, après cristallisation dans l'acétate d'éthyle.

LC-MS _{:} M+H = 369

PF(°C) _{:} 131-132°C

RMN ¹H (CDCl₃) δ (ppm) : 1,20-1,90 (massif, 5H) ; 2,90 (large t, 2H) ; 3,20 (t, 2H) ; 4,50 (large d, 2H) ; 4,60 (s, 2H) ; 5,0 (large s, 1H) ; 5,55 (large s, 1H) ; 6,15 (large s, 1H) ; 6,65 (d, 1H) ; 7,10 (d, 1H) ; 7,35-7,60 (m, 4H) ; 8,15 (dd, 2H).

### Exemple 15 (Composé N°130)

### 2-[1-(5-chloro-2-quinolinyl)-4-pipéridinyl]éthylcarbamate de 2-(méthylamino)2-oxoéthyle

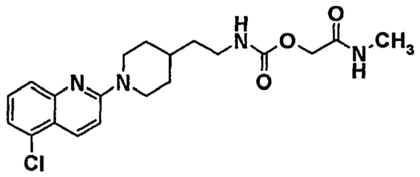

### 15.1. 2-[1-(5-chloro-2-quinolinyl)-4-pipéridinyl]éthanol

On procède comme décrit dans l'exemple 4 (étape 4.1.). A partir de 4,78 g (24,14 mmoles) de 2-chloro-5-chloroquinoline (J. Med. Chem., 2002, 45, 3130-3137) et de 3,43 g (26,55 mmoles) de 2-(4-pipéridinyl)éthanol, on obtient 7 g de produit sous forme d'huile utilisé tel quel dans l'étape suivante.

### 15.2. méthanesulfonate de 2-[1-(5-chloro-2-quinolinyl)-4-pipéridinyl]éthyle

On procède comme décrit dans l'exemple 3 (étape 3.2.). A partir de 7 g (24,07 mmoles) de 2-[1-(5-chloro-2-quinolinyl)-4-pipéridinyl]éthanol, obtenu à l'étape 15.1., de 3,31 g (28,89 mmoles) de chlorure de mésyle et de 5,10 ml (36,11 mmoles) de triéthylamine, on obtient 8,70 g de produit sous forme d'huile utilisé tel quel dans l'étape suivante.

### 15.3. 3-{2-[1-(5-chloro-2-quinolinyl)-4-pipéridinyl]éthyl}-1,3-oxazolidine-2,4-dione

On procède suivant la méthode décrite dans l'exemple 3 (étape 3.3.). A partir de 8,70 g (23,58 mmoles) de méthanesulfonate de 2-[1-(5-chloro-2-quinolinyl)-4-pipéridinyl]éthyle, obtenu à l'étape 15.2., de 2,86 g (28,30 mmoles) de 1,3-oxazolidin-2,4-dione et de 5,43 g (47,17 mmoles) de 1,1,3,3-tétraméthylguanidine, et après chromatographie sur gel de silice en éluant avec un mélange 99,5/0,5 de dichlorométhane et de méthanol, on obtient 6,40 g de produit sous forme de solide blanc.

PF(°C) : 136°C

### 15.4. 2-[1-(5-chloro-2-quinolinyl)-4-pipéridinyl]éthylcarbamate de 2-(méthylamino)-2-oxoéthyle

On procède suivant le mode opératoire décrit dans l'exemple 1 (étape 1.5.). A partir de 6,40 g (17,12 mmoles) de 3-{2-[1-(5-chloro-2-quinolinyl)-4-pipéridinyl]éthyl}-1,3-oxazolidine-2,4-dione, obtenu à l'étape 15.3., et de 60 ml (119,84 mmoles) d'une solution de méthylamine (2M) dans le tétrahydrofurane, et après chromatographie sur gel de silice en éluant avec un mélange 98/2 puis 96/4 de dichlorométhane et de méthanol, suivie d'une cristallisation dans le diisopropyléther, on obtient 5,14 g de produit sous forme de solide blanc.

LC-MS : M+H = 405

PF(°C) : 158-162°C

RMN ¹H (CDCl₃) δ (ppm) : 1,10-1,80 (massif, 5H) ; 1,9 (large d, 2H) ; 2,90 (d, 3H) ; 3,0 (m, 2H) ; 3,30 (q, 2H) ; 4,60 (m, 4H) ; 4,85 (large s, 1H) ; 6,10 (large s, 1H) ; 7,05 (d, 1H) ; 7,25 (d, 1H) ; 7,40 (dd, 1H) ; 7,60 (d, 1H) ; 8,30 (s, 1H) .

Le tableau 1 qui suit illustre les structures chimiques et les propriétés physiques de quelques composés selon l'invention.

Dans ce tableau :
- dans la colonne « base ou sel », « base » représente un composé sous forme de bas libre, alors que « HCl » représente un composé sous forme de chlorhydrate ;
- OMe représente un groupe méthoxy.
   Tableau 1

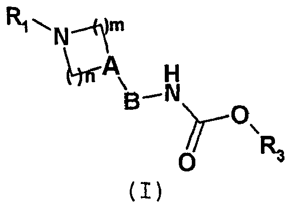

| **N°** | **R₁** | **m** | **n** | **A** | **B** | **R₃** | **base ou sel** | **PF (°C)** |
|---|---|---|---|---|---|---|---|---|
| 1. | 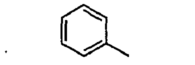 | 2 | 2 | N | (CH₂)₃ | CH₂CONHCH₃ | base | 131 |
| 2. | 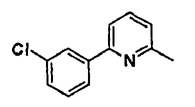 | 2 | 2 | N | (CH₂)₂ | CH₂CONH₂ | base | 417* |
| 3. | | 2 | 2 | N | (CH₂)₂ | CH₂CONHCH₃ | base | 165-166 |
| 4. | 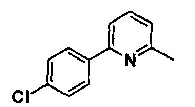 | 2 | 2 | N | (CH₂)₂ | CH₂CONH₂ | base | 144-145 |
| 5. | | 2 | 2 | N | (CH₂)₂ | CH₂CONHCH₃ | base | 138-139 |
| 6. | 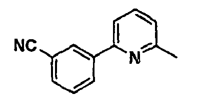 | 2 | 2 | N | (CH₂)₂ | CH₂CONH₂ | base | 173-174 |
| 7. | | 2 | 2 | N | (CH₂)₂ | CH₂CONHCH₃ | base | 196-197 |
| 8. | 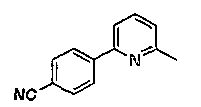 | 2 | 2 | N | ((CH₂)₂ | CH₂CONH₂ | base | 157-158 |
| 9. | | 2 | 2 | N | (CH₂)₂ | CH₂CONHCH₃ | base | 225-226 |
| 10. | 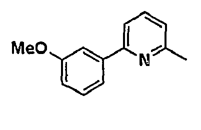 | 2 | 2 | N | (CH₂)₂ | CH₂CONH₂ | base | 121-122 |
| 11. | | 2 | 2 | N | (CH₂)₂ | CH₂CONHCH₃ | base | 141-142 |
| 12. | 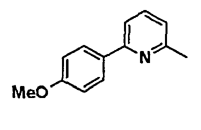 | 2 | 2 | N | (CH₂)₂ | CH₂CONH₂ | base | 139-140 |
| 13. | | 2 | 2 | N | (CH₂)₂ | CH₂CONHCH₃ | base | 170-171 |
| 14. | 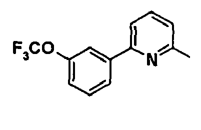 | 2 | 2 | N | (CH₂)₂ | CH₂CONH₂ | base | 128-129 |
| 15. | | 2 | 2 | N | (CH₂)₂ | CH₂CONHCH₃ | base | 153-154 |
| 16. | 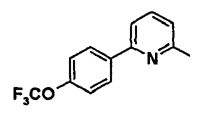 | 2 | 2 | N | (CH₂)₂ | CH₂CONH₂ | base | 160-161 |
| 17. | | 2 | 2 | N | (CH₂)₂ | CH₂CONHCH₃ | base | 148-149 |
| 18. | 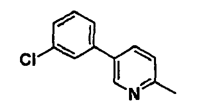 | 2 | 2 | N | (CH₂)₂ | CH₂CONH₂ | base | 156-157 |
| 19. | | 2 | 2 | N | (CH₂)₂ | CH₂CONHCH₃ | base | 157-158 |
| 20. | 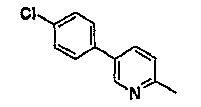 | 2 | 2 | N | (CH₂)₂ | CH₂CONH₂ | base | 178-179 |
| 21. | | 2 | 2 | N | (CH₂)₂ | CH₂CONHCH₃ | base | 432* |
| 22. | 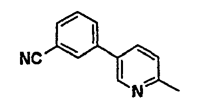 | 2 | 2 | N | (CH₂)₂ | CH₂CONH₂ | base | 158-159 |
| 23. | | 2 | 2 | N | (CH₂)₂ | CH₂CONHCH₃ | base | 180-181 |
| 24. | 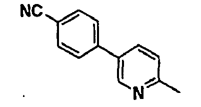 | 2 | 2 | N | (CH₂)₂ | CH₂CONH₂ | base | 185-186 |
| 25. | | 2 | 2 | N | (CH₂)₂ | CH₂CONHCH₃ | base | 193-194 |
| 26. | 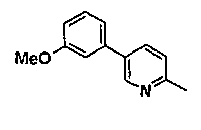 | 2 | 2 | N | (CH₂)₂ | CH₂CONH₂ | base | 153-154 |
| 27. | | 2 | 2 | N | (CH₂)₂ | CH₂CONHCH₃ | base | 125-126 |
| 28. | 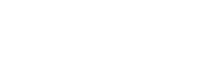 | 2 | 2 | N | (CH₂)₂ | CH₂CONH₂ | base | 193-194 |
| 29. | 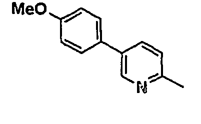 | 2 | 2 | N | (CH₂)₂ | CH₂CONHCH₃ | base | 176-177 |
| 30. | 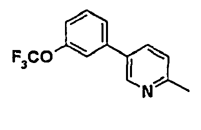 | 2 | 2 | N | (CH₂)₂ | CH₂CONH₂ | base | 160-161 |
| 31. | | 2 | 2 | N | (CH₂)₂ | CH₂CONHCH₃ | base | 156-157 |
| 32. | 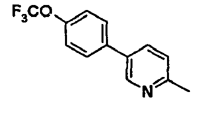 | 2 | 2 | N | (CH₂)₂ | CH₂CONH₂ | base | 189-190 |
| 33. | | 2 | 2 | N | (CH₂)₂ | CH₂CONHCH₃ | base | 196-197 |
| 34. | 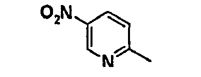 | 2 | 3 | N | (CH₂)₃ | CH₂CONHCH₃ | base | 100 (déc.) |
| 35. | 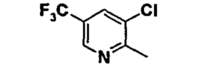 | 2 | 3 | N | (CH₂)₃ | CH₂CONHCH₃ | HCl | 451* |
| 36. | 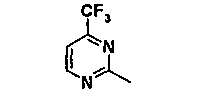 | 2 | 3 | N | (CH₂)₃ | CH₂CONHCH₃ | HCl | 172-174 |
| 37. | 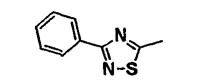 | 2 | 3 | N | (CH₂)₃ | CH₂CONHCH₃ | base | 118-122 |
| 38. | 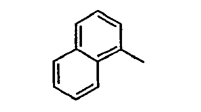 | 2 | 2 | N | (CH₂)₂ | CH₂CONHCH₃ | HCl | 166 |
| 39. | | 2 | 2 | N | (CH₂)₃ | CH₂CONHCH₃ | HCl | 167 |
| 40. | 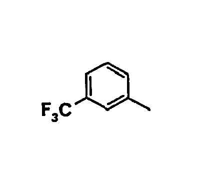 | 1 | 1 | CH | CH₂ | CH₂CONH₂ | base | 149-151 |
| 41. | | 1 | 1 | CH | CH₂ | CH₂CONHCH₃ | base | 137-139 |
| 42. | | 1 | 1 | CH | CH₂ | CH₂CONHCH₂ (cyclopropyle) | base | 119-121 |
| 43. | | 2 | 2 | CH | (CH₂)₂ | CH₂CONHCH₃ | base | 110-112 |
| 44. | | 2 | 2 | CH | (CH₂)₂ | CH₂CONH₂ | base | 140-142 |
| 45. | | 2 | 2 | CF | (CH₂)₂ | CH₂CONHCH₃ | base | 127-128 |
| 46. | 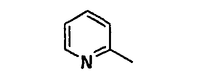 | 2 | 2 | CH | (CH₂)₂ | CH₂CONHCH₃ | HCl | 168-170 |
| 47. | 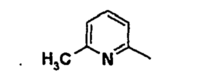 | 2 | 2 | CH | (CH₂)₂ | CH₂CONHCH₃ | HCl | 95-100 |
| 48. | 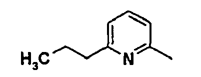 | 2 | 2 | CH | (CH₂)₂ | CH₂CONHCH₃ | base | 113-115 |
| 49. | 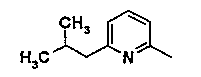 | 2 | 2 | CH | (CH₂)₂ | CH₂CONHCH₃ | base | 97-99 |
| 50. | | 2 | 2 | CH | (CH₂)₂ | CH₂CONH₂ | base | 107-109 |
| 51. | 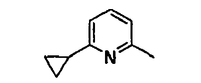 | 2 | 2 | CH | (CH₂)₂ | CH₂CONHCH₃ | base | 108-110 |
| 52. | 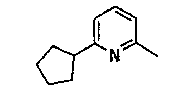 | 2 | 2 | CH | (CH₂)₂ | CH₂CONHCH₃ | base | 98-100 |
| 53. | 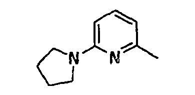 | 2 | 2 | CH | (CH₂)₂ | CH₂CONHCH₃ | base | 123-125 |
| 54. | 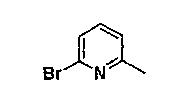 | 2 | 2 | CH | - | CH₂CONH₂ | base | 169 |
| 55. | | 2 | 2 | CH | CH₂ | CH₂CONH₂ | base | 148-149 |
| 56. | | 2 | 2 | CH | (CF2)2 | CH₂CONH₂ | base | 131-132 |
| 57. | 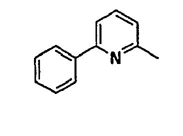 | 2 | 2 | CH | - | CH₂CONH₂ | base | 153 |
| 58. | | 2 | 2 | CH | CH₂ | CH₂CONH₂ | base | 131-132 |
| 59. | | 2 | 2 | CH | (CH₂)₂ | CH₂CONH₂ | base | 116-117 |
| 60. | 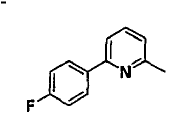 | 2 | 2 | CH | - | CH₂CONH₂ | base | 190-191 |
| 61. | | 2 | 2 | CH | CH₂ | CH₂CONH₂ | base | 155-156 |
| 62. | | 2 | 2 | CH | (CH₂)₂ | CH₂CONH₂ | base | 155-156 |
| 63. | 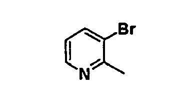 | 2 | 2 | CH | - | CH₂CONH₂ | base | 159-160 |
| 64. | | 2 | 2 | CH | CH₂ | CH₂CONH₂ | base | 132 |
| 65. | | 2 | 2 | CH | (CH₂)₂ | CH₂CONH₂ | base | 134-135 |
| 66. | 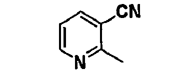 | 2 | 2 | CH | CH₂ | CH₂CONH₂ | base | 146-147 |
| 67. | | 2 | 2 | CH | (CH₂)₂ | CH₂CONH₂ | base | 332* |
| 68. | 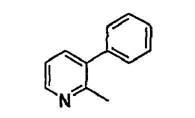 | 2 | 2 | CH | - | CH₂CONH₂ | base | 131-132 |
| 69. | | 2 | 2 | CH | CH₂ | CH₂CONH₂ | base | 129-130 |
| 70. | | 2 | 2 | CH | (CH₂)₂ | CH₂CONH₂ | base | 86-87 |
| 71. | 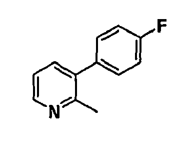 | 2 | 2 | CH | - | CH₂CONH₂ | base | 58-59 |
| 72. | | 2 | 2 | CH | CH₂ | CH₂CONH₂ | base | 387* |
| 73. | | 2 | 2 | CH | (CH₂)₂ | CH₂CONH₂ | base | 58-59 |
| 74. | 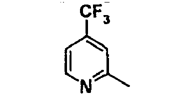 | 2 | 2 | CH | CH₂ | CH₂CONH₂ | base | 152-153 |
| 75. | | 2 | 2 | CH | (CH₂)₂ | CH₂CONH₂ | base | 163 |
| 76. | 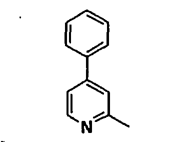 | 2 | 2 | CH | (CH₂)₂ | CH₂CONHCH₃ | base | 121-123 |
| 77. | | 2 | 2 | CH | (CH₂)₂ | CH₂CONH₂ | base | 137-139 |
| 78. | 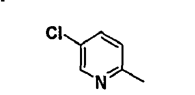 | 2 | 2 | CH | CH₂ | CH₂CONH₂ | base | 327* |
| 79. | | 2 | 2 | CH | (CH₂)₂ | CH₂CONH₂ | base | 162-163 |
| 80. | 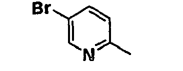 | 2 | 2 | CH | - | CH₂CONH₂ | base | 168 |
| 81. | | 2 | 2 | CH | CH₂ | CH₂CONH₂ | base | 176 |
| 82. | | 2 | 2 | CH | (CH₂)₂ | CH₂CONH₂ | base | 174-175 |
| 83. | 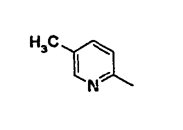 | 2 | 2 | CH | CH₂ | CH₂CONH₂ | base | 169-170 |
| 84. | | 2 | 2 | CH | (CH₂)₂ | CH₂CONH₂ | base | 145-146 |
| 85. | 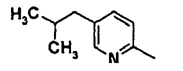 | 2 | 2 | CH | (CH₂)₂ | CH₂CONH₂ | base | 119-121 |
| 86. | | 2 | 2 | CH | (CH₂)₂ | CH₂CONHCH₃ | base | 109-111 |
| 87. | 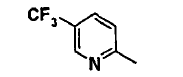 | 2 | 2 | CH | CH₂ | CH₂CONH₂ | base | 169-170 |
| 88. | | 2 | 2 | CH | (CH₂)₂ | CH₂CONH₂ | base | 164-165 |
| 89. | 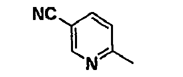 | 2 | 2 | CH | CH₂ | CH₂CONH₂ | base | 174-175 |
| 90. | 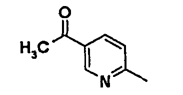 | 2 | 2 | CH | CH₂ | CH₂CONH₂ | base | 162-163 |
| 91. | | 2 | 2 | CH | (CH₂)₂ | CH₂CONH₂ | base | 151-152 |
| 92. | 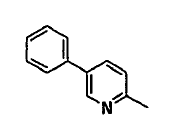 | 2 | 2 | CH | - | CH₂CONH₂ | base | 195-196 |
| 93. | | 2 | 2 | CH | CH₂ | CH₂CONH₂ | base | 229-230 |
| 94. | 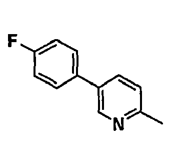 | 2 | 2 | CH | - | CH₂CONH₂ | base | 194-195 |
| 95. | | 2 | 2 | CH | CH₂ | CH₂CONH₂ | base | 222-223 |
| 96. | | 2 | 2 | CH | (CH₂)₂ | CH₂CONH₂ | base | 182 |
| 97. | 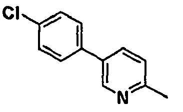 | 2 | 2 | CH | (CH₂)₂ | CH₂CONH₂ | base | 196-199 |
| 98. | | 2 | 2 | CH | (CH₂)₂ | CH₂CONHCH₃ | base | 161-163 |
| 99. | 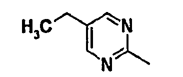 | 2 | 2 | CH | CH₂ | CH₂CONH₂ | base | 157-158 |
| 100. | | 2 | 2 | CH | (CH₂)₂ | CH₂CONH₂ | base | 175-176 |
| 101. | 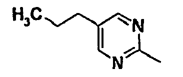 | 2 | 2 | CH | CH₂ | CH₂CONH₂ | base | 163-164 |
| 102. | 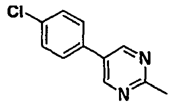 | 2 | 2 | CH | (CH₂)₂ | CH₂CONH₂ | base | 418* |
| 103. | 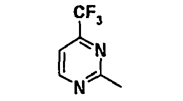 | 2 | 2 | CH | CH₂ | CH₂CONH₂ | base | 190-191 |
| 104. | 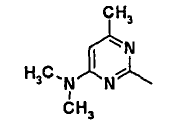 | 2 | 2 | CH | (CH₂)₂ | CH₂CONH₂ | base | 165-166 |
| 105. | 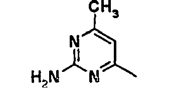 | 2 | 2 | CH | CH₂ | CH₂CONH₂ | base | 323* |
| 106. | | 2 | 2 | CH | (CH₂)₂ | CH₂CONH₂ | base | 202-203 |
| 107. | 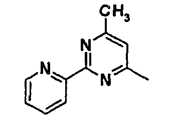 | 2 | 2 | CH | CH₂ | CH₂CONH₂ | base | 172-173 |
| 108. | 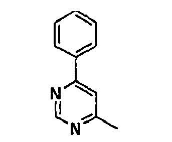 | 2 | 2 | CH | CH₂ | CH₂CONH₂ | basse | 370* |
| 109. | 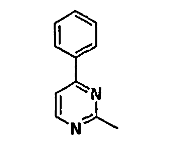 | 2 | 2 | CH | CH₂ | CH₂CONH₂ | base | 177-178 |
| 110. | 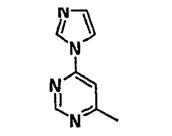 | 2 | 2 | CH | CH₂ | CH₂CONH₂ | base | 202-203 |
| 111. | | 2 | 2 | CH | (CH₂)₂ | CH₂CONH₂ | base | 185-186 |
| 112. | 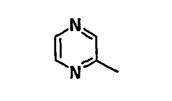 | 2 | 2 | CH | CH₂ | CH₂CONH₂ | base | 165-166 |
| 113. | | 2 | 2 | CH | (CH₂)₂ | CH₂CONH₂ | base | 126-127 |
| 114. | 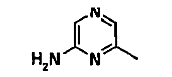 | 2 | 2 | CH | CH₂ | CH₂CONH₂ | base | 309* |
| 115. | | 2 | 2 | CH | (CH₂)₂ | CH₂CONH₂ | base | 159-160 |
| 116. | 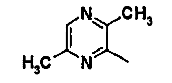 | 2 | 2 | CH | CH₂ | CH₂CONH₂ | base | 161-162 |
| 117. | | 2 | 2 | CH | (CH₂)₂ | CH₂CONH₂ | base | 126-127 |
| 118. | 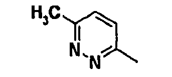 | 2 | 2 | CH | CH₂ | CH₂CONH₂ | base | 158-159 |
| 119. | | 2 | 2 | CH | (CH₂)₂ | CH₂CONH₂ | base | 174-175 |
| 120. | 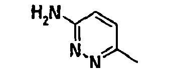 | 2 | 2 | CH | (CH₂)₂ | CH₂CONH₂ | base | 204-205 |
| 121. | 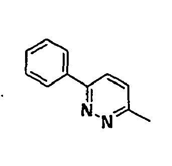 | 2 | 2 | CH | CH₂ | CH₂CONH₂ | base | 229-230 |
| 122. | | 2 | 2 | CH | (CH₂)₂ | CH₂CONH₂ | base | 228-229 |
| 123. | 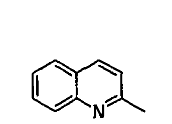 | 2 | 2 | CH | CH₂ | CH₂CONHCH₃ | base | 153-157 |
| 124. | | 2 | 2 | CH | CH₂ | CH₂CONH₂ | base | 206-212 |
| 125. | | 2 | 2 | CH | CH₂ | CH(CH₃)CONHCH₃ | base | 129-130 |
| 126. | | 2 | 2 | CH | (CH₂)₂ | CH₂CONHCH₃ | base | 126-128 |
| 127. | | 2 | 2 | CH | (CH₂)₂ | CH₂CONH₂ | base | 135-137 |
| 128. | | 2 | 2 | CF | (CH₂)₂ | CH₂CONHCH₃ | base | 147-149 |
| 129. | | 2 | 2 | C(OH) | (CH₂)₂ | CH₂CONHCH₃ | base | 82-90 |
| 130. | 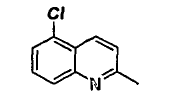 | 2 | 2 | CH | (CH₂)₂ | CH₂CONHCH₃ | base | 158-162 |
| 131. | | 2 | 2 | CH | (CH₂)₂ | CH₂CONH₂ | base | 171-175 |
| 132. | 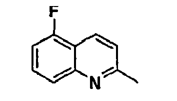 | 2 | 2 | CH | (CH₂)₂ | CH₂CONHCH₃ | base | 162-163 |
| 133. | 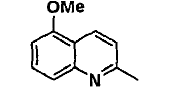 | 2 | 2 | CH | (CH₂)₂ | CH₂CONHCH₃ | base | 154-156 |
| 134. | 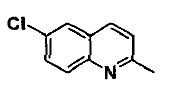 | 2 | 2 | CH | CH₂ | CH₂CONHCH₃ | base | 147-151 |
| 135. | | 2 | 2 | CH | CH₂ | CH₂CONH₂ | base | 207-208 |
| 136. | | 2 | 2 | CH | (CH₂)₂ | CH₂CONHCH₃ | base | 155-157 |
| 137. | | 2 | 2 | CH | (CH₂)₂ | CH₂CONH₂ | base | 189-191 |
| 138. | 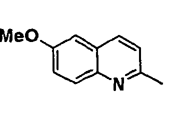 | 2 | 2 | CH | (CH₂)₂ | CH₂CONH₂ | base | 163-165 |
| 139. | | 2 | 2 | CH | (CH₂)₂ | CH₂CONHCH₃ | base | 135-137 |
| 140. | | 2 | 2 | CH | (CH₂)₂ | CH₂CONHCH₂CH₃ | base | 114-116 |
| 141. | | 2 | 2 | CH | (CH₂)₂ | CH₂CONHCH₂ (cyclopropyle) | base | 95-100 |
| 142. | 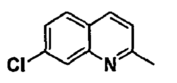 | 2 | 2 | CH | (CH₂)₂ | CH₂CONH₂ | base | 191-193 |
| 143. | | 2 | 2 | CH | (CH₂)₂ | CH₂CONHCH₃ | base | 149-151 |
| 144. | 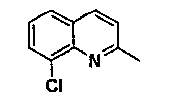 | 2 | 2 | CH | (CH₂)₂ | CH₂CONHCH₃ | base | 146-150 |
| 145. | | 2 | 2 | CH | (CH₂)₂ | CH₂CONH₂ | base | 171-173 |
| 146. | 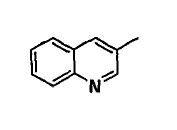 | 2 | 2 | CH | (CH₂)₂ | CH₂CONHCH₃ | base | 140-142 |
| 147. | | 2 | 2 | CH | (CH₂)₂ | CH₂CONH₂ | base | 160-162 |
| 148. | 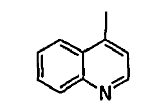 | 2 | 2 | CH | (CH₂)₂ | CH₂CONHCH₃ | HCl | 148-150 |
| 149. | | 2 | 2 | CH | (CH₂)₂ | CH₂CONH₂ | HCl | 219-221 |
| 150. | 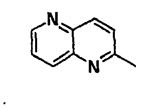 | 2 | 2 | CH | (CH₂)₂ | CH₂CONHCH₃ | base | 130-132 |
| 151. | 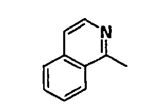 | 1 | 2 | CH | CH₂ | CH₂CONH₂ | base | 109-111 |
| 152. | | 1 | 2 | CH | CH₂ | CH₂CONHCH₃ | base | 99-102 |
| 153. | | 2 | 2 | CH | - | CH₂CONHCH₃ | HCl | 232-237 |
| 154. | | 2 | 2 | CH | CH₂ | CH₂CONHCH₃ | HCl | 208-212 |
| 155. | | 2 | 2 | CH | (CH₂)₂ | CH₂CONHCH₃ | HCl | 132-136 |
| 156. | | 2 | 2 | CH | (CH₂)₂ | CH₂CONH₂ | HCl | 120-124 |
| 157. | | 2 | 2 | CH | (CH₂)₃ | CH₂CONHCH₃ | HCl | 385* |
| 158. | 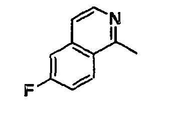 | 2 | 2 | CH | (CH₂)₂ | CH₂CONH₂ | base | 135-139 |
| 159. | | 2 | 2 | CH | (CH₂)₂ | CH₂CONHCH₃ | base | 144-148 |
| 160. | 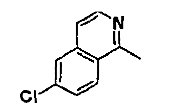 | 2 | 2 | CH | (CH₂)₂ | CH₂CONHCH₃ | base | 134-136 |
| 161. | 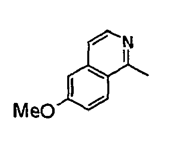 | 2 | 2 | CH | CH₂ | CH₂CONHCH₃ | HCl | 387* |
| 162. | | 2 | 2 | CH | (CH₂)₂ | CH₂CONHCH₃ | base | 100-104 |
| 163. | | 2 | 2 | CH | (CH₂)₂ | CH₂CONH₂ | base | 118-120 |
| 164. | 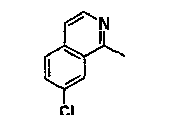 | 2 | 2 | CH | (CH₂)₂ | CH₂CONHCH₃ | HCl | 186-188 |
| 165. | 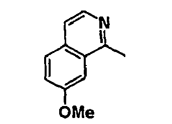 | 2 | 2 | CH | (CH₂)₂ | CH₂CONHCH₃ | base | 80-86 |
| 166. | 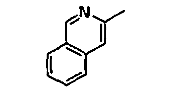 | 2 | 2 | CH | (CH₂)₂ | CH₂CONHCH₃ | base | 156-158 |
| 167. | | 2 | 2 | CH | (CH₂)₂ | CH₂CONH₂ | base | 176-178 |
| 168. | 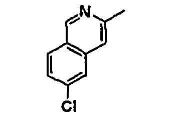 | 2 | 2 | CH | (CH₂)₂ | CH₂CONHCH₃ | base | 148-150 |
| 169. | 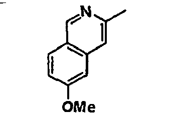 | 2 | 2 | CH | (CH₂)₂ | CH₂CONHCH₃ | base | 154-156 |
| 170. | 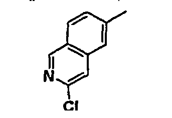 | 2 | 2 | CH | (CH₂)₂ | CH₂CONH₂ | base | 156-158 |
| 171. | | 2 | 2 | CH | (CH₂)₂ | CH₂CONHCH₃ | base | 156-158 |
| 172. | 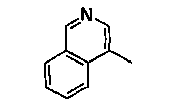 | 2 | 2 | CH | (CH₂)₂ | CH₂CONHCH₃ | HCl | 205-210 |
| 173. | 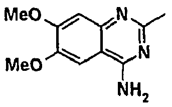 | 2 | 2 | CH | CH₂ | CH₂CONH₂ | base | 419* |
| 174. | | 2 | 2 | CH | (CH₂)₂ | CH₂CONH₂ | base | 105-106 |
| 175. | 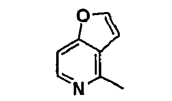 | 2 | 2 | CH | CH₂ | CH₂CONH₂ | base | 129-130 |
| 176. | | 2 | 2 | CH | (CH₂)₂ | CH₂CONH₂ | base | 112-113 |
| 177. | 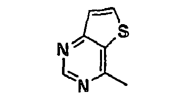 | 2 | 2 | CH | CH₂ | CH₂CONH₂ | base | 180-181 |
| 178. | 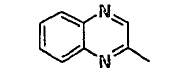 | 2 | 2 | CH | CH₂ | CH₂CONH₂ | base | 244-245 |
| 179. | | 2 | 2 | CH | (CH₂)₂ | CH₂CONHCH₃ | base | 146-150 |
| 180. | | 2 | 2 | CH | (CH₂)₂ | CH₂CONH₂ | base | 169-171 |
| 181. | 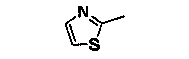 | 2 | 2 | CH | CH₂ | CH₂CONH₂ | base | 137-138 |
| 182. | | 2 | 2 | CH | (CH₂)₂ | CH₂CONH₂ | base | 208-209 |
| 183. | 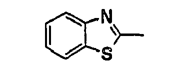 | 2 | 2 | CH | CH₂ | CH₂CONH₂ | base | 183-184 |
| 184. | | 2 | 2 | CH | (CH₂)₂ | CH₂CONH₂ | base | 172-173 |
| 185. | 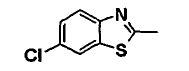 | 2 | 2 | CH | CH₂ | CH₂CONH₂ | base | 187-188 |
| 186. | | 2 | 2 | CH | (CH₂)₂ | CH₂CONH₂ | base | 191-192 |
| 187. | 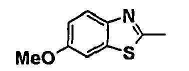 | 2 | 2 | CH | CH₂ | CH₂CONH₂ | base | 222-223 |
| 188. | 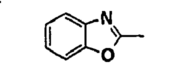 | 2 | 2 | CH | CH₂ | CH₂CONH₂ | base | 149-150 |
| 189. | | 2 | 2 | CH | (CH₂)₂ | CH₂CONH₂ | base | 167-168 |
| 190. | 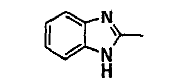 | 2 | 2 | CH | CH₂ | CH₂CONH₂ | base | 220-221 |
| 191. | | 2 | 2 | CH | (CH₂)₂ | CH₂CONH₂ | base | 201-202 |

| | | | | | | | | |
|---|---|---|---|---|---|---|---|---|
| *** M+H (LC-MS)** déc. = décomposition du produit | | | | | | | | |

Les composés de l'invention ont fait l'objet d'essais pharmacologiques permettant de déterminer leur effet inhibiteur de l'enzyme FAAH (Fatty Acid Amide Hydrolase).

L'activité inhibitrice a été mise en évidence dans un test radioenzymatique basé sur la mesure du produit d'hydrolyse (éthanolamine [1-³H]) de l'anandamide [éthanolamine 1-³H] par la FAAH (*Life Sciences (1995), 56, 1999-2005* et *Journal of Pharmacology* et *Experimented Therapeutics (1997), 283, 729-734*). Ainsi, les cerveaux de souris (moins le cervelet) sont prélevés et conservés à -80°C. Les homogénats membranaires sont préparés extemporanément par homogénéisation des tissus au Polytron dans un tampon Tris-HCl 10mM (pH 8,0) contenant 150 mM NaCl et 1 mM EDTA. La réaction enzymatique est ensuite conduite dans 70 µl de tampon contenant de l'albumine de sérum bovin sans acides gras (1 mg/ml). Sont ajoutés successivement les composés testés à différentes concentrations, l'anandamide [éthanolamine 1-³H] (activité spécifique de 15-20 Ci/mmol) diluée à 10 µM avec de l'anandamide froide et la préparation membranaire (400 µg tissu congelé par essai). Après 15 minutes à 25°C, la réaction enzymatique est arrêtée par addition de 140 µL de chloroforme/méthanol (2 :1). Le mélange est agité 10 minutes puis centrifugé pendant 15 minutes à 3500g. Un aliquot (30 µL) de la phase aqueuse contenant l'éthanolamine [1-³H] est compté par scintillation liquide. Dans ces conditions, les composés les plus actifs de l'invention présentent des CI₅₀ (concentration inhibant de 50 % l'activité enzymatique contrôle de la FAAH) comprises entre 0,001 et 1 µM.

Le tableau 2 ci-dessous présente les CI₅₀ de quelques composés selon l'invention.

**Tableau 2**

| Composé N° | CI₅₀ |
|---|---|
| 47 | 85 nM |
| 126 | 113 nM |
| 166 | 87 nM |

Il apparaît donc que les composés selon l'invention ont une activité inhibitrice sur l'enzyme FAAH.

L'activité *in vivo* des composés de l'invention a été évaluée dans un test d'analgésie.

Ainsi, l'administration intrapéritonéale (i.p.) de PBQ (phénylbenzoquinone, 2 mg/kg dans une solution de chlorure de sodium à 0,9 % contenant 5 % d'éthanol) chez des souris mâles OF1 de 25 à 30 g, provoque des étirements abdominaux, en moyenne 30 torsions ou contractions pendant la période de 5 à 15 minutes après injection. Les composés testés sont administrés par voie orale ou par voie intrapéritonéale en suspension dans du Tween 80 à 0,5 %, 60 minutes ou 120 minutes avant l'administration de PBQ. Dans ces conditions, les composés les plus puissants de l'invention réduisent de 35 à 70 % le nombre d'étirements induits par le PBQ, dans une gamme de doses comprise entre 1 et 30 mg/kg.

Le tableau 3 ci-dessous présente les résultats du test d'analgésie pour quelques composés selon l'invention.

**Tableau 3**

| Composé N° | Réduction du nombre d'étirements (%) |
|---|---|
| 47 | - 63 % (b) |
| 126 | - 43 % (b) |
| 166 | - 62 % (a) |

| | |
|---|---|
| (a)1 mg/kg p.o. à 2 heures ; (b)3 mg/kg p.o. à 1 heure | |

L'enzyme FAAH (Chemistry and Physics of Lipids, (2000), 108, 107-121) catalyse l'hydrolyse des dérivés endogènes d'amides et d'esters de différents acides gras tels que la *N*-arachidonoyléthanolamine (anandamide), la *N*-palmitoyléthanolamine, la *N*-oléoyléthanolamine, l'oléamide ou le 2-arachidonoylglycérol. Ces dérivés exercent différentes activités pharmacologiques en interagissant, entre autres, avec les récepteurs cannabinoïdes et vanilloïdes.

Les composés de l'invention, bloquent cette voie de dégradation et augmentent le taux tissulaire de ces substances endogènes. Ils peuvent être utilisés à ce titre dans la prévention et le traitement des pathologies dans lesquelles les cannabinoïdes endogènes et/ou tous autres substrats métabolisés par l'enzyme FAAH, sont impliqués. On peut par exemple citer les maladies et les affections suivantes :
la douleur notamment les douleurs aiguës ou chroniques de type neurogène : migraine, douleurs neuropathiques incluant les formes associées au virus de l'herpès et au diabète,
les douleurs aiguës ou chroniques associées aux maladies inflammatoires : arthrite, arthrite rhumatoïde, ostéoarthrite, spondylite, goutte, vascularite, maladie de Crohn, syndrome du colon irritable,
les douleurs aiguës ou chroniques périphériques,
les vertiges, les vomissements, les nausées en particulier celles consécutives à une chimiothérapie,
les troubles du comportement alimentaire en particulier les anorexies et cachexies de diverses natures ,
les pathologies neurologiques et psychiatrique tremblements, dyskinésies, dystonies, spasticité, comportements compulsifs et obsessionnels, syndrome de Tourette, toutes les formes de dépression et d'anxiété de toute nature et origine, troubles de l'humeur, psychoses,
les maladies neuro-dégénératives aiguës et chroniques : maladie de Parkinson, maladie d'Alzheimer, démence sénile, chorée de Huntington, lésions liées à l'ischémie cérébrale et aux traumatismes crâniens et médullaires,
l'épilepsie,
les troubles du sommeil incluant les apnées du sommeil, les maladies cardiovasculaires en particulier hypertension, arythmies cardiaques, artériosclérose, crise cardiaque, ischémies cardiaques,
l'ischémie rénale,
les cancers : tumeurs bénignes de la peau, papillomes et tumeurs cérébrales, tumeurs de la prostate, tumeurs cérébrales (glioblastomes, médullo-épithéliomes, médullo-blastomes, neuroblastomes, tumeurs d'origine embryonnaires, astrocytomes, astroblastomes, épendyomes, oligodendrogliomes, tumeur du plexus, neuroepithéliomes, tumeur de l'épiphyse, épendymoblastomes, méningiomes malins, sarcomatoses, mélanomes malins, schwénnomes),
les désordres du système immunitaire, notamment les maladies auto-immunes : psoriasis, lupus érythémateux, maladies du tissu conjonctif ou connectivites, syndrome de Sjögrer's, spondylarthrite ankylosante, spondylarthrite indifférenciée, maladie de Behcet's, anémies auto-immunes hémolytiques, sclérose en plaques, sclérose latérale amyotrophique, amyloses, rejet de greffes, maladies affectant la lignée plasmocytaire,
les maladies allergiques : l'hypersensibilité immédiate ou retardée, rhinites ou conjonctivites allergiques, dermatites de contact,
les maladies infectieuses parasitaires, virales ou bactériennes : SIDA, méningites, les maladies inflammatoires, notamment les maladies articulaires : arthrite, arthrite rhumatoïde, ostéoarthrite, spondylite, goutte, vascularite, maladie de Crohn, syndrome du colon irritable, l'ostéoporose, les affections oculaires : hypertension oculaire, glaucome,
les affections pulmonaires : maladies des voies respiratoires, bronchospasmes, toux, asthme, bronchite chronique, obstruction chronique des voies respiratoires, emphysème,
les maladies gastro-intestinales: syndrome du colon irritable, désordres inflammatoires intestinaux, ulcères, diarrhées,
l'incontinence urinaire et l'inflammation vésicale.

L'utilisation des composés selon l'invention, à l'état de base, de sel d'addition à un acide, d'hydrate ou de solvat pharmaceutiquement acceptable, pour la préparation d'un médicament destiné à traiter les pathologies ci-dessus mentionnées fait partie intégrante de l'invention.

L'invention a également pour objet des médicaments qui comprennent un composé de formule (I), ou un sel d'addition à un acide, ou encore un hydrate ou un solvat pharmaceutiquement acceptable du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou un sel d'addition à un acide, ou un hydrate, ou un solvat pharmaceutiquement acceptable dudit composé, et éventuellement un ou plusieurs excipients pharmaceutiquement acceptables.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intrathécale, intranasale, transdermique, pulmonaire, oculaire ou rectale, le principe actif de formule (I) ci-dessus, ou son sel d'addition à un acide, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intra-trachéale, intraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,01 à 20 mg de principe actif par kg de poids corporel, selon la forme galénique.

Il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

L'invention selon un autre de ses aspects, concerne également une méthode de traitement des pathologies ci-dessus indiquées qui comprend l'administration d'une dose efficace d'un composé selon l'invention, d'un de ses sels d'addition à un acide pharmaceutiquement acceptable, d'un solvat ou d'un hydrate dudit composé.

## Revendications

1. Composé répondant à la formule (I) 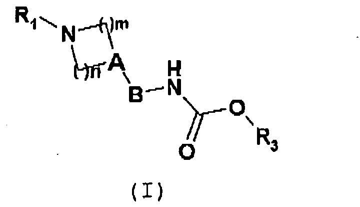 dans laquelle
A représente un atome d'azote ou un groupe CR₂ dans lequel R₂ représente un atome d'hydrogène, de fluor ou un groupe hydroxyle, * cyano, trifluorométhyle, C₁₋₆-alkyle, C₁₋₆-alcoxy ;
n représente un nombre entier égal à 2 ou 3 et m représente un nombre entier égal à 2 lorsque A représente un atome d'azote ;
n représente un nombre entier égal à 1, 2 ou 3 et m un nombre entier égal à 1 ou 2 lorsque A représente un groupe CR₂ ;
B représente une liaison covalente ou un groupe C₁₋₈-alkylène ;
R₁ représente un groupe choisi parmi un phényle, pyridinyle, pyridazinyle, pyrimidinyle, pyrazinyle, triazinyle, oxazolyle, isoxazolyle, thiazolyle, isothiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazopyrimidinyle, thiénopyrimidinyle, benzofuranyle, benzothiényle, benzimidazolyle, benzoxazolyle, benzisoxazolyle, benzothiazolyle, benzisothiazolyle, indazolyle, pyrrolopyridinyle, furopyridinyle, dihydrofuropyridinyle, thiénopyridinyle, dihydrothiénopyridinyle, imidazopyridinyle, pyrazolopyridinyle, oxazolopyridinyle, isoxazolopyridinyle, thiazolopyridinyle ;
le groupe R₁ étant éventuellement substitué par un ou
plusieurs groupes R' et/ou R" ;
R' représente un atome d'halogène ou un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyl, C₃₋₇-cycloalkyle-C₁₋₆-alkyléne, azétidinyle,
pipéridinyle, pyrrolidinyle, morpholinyle, pipérazinyle, azépinyle, NH₂, NHR₆, NR₆R₇, NR₆COR₇, NR₆SO₂R₇, COR₆, CO₂R₆, SO₂R₆, SO₂NR₆R₇ ou -O-(C₁₋₆-alkylène)-O- ;
R" représente un phényle, imidazolyle, pyridinyle ou pyrimidinyle;
le ou les groupes R" étant éventuellement substitués par un ou plusieurs groupes R' identiques ou différents l'un de l'autre ;
R₃ représente un groupe de formule générale CHR₄CONHR₅ dans laquelle
R₄ représente un atome d'hydrogène ou un groupe C₁₋₆-alkyle et
R₅ représente un atome d'hydrogène ou un groupe C₁₋₆-alkyle, C₃₋₇-cycloalkyle, C₃₋₇-cycloalkyle-C₁₋₆-alkyléne ;
R₆ et R₇ représentent indépendamment l'un de l'autre un groupe C₁₋₆-alkyle ;
à l'état de base, de sel d'addition à un acide, d'hydrate ou de solvat.

2. Composé de formule (I) selon la revendication 1, **caractérisé en ce que** :
A représente un atome d'azote ;
n représente un nombre entier égal à 2 ou 3 et m représente un nombre entier égal à 2 ;
B représente un groupe C₁₋₈-alkylène ;
R₁ représente un groupe choisi parmi phényle, pyridinyle, pyrimidinyle, thiadiazolyle, naphtalènyle ;
le groupe R₁ étant éventuellement substitué par un ou
plusieurs groupes R' et/ou R" ;
R' représente un atome d'halogène, ou un groupe nitro ou C₁₋₆-fluoroalkyle ;
R" représente un phényle éventuellement substitué par un ou plusieurs groupes, identiques ou différents l'un de l'autre, choisis parmi un atome d'halogène ou un groupe cyano, C₁₋₆-alcoxy, C₁₋₆-fluoroalcoxy ;
R₃ représente un groupe de formule générale CHR₄CONHR₅ dans laquelle
R₄ représente un atome d'hydrogène et
R₅ représente un atome d'hydrogène ou un groupe C₁₋₆-alkyle, C₃₋₇-cycloalkyle, C₃₋₇-cycloalkyle-C₁₋₆-alkylène ;
à l'état de base, de sel d'addition à un acide, d'hydrate ou de solvat.

3. Composé de formule (I) selon la revendication 1, **caractérisé en ce que** :
A représente un groupe CR₂ dans lequel R₂ représente un atome d'hydrogène, de fluor ou un groupe hydroxyle ;
m représente un nombre entier égal à 1 ou 2 et n représente un nombre entier égal à 1 ou 2 ;
B représente une liaison covalente ou un groupe C₁₋₄-alkylène
R₁ représente un groupe choisi parmi phényle, pyridinyle, pyridazinyle, pyrimidinyle, pyrazinyle, thiazolyle, quinolinyle, isoquinolinyle, phtalazinyle, quinazolinyle, quinoxalinyle, naphthyridinyle, furopyridinyle, thiénopyrimidinyle, imidazopyrimidinyle, benzothiazolyle, benzimidazolyle, benzoxazolyle ;
le groupe R₁ étant éventuellement substitué par un ou plusieurs groupes R' et/ou R" ;
R' représente un atome d'halogène ou un groupe cyano, C₁₋₆-alkyle, un C₁₋₆-alcoxy, C₁₋₆-fluoroalkyle, C₁₋₆-fluoroalcoxy, C₃₋₇-cycloalkyle, pyrrolidinyle, NH₂, NR₆R₇, COR₆ ;
R" représente un phényle, imidazolyle, ou pyridinyle;
le ou les groupes R" étant éventuellement substitués par un ou plusieurs groupes R' identiques ou différents l'un de l'autre
R₃ représente un groupe de formule générale CHR₄CONHR₅ dans laquelle
R₄ représente un atome d'hydrogène ou un groupe C₁₋₆-alkyle et
R₅ représente un atome d'hydrogène ou un groupe C₁₋₆-alkyle, C₃₋₇-cycloalkyle, C₃₋₇-cycloalkyle-C₁₋₆-alkylène ;
R₆ et R₇ représentent indépendamment l'un de l'autre un groupe C₁₋₆-alkyle ;
à l'état de base, de sel d'addition à un acide, d'hydrate ou de solvat.

4. Composé de formule (I) selon la revendication 1 ou 3, **caractérisé en ce que** :
A représente un groupe CR₂ dans lequel R₂ représente un atome d'hydrogène
m est égal à 2 et n est égal à 2 ;
B représente un groupe éthyle ;
R₁ représente un groupe choisi parmi phényle, pyridinyle, pyridazinyle, pyrimidinyle, pyrazinyle, thiazolyle, quinolinyle, isoquinolinyle, phtalazinyle, quinazolinyle, quinoxalinyle, naphthyridinyle, furopyridinyle, thiénopyrimidinyle, imidazopyrimidinyle, benzothiazolyle, benzimidazolyle, benzoxazolyle
le groupe R₁ étant éventuellement substitué par un ou plusieurs groupes R' et/ou R" ;
R' représente un atome d'halogène ou un groupe cyano, C₁₋₆-alkyle, un C₁₋₆-alcoxy, C₁₋₆-fluoroalkyle, C₁₋₆-fluoroalcoxy, C₃₋₇-cycloalkyle, pyrrolidinyle, NH₂, NR₆R₇, COR₆ ;
R" représente un phényle, imidazolyle, ou pyridinyle;
le ou les groupes R" étant éventuellement substitués par un ou plusieurs groupes R' identiques ou différents l'un de l'autre
R₃ représente un groupe de formule générale CHR₄CONHR₅ dans laquelle
R₄ représente un atome d'hydrogène et
R₅ représente un atome d'hydrogène ou un groupe C₁₋₆-alkyle
R₆ et R₇ représentent indépendamment l'un de l'autre un groupe C₁₋₆-alkyle ;
à l'état de base, de sel d'addition à un acide, d'hydrate ou de solvat.

5. **Composé de formule (I) selon l'une quelconque des revendications 1 à 4, caractérisé en ce qu' il est choisi parmi :**
• [3-(4-phényl-pipérazin-1-yl)-propyl]-carbamate de 2-(méthylamino)-2-oxoéthyle
• (2-{4-[6-(3-Chloro-phényl)-pyridin-2-yl]-pipérazin-1-yl}-éthyl)-carbamate de 2-amino-2-oxoéthyle
• (2-{4-[6-(3-Chloro-phényl)-pyridin-2-yl]-pipérazin-1-yl}-éthyl)-carbamate de 2-(méthylamino)-2-oxoéthyle
• (2-{4-[6-(4-Chloro-phényl)-pyridin-2-yl]-pipérazin-1-yl}-éthyl)-carbamate de 2-amino-2-oxoéthyle
• (2-{4-[6-(4-Chloro-phényl)-pyridin-2-yl]-pipérazin-1-yl}-éthyl)-carbamate de 2-(méthylamino)-2-oxoéthyle
• (2-{4-[6-(3-Cyano-phényl)-pyridin-2-yl]-pipérazin-l-yl}-éthyl)-carbamate de 2-amino-2-oxoéthyle
• (2-{4-[6-(3-Cyana-phényl)--pyridin-2-yl]-pipérazin--1-yl}-éthyl)-carbamate de 2-(méthylamino)-2-oxoéthyle
• (2-{4-[6-(4-Cyano-phényl)-pyridin-2-yl]-pipérazin-1-yl}-éthyl)-carbamate de 2-amino-2-oxoéthyle
• (2-(4-[6-(4-Cyano-phényl)-pyridin-2-yl]-pipérazin-1-yl}-éthyl)-carbamate de 2-(méthylamino)-2-oxoéthyle
• (2-{4-[6-(3-Méthoxy-phényl)-pyridin-2-yl]-pipérazin-1-yl}-éthyl)-carbamate de 2-amino-2-oxoéthyle
• (2-{4-[6-(3-Méthoxy-phényl)-pyridin-2-yl]-pipérazin-1-yl}-éthyl)-carbamate de 2-(méthylamino)-2-oxoéthyle
• (2-{4-[6-(4-Méthoxy-phényl)-pyridin-2-yl]-pipérazin-1-yl}-éthyl)-carbamate de 2-amino-2-oxoéthyle
• (2-{4-[6-(4-Méthoxy-phényl)-pyridin-2-yl]-pipérazin-1-yl}-éthyl)-carbamate de 2-(méthylamino)-2-oxoéthyle
• (2-{4-[6-(3-Trifluorométhoxy-phényl)-pyridin-2-yl]-pipérazin-1-yl}-éthyl)-carbamate de 2-amino-2-oxoéthyle
• (2-{4-[6-(3-Trifluorométhoxy-phényl)-pyridin-2-yl]-pipérazin-1-yl}-éthyl)-carbamate de 2-(méthylamino)-2-oxoéthyle
• (2-{4-[6-(4-Trifluorométhoxy-phényl)-pyridin-2-yl]-pipérazin-1-yl}-éthyl)-carbamate de 2-amino-2-oxoéthyle
• (2-{4-[6-(4-Trifluorométhoxy-phényl)-pyridin-2-yl]-pipérazin-1-yl}-éthyl)-carbamate de 2-(méthylamino)-2-oxoéthyle
• (2-{4-[5-(3-Chloro-phényl)-pyridin-2-yl]-pipérazin-1-yl}-éthyl)-carbamate de 2-amino-2-oxoéthyle
• (2-{4-[5-(3-Chloro-phényl)-pyridin-2-yl]-pipérazin-1-yl}-éthyl)-carbamate de 2-(méthylamino)-2-oxoéthyle
• (2-{4-[5-(4-Chloro-phényl)-pyridin-2-yl]-pipérazin-1-yl}-éthyl)-carbamate de 2-amino-2-oxoéthyle
• (2-{4-[5-(4-Chloro-phényl)-pyridin-2-yl]-pipérazin-1-yl}-éthyl)-carbamate de 2-(méthylamino)-2-oxoéthyle
• (2-{4-[5-(3-cyano-phényl)-pyridin-2-yl]-pipérazin-1-yl}-éthyl)-carbamate de 2-amino-2-oxoéthyle
• (2-{4-[5-(3-Cyano-phényl)-pyridin-2-yl]-pipérazin-1-yl}-éthyl)-carbamate de 2-(méthylamino)-2-oxoéthyle
• (2-{4-[5-(4-Cyano-phényl)-pyridin-2-yl]-pipérazin-1-yl}-éthyl)-carbamate de 2-amino-2-oxoéthyle
• (2-{4-[5-(4-cyano-phényl)-pyridin-2-yl]-pipérazin-1-yl}-éthyl)-carbamate de 2-(méthylamino)-2-oxoéthyle
• (2-{4-[5-(3-Méthoxy-phényl)-pyridin-2-yl]-pipérazin-1-yl}-éthyl)-carbamate de 2-amino-2-oxoéthyle
• (2-{4-[5-(3-Méthoxy-phényl)-pyridin-2-yl]-pipérazin-1-yl}-éthyl)-carbamate de 2-(méthylamino)-2-oxoéthyle
• (2-{4-[5-(4-Méthoxy-phényl)-pyridin-2-yl]-pipérazin-1-yl}-éthyl)-carbamate de 2-amino-2-oxoéthyle
• (2-{4-[5-(4-Méthoxy-phényl)-pyridin-2-yl]-pipérazin-1-yl}-éthyl)-carbamate de 2-(méthylamino)-2-oxoéthyle
• (2-{4-[5-(3-Trifluorométhoxy-phényl)-pyridin-2-yl]-pipérazin-1-yl}-éthyl)-carbamate de 2-amino-2-oxoéthyle
• (2-{4-[5-(3-Trifluorométhoxy-phényl)-pyridin-2-yl]-pipérazin-1-yl}-éthyl)-carbamate de 2-(méthylamino)-2-oxoéthyle
• (2-{4-[5-(4-Trifluorométhoxy-phényl)-pyridin-2-yl]-pipérazin-1-yl}-éthyl)-carbamate de 2-amino-2-oxoéthyle
• (2-{4-[5-(4-Trifluorométhoxy-phényl)-pyridin-2-yl]-pipérazin-1-yl}-éthyl)-carbamate de 2-(méthylamino)-2-oxoéthyle
• {3-[4-(5-Nitro-pyridin-2-yl)-[1,4]diazepan-1-yl]-propyl}-carbamate de 2-(méthylamino)-2-oxoéthyle
• {3-[4-(3-Chloro-5-trifluorométhyl-pyridin-2-yl)-[1,4]diazepan-1-yl]-propyl}-carbamate de 2-(méthylamino)-2-oxoéthyle
• {3-[4-(4-Trifluorométhyl-pyrimidin-2-yl)-[1,4]diazepan-1-yl]-propyl}-carbamate de 2-(méthylamino)-2-oxoéthyle
• {3-[4-(3-Phényl-[1,2,4]thiadiazol-5-yl)-[1,4]diazepan-1-yl]-propyl}-carbamate de 2-(méthylamino)-2-oxoéthyle
• [2-(4-Naphthalen-1-yl-pipérazin-1-yl)-éthyl]-carbamate de 2-(méthylamino)-2-oxoéthyle
• [3-(4-Naphthalen-1-yl-pipérazin-1-yl)-propyl]-carbamate de 2-(méthylamino)-2-oxoéthyle
• [1-(3-Trifluorométhyl-phényl)-azétidin-3-ylméthyl]-carbamate de 2-amino-2-oxoéthyle
• [1-(3-Trifluorométhyl-phényl)-azétidin-3-ylméthyl]-carbamate de 2-(méthylamino)-2-oxoéthyle
• [1-(3-Trifluorométhyl-phényl)-azétidin-3-ylméthyl]-carbamate de 2-(cyclopropylméthylamino)-2-oxoéthyle
• {2-[1-(3-Trifluorométhyl-phényl)-pipéridin-4-yl]-éthyl}-carbamate de 2-(méthylamino)-2-oxoéthyle
• {2-[1-(3-Trifluorométhyl-phényl)-pipéridin-4-yl]-éthyl}-carbamate de 2-amino-2-oxoéthyle
• (2-[4-Fluoro-1-(3-trifluorométhyl-phényl)-pipéridin-4-yl]-éthyl}-carbamate de 2-(méthylamino)-2-oxoéthyle
• [2-(3,4,5,6-Tétrahydro-2H-[1,2']bipyridinyl-4-yl)-éthyl]-carbamate de 2-(méthylamino)-2-oxoéthyle
• [2-(6'-Méthyl-3,4,5,6-tétrahydro-2H-[1,2']bipyridinyl-4-yl)-éthyl]-carbamate de 2-(méthylamino)-2-oxoéthyle
• [2-(6'-Propyl-3,4,5,6-tétrahydro-2H-[1,2']bipyridinyl-4-yl)-éthyl]-carbamate de 2-(méthylamino)-2-oxoéthyle
• [2-(6'-Isobutyl-3,4,5,6-tétrahydro-2H-[1,2']bipyridinyl-4-yl}-éthyl]-carbamate de 2-(méthylamino)-2-oxoéthyle
• [2-(6'-Isobutyl-3,4,5,6-tétrahydro-2H-[1,2']bipyridinyl-4-yl)-éthyl]-carbamate de 2-amino-2-oxoéthyle
• [2-(6'-Cyclopropyl-3,4,5,6-tétrahydro-2H-[1,2']bipyridinyl-4-yl)-éthyl]-carbamate de 2-(méthylamino)-2-oxoéthyle
• [2-(6'-Cyclopentyl-3,4,5,6-tétrahydro-2H-[1,2']bipyridinyl-4-yl)-éthyl]-carbamate de 2-(méthylamino)-2-oxoéthyle
• [2-(6'-Pyrrolidin-1-yl-3,4,5,6-tétrahydro-2H-[1,2']bipyridinyl-4-yl)-éthyl]-carbamate de 2-(méthylamino)-2-oxoéthyle
• (6'-Bromo-3,4,5,6-tétrahydro-2H-[1,2']bipyridinyl-4-yl)-carbamate de 2-amino-2-oxoéthyle
• (6'-Bromo-3,4,5,6-tétrahydro-2H-[1,2']bipyridinyl-4-ylméthyl)-carbamate de 2-amino-2-oxoéthyle
• [2-(6'-Bromo-3,4,5,6-tétrahydro-2H-[1,2']bzpyridinyl-4-yl)-éthyl]-carbamate de 2-amino-2-oxoéthyle
• (6'-Phényl-3,4,5,6-tétrahydro-2H-[1,2']bipyridinyl-4-yl)-carbamate de 2-amino-2-oxoéthyle
• (6'-Phényl-3,4,5,6-tétrahydro-2H-[1,2']bipyridinyl-4-ylméthyl)-carbamate de 2-amino-2-oxoéthyle
• [2-(6'-Phényl-3,4,5,6-tétrahydro-2H-[1,2']bipyridinyl-4-yl)-éthyl]-carbamate de 2-amino-2-oxoéthyle
• [6'-(4-Fluoro-phényl)-3,4,5,6-tétrahydro-2H-[1,2']bipyridinyl-4-yl]-carbamate de 2-amino-2-oxoéthyle
• [6'-(4-Fluoro-phényl)-3,4,5,6-tétrahydro-2H-[1,2']bipyridinyl-4-ylméthyl]-carbamate de 2-amino-2-oxoéthyle
• {2-[6'-(4-Fluoro-phényl)-3,4,5,6-tétrahydro-2H-[1,2']bipyridinyl-4-yl]-éthyl}-carbamate de 2-amino-2-oxoéthyle
• (3'-Bromo-3,4,5,6-tétrahydro-2H-[1,2']bipyridinyl-4-yl)-carbamate de 2-amino-2-oxoéthyle
• (3'-Bromo-3,4,5,6-tétrahydro-2H-[1,2']bipyridinyl-4-ylméthyl)-carbamate de 2-amino-2-oxoéthyle
• [2-(3'-Bromo-3,4,5,6-tétrahydro-2H-[1,2']bipyridinyl-4-yl)-éthyl]-carbamate de 2-amino-2-oxoéthyle
• (3'-Cyano-3,4,5,6-tétrahydro-2H-[1,2']bipyridinyl-4-ylméthyl)-carbamate de 2-amino-2-oxoéthyle
• [2-{3'-Cyano-3,4,5,6-tétrahydro-2H-[1,2']bipyridinyl-4-yl)-éthyl]-carbamate de 2-amino-2-oxoéthyle
• (3'-Phényl-3,4,5,6-tétrahydro-2H-[1,2']bipyridinyl-4-yl)-carbamate de 2-amino-2-oxoéthyle
• (3'-Phényl-3,4,5,6-tétrahydro-2H-[1,2']bipyridinyl-4-ylméthyl)-carbamate de 2-amino-2-oxoéthyle
• [2-(3'-Phényl-3,4,5,6-tétrahydro-2H-[1,2']bipyridinyl-4-yl)-éthyl]-carbamate de 2-amino-2-oxoéthyle
• [3'-(4-Fluoro-phényl)-3,4,5,6-tétrahydro-2H-[1,2']bipyridinyl-4-yl]-carbamate de 2-amino-2-oxoéthyle
• [3'-(4-Fluoro-phényl)-3,4,5,6-tétrahydro-2H-[1,2']bipyridinyl-4-ylméthyl]-carbamate de 2-amino-2-oxoéthyle
• {2-[3'-(4-Fluoro-phényl)-3,4,5,6-tétrahydro-2H-[1,2']bipyridinyl-4-yl]-éthyl}-carbamate de 2-amino-2-oxoéthyle
• (4'-Trifluorométhyl-3,4,5,6-tétrahydry-2H-[1,2']bipyridinyl-4-ylméthyl)-carbamate de 2-amino-2-oxoéthyle
• [2-(4'-Trifluorométhyl-3,4,5,6-tétrahydro-2H-[1,2']bipyridinyl-4-yl)-éthyl]-carbamate de 2-amino-2-oxoéthyle
• [2-(4'-Phényl-3,4,5,6-tétrahydro-2H-[1,2']bipyridinyl-4-yl)-éthyl]-carbamate de 2-(méthylamino)-2-oxoéthyle
• [2-(4'-Phényl-3,4,5,6-tétrahydro-2H-[1,2']bipyridinyl-4-yl)-éthyl]-carbamate de 2-amino-2-oxoéthyle
• (5'-Chloro-3,4,5,6-tétrahydro-2H-[1,2']bipyridinyl-4-ylméthyl)-carbamate de 2-amino-2-oxoéthyle
• [2-(5'-Chloro-3,4,5,6-tétrahydro-2H-[1,2']bipyridinyl-4-yl)-éthyl]-carbamate de 2-amino-2-oxoéthyle
• (5'-Bromo-3,4,5,6-tétrahydro-2H-[1,2']bipyridinyl-4-yl)-carbamate de 2-amino-2-oxoéthyle
• (5'-Bromo-3,4,5,6-tétrahydro-2H-[1,2']bipyridinyl-4-ylméthyl)-carbamate de 2-amino-2-oxoéthyle
• [2-(5-Bromo-3,4,5,6-tétrahydro-2H-[1,2']bipyridinyl-4-yl)-éthyl]-carbamate de 2-amino-2-oxoéthyle
• (5'-Méthyl-3,4,5,6-tétrahydro-2H-[1,2']bipyridinyl-4-ylméthyl)-carbamate de 2-amino-2-oxoéthyle
• [2-(5'-Méthyl-3,4,5,6-tétrahydro-2H-[1,2']bipyridinyl-4-yl)-éthyl]-carbamate de 2-amino-2-oxoéthyle
• [2-(5'-Isobutyl-3,4,5,6-tétrahydro-2H-[1,2']bipyridinyl-4-yl)-éthyl]-carbamate de 2-amino-2-oxoéthyle
• [2-(5'-Isobutyl-3,4,5,6-tétrahydro-2H-[1,2']bipyridinyl-4-yl)-éthyl]-carbamate de 2-(méthylamino)-2-oxoéthyle
• (5'-Trifluorométhyl-3,4,5,6-tétrahydro-2H-[1,2']bipyridinyl-4-ylméthyl)-carbamate de 2-amino-2-oxoéthyle
• [2-(5'-Trifluorométhyl-3,4,5,6-tétrahydro-2H-[1,2']bipyridinyl-4-yl)-éthyl]-carbamate de 2-amino-2-oxoéthyle
• (5'-Cyano-3,4,5,6-tétrahydro-2H-[1,2']bipyridinyl-4-ylméthyl)-carbamate de 2-amino-2-oxoéthyle
• (5'-Acetyl-3,4,5,6-tétrahydro-2H-[1,2']bipyridinyl-4-ylméthyl)-carbamate de 2-amino-2-oxoéthyle
• [2-(5'-Acetyl-3,9,5,6-tétrahydro-2H-[1,2']bipyridinyl-4-yl)-éthyl]-carbamate de 2-amino-2-oxoéthyle
• (5'-Phényl-3,4,5,6-tétrahydro-2H-[1,2']bipyridinyl-4-yl)-carbamate de 2-amino-2-oxoéthyle
• (5'-Phényl-3,4,5,6-tétrahydro-2H-[1,2']bipyridinyl-4-ylméthyl)-carbamate de 2-amino-2-oxoéthyle
• [5'-(4-Fluoro-phényl)-3,4,5,6-tétrahydro-2H-[1,2']bipyridinyl-4-yl]-carbamate de 2-amino-2-oxoéthyle
• [5'-(4-Fluoro-phényl)-3,4,5,6-tétrahydro-2H-[1,2']bipyridinyl-4-ylméthyl]-carbamate de 2-amino-2-oxoéthyle
• {2-[5'-(4-Fluoro-phényl)-3,4,5,6-tétrahydro-2H-[1,2']bipyridinyl-4-yl]-éthyl}-carbamate de 2-amino-2-oxoéthyle
• {2-[5'-(4-Chloro-phényl)-3,4,5,6-tétrahydro-2H-[1,2']bipyridinyl-4-yl]-éthyl}-carbamate de 2-amino-2-oxoéthyle
• {2-[5'-(4-Chloro-phényl)-3,4,5,6-tétrahydro-2H-[1,2']bipyridinyl-4-yl]-éthyl}-carbamate de 2-(méthylamino)-2-oxoéthyle
• [1-(5-Éthyl-pyrimidin-2-yl)-pipéridin-4-ylméthyl]-carbamate de 2-amino-2-oxoéthyle
• {2-[1-(5-Éthyl-pyrimidin-2-yl)-pipéridin-4-yl]-éthyl}-carbamate de 2-amino-2-oxoéthyle
• [1-(5-Propyl-pyrimidin-2-yl)-pipéridin-4-ylméthyl]-carbamate de 2-amino-2-oxoéthyle
• (2-{1-[5-(4-Chloro-phényl)-pyrimidin-2-yl]-pipéridin-4-yl}-éthyl)-carbamate de 2-amino-2-oxoéthyle
• [1-(4-Trifluorométhyl-pyrimidin-2-yl)-pipéridin-4-ylméthyl]-carbamate de 2-amino-2-oxoéthyle
• {2-[1-(4-Diméthylamino-6-méthyl-pyrimidin-2-yl)-pipéridin-4-yl]-éthyl}-carbamate de 2-amino-2-oxoéthyle
• [1-(2-Amino-6-méthyl-pyrimidin-4-yl)-pipéridin-4-ylméthyl]-carbamate de 2-amino-2-oxoéthyle
• {2-[1-(2-Amino-6-méthyl-pyrimidin-4-yl)-pipéridin-4-yl]-éthyl}-carbamate de 2-amino-2-oxoéthyle
• [1-(6-Méthyl-2-pyridin-2-yl-pyrimidin-4-yl)-pipéridin-4-ylméthyl]-carbamate de 2-amino-2-oxoéthyle
• [1-(6-Phényl-pyrimidin-4-yl)-pipéridin-4-ylméthyl]-carbamate de 2-amino--2-oxoéthyle
• [1-(4-Phényl-pyrimidin-2-yl)-pipéridin-4-ylméthyl]-carbamate de 2-amino-2-oxoéthyle
• [1-(6-Imidazol-1-yl-pyrimidin-4-yl)-pipéridin-4-ylméthyl]-carbamate de 2-amino-2-oxoéthyle
• {2-[1-(6-Imidazol-1-yl-pyrimidin-4-yl)-pipéridin-4-yl]-éthyl}-carbamate de 2-amino-2-oxoéthyle
• Pyrazin-2-yl-pipéridin-4-ylméthyl)-carbamate de 2-amino-2-oxoéthyle
• [2-(1-Pyrazin-2-yl-pipéridin-4-yl)-éthyl]-carbamate de 2-amino-2-oxoéthyle
• [1-(6-Amino-pyrazin-2-yl)-pipéridin-4-ylméthyl]-carbamate de 2-amino-2-oxoéthyle
• {2-[1-(6-Amino-pyrazin-2-yl)-pipéridin-4-yl]-éthyl}-carbamate de 2-amino-2-oxoéthyle
• [1-(3,6-Diméthyl-pyrazin-2-yl)-pipéridin-4-ylméthyl]-carbamate de 2-amino-2-oxoéthyle
• {2-[1-(3,6-Diméthyl-pyrazin-2-yl)-pipéridin-4-yl]-éthyl}-carbamate de 2-amino-2-oxoéthyle
• [1-(6-Méthyl-pyridazin-3-yl)-pipéridin-4-ylméthyl]-carbamate de 2-amino-2-oxoéthyle
• {2-[1-(6-Méthyl-pyridazin-3-yl)-pipéridin-4-yl]-éthyl}-carbamate de 2-amino-2-oxoéthyle
• {2-[1-(6-Amino-pyridazin-3-yl)-pipéridin-4-yl]-éthyl}-carbamate de 2-amino-2-oxoéthyle
• [1-(6-Phényl-pyridazin-3-yl)-pipéridin-4-ylméthyl]-carbamate de 2-amino-2-oxoéthyle
• {2-[1-(6-Phényl-pyridazin-3-yl)-pipéridin-4-yl]-éthyl}-carbamate de 2-amino-2-oxoéthyle
• Quinolin-2-yl-pipéridin-4-ylméthyl)-carbamate de 2-(méthylamino)-2-oxoéthyle
• Quinolin-2-yl-pipéridin-4-ylméthyl)-carbamate de 2-amino-2-oxoéthyle
• Quinolin-2-yl-pipéridin-4-ylméthyl)-carbamate de 1-méthyl-2-(méthylamino)-2-oxoéthyle
• [2-(1-Quinolin-2-yl-pipéridin-4-yl)-éthyl]-carbamate de 2-(méthylamino)-2-oxoéthyle
• [2-(1-Quinolin-2-yl-pipéridin-4-yl)-éthyl]-carbamate de 2-amino-2-oxoéthyle
• [2-(4-Fluoro-1-quinolin-2-yl-pipéridin-4-yl)-éthyl]-carbamate de 2-(méthylamino)-2-oxoéthyle
• [2-(4-Hydroxy-1-quinolin-2-yl-pipéridin-4-yl)-éthyl]-carbamate de 2-(méthylamino)-2-oxoéthyle
• {2-[1-(5-Chloro-quinolin-2-yl)-pipéridin-4-yl]-éthyl}-carbamate de 2-(méthylamino)-2-oxoéthyle
• {2-[1-(5-Chloro-quinolin-2-yl)-pipéridin-4-yl]-éthyl}-carbamate de 2-amino-2-oxoéthyle
• {2-[1-(5-Fluoro-quinolin-2-yl)-pipéridin-4-yl]-éthyl}-carbamate de 2-(méthylamino)-2-oxoéthyle
• {2-[1-(5-Méthoxy-quinolin-2-yl)-pipéridin-4-yl]-éthyl}-carbamate de 2-(méthylamino)-2-oxoéthyle
• [1-(6-Chloro-quinolin-2-yl)-pipéridin-4-ylméthyl]-carbamate de 2-(méthylamino)-2-oxoéthyle
• [1-(6-Chloro-quinolin-2-yl)-pipéridin-4-ylméthyl]-carbamate de 2-amino-2-oxoéthyle
• {2-[1-(6-Chloxo-quinolin-2-yl)-pipéridin-4-yl]-éthyl}-carbamate de 2-(méthylamino)-2-oxoéthyle
• {2-[1-(6-Chloro-quinolin-2-yl)-pipéridin-4-yl]-éthyl}-carbamate de 2-amino-2-oxoéthyle
• {2-[1-(6-Méthoxy-quinolin-2-yl)-pipéridin-4-yl]-éthyl}-carbamate de 2-amino-2-oxoéthyle
• {2-[1-(6-Méthoxy-quinolin-2-yl)-pipéridin-9-yl]-éthyl}-carbamate de 2-(méthylamino)-2-oxoéthyle
• {2-[1-(6-Méthoxy-quinolin-2-yl)-pipéridin-4-yl]-éthyl}-carbamate de 2-(éthylamino)-2-oxoéthyle
• {2-[1-(6-Méthoxy-quinolin-2-yl)-pipéridin-4-yl]-éthyl}-carbamate de 2-(cyclopropylméthylamino)-2-oxoéthyle
• {2-[1-(7-Chloro-quinolin-2-yl)-pipéridin-4-yl]-éthyl}-carbamate de 2-amino-2-oxoéthyle
• {2-[1-(7-Chloro-quinolin-2-yl)-pipéridin-9-yl]-éthyl}-carbamate de 2-(méthylamino)-2-oxoéthyle
• {2-[1-(8-Chloro-quinolin-2-yl)-pipéridin-4-yl]-éthyl}-carbamate de 2-(méthylamino)-2-oxoéthyle
• {2-[1-(8-Chloro-quinolin-2-yl)-pipéridin-4-yl]-éthyl}-carbamate de 2-amino-2-oxoéthyle
• [2-(1-Quinolin-3-yl-pipéridin-4-yl)-éthyl]-carbamate de 2-(méthylamino)-2-oxoéthyle
• [2-(1-Quinolin-3-yl-pipéridin-4-yl)-éthyl]-carbamate de 2-amino-2-oxoéthyle
• [2-(1-Quinolin-4-y1-pipéridin-4-yl)-éthyl]-carbamate de 2-(méthylamino)-2-oxoéthyle
• [2-(1-Quinolin-4-yl-pipéridin-4-yl)-éthyl]-carbamate de 2-amino-2-oxoéthyle
• [2-(1-[1,5]Naphthyridin-2-yl-pipéridin-4-yl)-éthyl]-carbamate de 2-(méthylamino)-2-oxoéthyle
• Isoquinolin-1-yl-pyrrolidin-3-ylméthyl)-carbamate de 2-amino-2-oxoéthyle
• Isoquinolin-1-yl-pyrrolidin-3-ylméthyl)-carbamate de 2-(méthylamino)-2-oxoéthyle
• Isoquinolin-1-yl-pipéridin-4-yl)-carbamate de 2-(méthylamino)-2-oxoéthyle
• Isoquinolin-1-yl-pipéridin-4-ylméthyl)-carbamate de 2-(méthylamino)-2-oxoéthyle
• [2-(1-Isoquinolin-1-yl-pipéridin-4-yl)-éthyl]-carbamate de 2-(méthylamino)-2-oxoéthyle
• [2-(1-Isoquinolin-1-yl-pipéridin-4-yl)-éthyl]-carbamate de 2-amino-2-oxoéthyle
• [3-(1-Isoquinolin-1-yl-pipéridin-4-yl)-propyl]-carbamate de 2-(méthylamino)-2-oxoéthyle
• {2-[1-(6-Fluoro-isoquinolin-1-yl)-pipéridin-4-yl]-éthyl}-carbamate de 2-amino-2-oxoéthyle
• {2-[1-(6-Fluoro-isoquinolin-1-yl)-pipéridin-4-yl]-éthyl}-carbamate de 2-(méthylamino)-2-oxoéthyle
• {2-[1-(6-Chloro-isoquinolin-1-yl)-pipéridin-4-yl]-éthyl}-carbamate de 2-(méthylamino)-2-oxoéthyle
• [1-(6-Méthoxy-isoquinolin-1-yl)-pipéridin-4-ylméthyl]-carbamate de 2-(méthylamino)-2-oxoéthyle
• {2-[1-(6-Méthoxy-isoquinolin-1-yl)-pipéridin-4-yl]-éthyl}-carbamate de 2-(méthylamino)-2-oxoéthyle
• {2-[1-(6-Méthoxy-isoquinolin-1-yl)-pipéridin-4-yl]-éthyl}-carbamate de 2-amino-2-oxoéthyle
• {2-[1-(7-Chloro-isoquinolin-1-yl)-pipéridin-4-yl]-éthyl}-carbamate de 2-(méthylamino)-2-oxoéthyle
• {2-[1-(7-Méthoxy-isoquinolin-1-yl)-pipéridin-4-yl]-éthyl}-carbamate de 2-(méthylamino)-2-oxoéthyle
• [2-(1-Isoquinolin-3-yl-pipéridin-4-yl)-éthyl]-carbamate de 2-(méthylamino)-2-oxoéthyle
• [2-(1-Isoquinolin-3-yl-pipéridin-4-yl)-éthyl]-carbamate de 2-amino-2-oxoéthyle
• {2-[1-(6-Chloro-isoquinolin-3-yl)-pipéridin-4-yl]-éthyl}-carbamate de 2-(méthylamino)-2-oxoéthyle
• {2-[1-(6-Méthoxy-isoquinolin-3-yl)-pipéridin-4-yl]-éthyl}-carbamate de 2-(méthylamino)-2-oxoéthyle
• {2-[1-(3-Chloro-isoquinolin-6-yl)-pipéridin-4-yl]-éthyl}-carbamate de 2-amino-2-oxoéthyle
• {2-[1-(3-Chloro-isoquinolin-6-yl)-pipéridin-4-yl]-éthyl}-carbamate de 2-(méthylamino)-2-oxoéthyle
• [2-(1-Isoquinolin-4-yl-pipéridin-4-yl)-éthyl]-carbamate de 2-(méthylamino)-2-oxoéthyle
• [1-(4-Amino-6,7-diméthoxy-quinazolin-2-yl)-pipéridin-4-ylméthyl]-carbamate de 2-amino-2-oxoéthyle
• {2-[1-(4-Amino-6,7-diméthoxy-quinazolin-2-yl)-pipéridin-4-yl]-éthyl}-carbamate de 2-amino-2-oxoéthyle
• Furo[3,2-c]pyridin-4-yl-pipéridin-4-ylméthyl)-carbamate de 2-amino-2-oxoéthyle
• [2-(1-Furo[3,2-c]pyridin-4-yl-pipéridin-4-yl)-éthyl]-carbamate de 2-amino-2-oxoéthyle
• Thieno[3,2-d]pyrimidin-4-yl-pipéridin-4-ylméthyl)-carbamate de 2-amino-2-oxoéthyle
• Quinoxalin-2-yl-pipéridin-4-ylméthyl)-carbamate de 2-amino-2-oxoéthyle
• [2-(1-Quinoxalin-2-yl-pipéridin-4-yl)-éthyl]-carbamate de 2-(méthylamino)-2-oxoéthyle
• [2-(1-Quinoxalin-2-yl-pipéridin-4-yl)-éthyl]-carbamate de 2-amino-2-oxoéthyle
• Thiazol-2-yl-pipéridin-4-ylméthyl)-carbamate de 2-amino-2-oxoéthyle
• [2-(1-Thiazol-2-yl-pipéridin-4-yl)-éthyl]-carbamate de 2-amino-2-oxoéthyle
• Benzothiazol-2-yl-pipéridin-4-ylméthyl)-carbamate de 2-amino-2-oxoéthyle
• [2-(1-Benzothiazol-2-yl-pipéridin-4-yl)-éthyl]-carbamate de 2-amino-2-oxoéthyle
• (1-(6-Chloro-benzothiazol-2-yl)-pipéridin-4-ylméthyl]-carbamate de 2-amino-2-oxoéthyle
• {2-[1-(6-Chloro-benzothiazol-2-yl)-pipéridin-4-yl]-éthyl}-carbamate de 2-amino-2-oxoéthyle
• [1-(6-Méthoxy-benzothiazol-2-yl)-pipéridin-4-ylméthyl]-carbamate de 2-amino-2-oxoéthyle
• Benzooxazol-2-yl-pipéridin-4-ylméthyl)-carbamate de 2-amino-2-oxoéthyle
• [2-(1-Benzooxazol-2-yl-pipéridin-4-yl)-éthyl]-carbamate de 2-amino-2-oxoéthyle
• [1-(1H-Benzoimidazol-2-yl)-pipéridin-4-ylméthyl]-carbamate de 2-amino-2-oxoéthyle
• {2-[1-(1H-Benzoimidazol-2-yl)-pipéridin-4-yl]-éthyl}-carbamate de 2-amino-2-oxoéthyle.

6. Procédé de préparation d'un composé de formule (I) selon l'une quelconque des revendications 1 à 5, comprenant l'étape consistant à
transformer le carbamate-ester de formule générale (Ia) 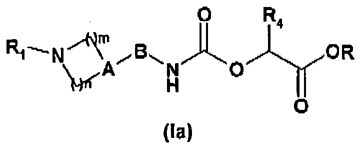 dans laquelle n, m, A, B, R₁ et R₄ sont tels que définis dans la formule générale (I) selon la revendication 1 et R représente un groupe méthyle ou éthyle,
par aminolyse au moyen d'une amine de formule générale R₅NH₂ dans laquelle R₅ est tel que défini dans la formule générale (I) selon la revendication 1.

7. Procédé de préparation d'un composé de formule (I) selon selon l'une quelconque des revendications 1 à **5**, comprenant l'étape consistant à
transformer le dérivé oxazolidine-dione de formule générale (V) 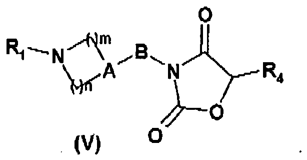 dans laquelle n, m, A, B, R₁ et R₄ sont tels que définis dans la formule générale (I) selon la revendication 1, par aminolyse au moyen d'une amine de formule générale R₅NH₂ dans laquelle R₅ est tel que défini dans la formule générale (I) selon la revendication 1.

8. Composé répondant à la formule générale (Ia), 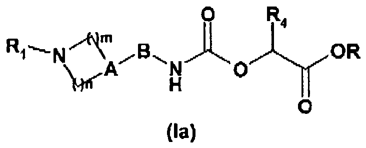 dans laquelle
A représente un atome d'azote ou un groupe CR₂ dans lequel R₂ représente un atome d'hydrogène, de fluor ou un groupe hydroxyle, cyano, trifluorométhyle, C₁₋₆-alkyle, C₁₋₆-alcoxy ;
n représente un nombre entier égal à 2 ou 3 et m représente un nombre entier égal à 2 lorsque A représente un atome d'azote ;
n représente un nombre entier égal à 1, 2 ou 3 et m un nombre entier égal à 1 ou 2 lorsque A représente un groupe CR₂ ;
B représente une liaison covalente ou un groupe C₁₋₆-alkylène ;
R₁ représente un groupe choisi parmi un phényle, pyridinyle, pyridazinyle, pyrimidinyle, pyrazinyle, triazinyle, oxazolyle, isoxazolyle, thiazolyle, isothiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazopyrimidinyle, thiénopyrimidinyle, benzofuranyle, benzothiényle, benzimidazolyle, benzoxazolyle, benzisoxazolyle, benzothiazolyle, benzisothiazolyle, indazolyle, pyrrolopyridinyle, furopyridinyle, dihydrofuropyridinyle, thiénopyridinyle, dihydrothiénopyridinyle, imidazopyridinyle, pyrazolopyridinyle, oxazolopyridinyle, isoxazolopyridinyle, thiazolopyridinyle ;
le groupe R₁ étant éventuellement substitué par un ou plusieurs groupes R' et/ou R" ;
R' représente un atome d'halogène ou un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyle, C₃₋₇-cycloalkyle-C₁₋₆-alkylène, azétidinyle, pipéridinyle, pyrrolidinyle, morpholinyle, pipérazinyle, azépinyle, NH₂, NHR₆, NR₆R₇, NR₆COR₇, NR₆SO₂R₇, COR₆, CO₂R₆, SO₂R₆, SO₂NR₆R₇ ou -O-(C₁₋₆-alkylène)-O- ;
R" représente un phényle, imidazolyle, pyridinyle, pyrazinyle, pyridazinyle ou pyrimidinyle;
le ou les groupes R" étant éventuellement substitués par un ou plusieurs groupes R' identiques ou différents l'un de l'autre :
R₄ représente un atome d'hydrogène ou un groupe C₁₋₆-alkyle ;
R₆ et R₇ représentent indépendamment l'un de l'autre un groupe C₁₋₆-alkyle ;
R représente un groupe méthyle ou éthyle.

9. Composé répondant à la formule générale (V), 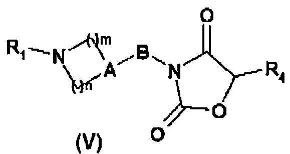 dans laquelle
A représente un atome d'azote ou un groupe CR₂ dans lequel R₂ représente un atome d'hydrogène, de fluor ou un groupe hydroxyle, cyano, trifluorométhyle, C₁₋₆-alkyle, C₁₋₆-alcoxy ;
n représente un nombre entier égal à 2 ou 3 et m représente un nombre entier égal à 2 lorsque A représente un atome d'azote ;
n représente un nombre entier égal à 1, 2 ou 3 et m un nombre entier égal à 1 ou 2 lorsque A représente un groupe CR₂;
B représente une liaison covalente ou un groupe C₁₋₈-alkylène ;
R₁ représente un groupe choisi parmi un phényle, pyridinyle, pyridazinyle, pyrimidinyle, pyrazinyle, triazinyle, oxazolyle, isoxazolyle, thiazolyle, isothiazolyle, imidazolyle, oxadiazolyle, thiadiazolyle, triazolyle, naphtyle, quinolinyle, tétrahydroquinolinyle, isoquinolinyle, tétrahydroisoquinolinyle, phtalazinyle, quinazolinyle, quinoxalinyle, naphthyridinyle, cinnolinyle, imidazopyrimidinyle, thiénopyrimidinyle, benzofuranyle, benzothiényle, benzimidazolyle, benzoxazolyle, benzisoxazolyle, benzothiazolyle, benzisothiazolyle, indazolyle, pyrrolopyridinyle, furopyridinyle, dihydrofuropyridinyle, thiénopyridinyle, dihydrothiénopyridinyle, imidazopyridinyle, pyrazolopyridinyle, oxazolopyridinyle, isoxazolopyridinyle, thiazolopyridinyle ;
le groupe R₁ étant éventuellement substitué par un ou plusieurs groupes R' et/ou R" ;
R' représente un atome d'halogène ou un groupe cyano, nitro, hydroxyle, C₁₋₆-alkyle, C₁₋₆-alcoxy, C₁₋₆-thioalkyle, C₁₋₆-fluoroalkyle, C₁₋₆-fluoroalcoxy, C₁₋₆-fluorothioalkyle, C₃₋₇-cycloalkyle, C ₃₋₇-cycloalkyle-C₁₋₆-alkyléne, azétidinyle, pipéridinyle, pyrrolidinyle, morpholinyle, pipérazinyle, azépinyle, NH₂, NHR₆, NR₆R₇, NR₆COR₇, NR₆SO₂R₇, COR₆, CO₂R₆, SO₂R₆, SO₂NR₆R₇ ou -O- (C₁₋₆-alkylène) -O- ;
R" représente un phényle, imidazolyle, pyridinyle, pyrazinyle, pyridazinyle ou pyrimidinyle;
le ou les groupes R" étant éventuellement substitués par un ou plusieurs groupes R' identiques ou différents l'un de l'autre ;
R₄ représente un atome d'hydrogène ou un groupe C₁₋₆-alkyle ;
R₆ et R₇ représentent indépendamment l'un de l'autre un groupe C₁₋₆-alkyle ;
les composés suivants étant exclus :
* 3-[1-(4-amino-6,7-diméthoxy-2-quinazolinyl)-4-pipéridinyl]-2,4-oxazolidinedione
* 3-[1-(4-amino-6,7-diméthoxy-2-quinazolinyl)-4-pipéridinyl]-5-méthyl-2,4-oxazozidinedione
* 3-[1-(4-amino-6,7-diméthoxy-2-quinazolinyl)-4-pipéridinyl]-5-éthyl-2,4-oxazolidinedione
* 3-[1-(4-amino-6,7-diméthoxy-2-quinazolinyl)-4-pipéridinyl]-5-propyl-2,4-oxazolidinedione
* 3-[1-(4-amino-6,7-diméthoxy-2-quinazilinyl)-4-pipéridinyl]-5-(1-méthyléthyl)-2,4-oxazolidinedione.

10. Composé de formule (I) selon l'une quelconque des revendications 1 à 5, à l'état de base, de sel d'addition à un acide, d'hydrate ou de solvat pharmaceutiquement acceptable, pour son utilisation comme médicament.

11. Composition pharmaceutique contenant au moins un composé de formule (I) selon l'une quelconque des revendications 1 à 5, à l'état de base, de sel d'addition à un acide, d'hydrate ou de solvat pharmaceutiquement acceptable et éventuellement un ou plusieurs excipients pharmaceutiquement acceptables.

12. Utilisation d'un composé de formule (I) selon l'une quelconque des revendications 1 à 5, à l'état de base, de sel d'addition à un acide, d'hydrate ou de solvat pharmaceutiquement acceptable, pour la préparation d'un médicament destiné à prévenir ou à traiter une pathologie dans laquelle les cannabinoïdes endogènes et/ou tous autres substrats métabolisés par l'enzyme FAAH, sont impliqués.

13. Utilisation d'un composé de formule (I) selon l'une quelconque des revendications 1 à 5, à l'état de base, de sel, d'hydrate ou de solvat pharmaceutiquement acceptable, pour la préparation d'un médicament destiné à prévenir ou à traiter les douleurs aiguës ou chroniques, les vertiges, les vomissements, les nausées, les troubles du comportement alimentaire, les pathologies neurologiques et psychiatriques, les maladies neuro-dégénératives aiguës ou chroniques, l'épilepsie, les troubles du sommeil, les maladies cardiovasculaires, l'ischémie rénale, les cancers, les désordres du système immunitaire, les maladies allergiques, les maladies infectieuses parasitaires , virales ou bactériennes, les maladies inflammatoires, l'ostéoporose, les affections oculaires, les affections pulmonaires, les maladies gastro-intestinales ou l'incontinence urinaire.

## Claims

1. Compound corresponding to formula (I) 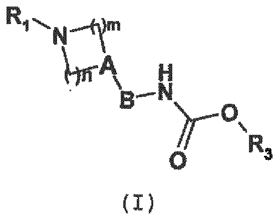 in which
A represents a nitrogen atom or a group CR₂ in which R₂ represents a hydrogen or fluorine atom or a hydroxyl, cyano, trifluoromethyl, C₁₋₆-alkyl or C₁₋₆-alkoxy group; n represents an integer equal to 2 or 3 and m represents an integer equal to 2 when A represents a nitrogen atom;
n represents an integer equal to 1, 2 or 3 and m represents an integer equal to or 2 when A represents a group CR₂;
B represents a covalent bond or a C₁₋₈-alkylene group;
R₁ represents a group chosen from phenyl, pyridyl, pyridazinyl, pyrimidinyl, pyrazinyl, triazinyl, oxazolyl, isoxazolyl, thiazolyl, isothiazolyl, imidazolyl, oxadiazolyl, thiadiazolyl, triazolyl, naphthyl, quinolyl, tetrahydroquinolyl, isoquinolyl, tetrahydroisoquinolyl, phthalazinyl, quinazolinyl, quinoxalinyl, naphthyridinyl, cinnolyl, imidazopyrimidinyl, thienopyrimidinyl, benzofuranyl,
benzothienyl, benzimidazolyl, benzoxazolyl, benzisoxazolyl, benzothiazolyl, benzisothiazolyl, indazolyl, pyrrolopyridyl, furopyridyl, dihydrofuropyridyl, thienopyridyl, dihydrothienopyridyl, imidazopyridyl, pyrazolopyridyl, oxazolopyridyl, isoxazolopyridyl and thiazolopyridyl; the group R₁ optionally being substituted with one or more groups R' and/or R";
R' represents a halogen atom or a cyano, nitro, hydroxyl, C₁₋₆-alkyl, C₁₋₆-alkoxy, C₁₋₆-thioalkyl, C₁₋₆-fluoroalkyl, C₁₋₆-fluoroalkoxy, C₁₋₆-fluorothioalkyl,
C₃₋₇-cycloalkyl, C₃₋₇-cycloalkyl-C₁₋₆-alkylene, azetidinyl, piperidyl, pyrrolidinyl, morpholinyl, piperazinyl, azepinyl, NH₂, NHR₆, NR₆R₇, NR₆COR₇, NR₆SO₂R₇, COR₆, CO₂R₆, SO₂R₆, SO₂NR₆R₇ or -O-(C₁-₆-alkylene)-O- group;
R" represents a phenyl, imidazolyl, pyridyl or pyrimidinyl;
the group(s) R" being optionally substituted with one or more groups R', which may be identical to or different from each other;
R₃ represents a group of general formula CHR₄CONHR₅ in which
R₄ represents a hydrogen atom or a C₁₋₆-alkyl group and R₅ represents a hydrogen atom or a C₁₋₆-alkyl, C₃-₇-cycloalkyl or C₃-₇-cycloalkyl-C₁-₆-alkylene group;
R₆ and R₇ represent, independently of each other, a C₁-₆-alkyl group;
in the form of a base, an acid-addition salt, a hydrate or a solvate.

2. Compound of formula (I) according to Claim 1, **characterized in that**:
A represents a nitrogen atom;
n represents an integer equal to 2 or 3 and m represents an integer equal to 2;
B represents a C₁-₈-alkylene group;
R₁ represents a group chosen from phenyl, pyridyl, pyrimidinyl, thiadiazolyl and naphthyl;
the group R₁ being optionally substituted with one or more groups R' and/or R";
R' represents a halogen atom or a nitro or C₁-₆-fluoroalkyl group;
R" represents a phenyl optionally substituted with one or more groups, which may be identical to or different from each other, chosen from a halogen atom or a cyano, C₁-₆-alkoxy or C₁-₆-fluoroalkoxy group;
R₃ represents a group of general formula CHR₄CONHR₅ in which
R₄ represents a hydrogen atom and
R₅ represents a hydrogen atom or a C₁-₆-alkyl, C₃-₇-cycloalkyl or C₃-₇-cycloalkyl-C₁-₆-alkylene group;
in the form of a base, an acid-addition salt, a hydrate or a solvate.

3. Compound of formula (I) according to Claim 1, **characterized in that**:
A represents a group CR₂ in which R₂ represents a hydrogen or fluorine atom or a hydroxyl group;
m represents an integer equal to 1 or 2 and n represents an integer equal to 1 or 2;
B represents a covalent bond or a C₁-₄-alkylene group;
R₁ represents a group chosen from phenyl, pyridyl, pyridazinyl, pyrimidinyl, pyrazinyl, thiazolyl, quinolyl, isoquinolyl, phthalazinyl, quinazolinyl, quinoxalinyl, naphthyridinyl, furopyridyl, thienopyrimidinyl, imidazopyrimidinyl, benzothiazolyl, benzimidazolyl and benzoxazolyl;
the group R₁ being optionally substituted with one or more groups R' and/or R";
R' represents a halogen atom, a cyano or C₁-₆-alkyl, a C₁-₆-alkoxy, C₁-₆-fluoroalkyl, C₁-₆-fluoroalkoxy, C₃-₇-cycloalkyl, pyrrolidinyl, NH₂, NR₆R₇ or COR₆ group;
R" represents a phenyl, imidazolyl or pyridyl;
the group(s) R" being optionally substituted with one or more groups R', which may be identical to or different from each other;
R₃ represents a group of general formula CHR₄CONHR₅ in which
R₄ represents a hydrogen atom or a C₁-₆-alkyl group, and
R₅ represents a hydrogen atom or a C₁-₆-alkyl, C₃-₇-cycloalkyl or C₃-₇-cycloalkyl-C₁-C₆-alkylene group;
R₆ and R₇ represent, independently of each other, a C₁-₆-alkyl group;
in the form of a base, an acid-addition salt, a hydrate or a solvate.

4. Compound of formula (I) according to Claim 1 or 3, **characterized in that**:
A represents a group CR₂ in which R₂ represents a hydrogen atom;
m is equal to 2 and n is equal to 2;
B represents an ethyl group;
R₁ represents a group chosen from phenyl, pyridyl, pyridazinyl, pyrimidinyl, pyrazinyl, thiazolyl, quinolyl, isoquinolyl, phthalazinyl, quinazolinyl, quinoxalinyl, naphthyridinyl, furopyridyl, thienopyrimidinyl, imidazopyrimidinyl, benzothiazolyl,
benzimidazolyl and benzoxazolyl;
the group R₁ being optionally substituted with one or more groups R' and/or R";
R' represents a halogen atom or a cyano or C₁-₆-alkyl, a C₁-₆-alkoxy, C₁-₆-fluoroalkyl, C₁-₆-fluoroalkoxy, C₃-₇-cycloalkyl, pyrrolidinyl, NH₂, NR₆R₇ or COR₆ group;
R" represents a phenyl, imidazolyl or pyridyl;
the group(s) R" being optionally substituted with one or more groups R', which may be identical to or different from each other;
R₃ represents a group of general formula CHR₄CONHR₅ in which
R₄ represents a hydrogen atom and
R₅ represents a hydrogen atom or a C₁-C₆-alkyl group;
R₆ and R₇ represent, independently of each other, a C₁-₆-alkyl group;
in the form of the base, an acid-addition salt, a hydrate or a solvate.

5. Compound of formula (I) according to any one of Claims 1 to 4, **characterized in that** it is chosen from:
• 2-(methylamino)-2-oxoethyl [3-(4-phenylpiperazin-1-yl)propyl]carbamate
• 2-amino-2-oxoethyl (2-{4-[6-(3-chlorophenyl)-pyridin-2-yl]piperazine-1-yl}ethyl)carbamate
• 2-(methylamino)-2-oxoethyl (2-{4-[6-(3-chlorophenyl)pyridin-2-yl]piperazin-1-yl}ethyl)carbamate
• 2-amino-2-oxoethyl (2-{4-[6-(4-chlorophenyl)-pyridin-2-yl]piperazin-1-yl}ethyl)carbamate
• 2-(methylamino)-2-oxoethyl (2-{4-[6-(4-chlorophenyl)pyridin-2-yl]piperazin-1-yl}-ethyl)-carbamate
• 2-amino-2-oxoethyl (2-{4-[6-(3-cyanophenyl)-pyridin-2-yl]piperazin-1-yl}ethyl)carbamate
• 2-(methylamino)-2-oxoethyl (2-{4-[6-(3-cyanophenyl)pyridin-2-yl]piperazin-1-yl}ethyl)carbamate
• 2-amino-2-oxoethyl (2-{4-[6-(4-cyanophenyl)-pyridin-2-yl]piperazin-1-yl}ethyl)carbamate
• 2-(methylamino)-2-oxoethyl (2-{4-[6-(4-cyanophenyl)pyridin-2-yl]piperazin-1-yl}ethyl)carbamate
• 2-amino-2-oxoethyl (2-{4-[6-(3-methoxyphenyl)-pyridin-2-yl]piperazin-1-yl}ethyl)carbamate
• 2-(methylamino)-2-oxoethyl (2-{4-[6-(3-methoxyphenyl)pyridin-2-yl]piperazin-1-yl}ethyl)carbamate
• 2-amino-2-oxoethyl (2-{4-[6-(4-methoxyphenyl)-pyridin-2-yl]piperazin-1-yl}-ethyl)carbamate
• 2-(methylamino)-2-oxoethyl (2-{4-[6-(4-methoxyphenyl)pyridin-2-yl]piperazin-1-yl}ethyl)carbamate
• 2-amino-2-oxoethyl (2-{4-[6-(3-trifluoromethoxyphenyl)pyridin-2-yl]piperazin-1-yl}ethyl)carbamate
• 2-(methylamino)-2-oxoethyl (2-{4-[6-(3-trifluoromethoxyphenyl)pyridin-2-yl]piperazin-1-yl}ethyl)-carbamate
• 2-amino-2-oxoethyl (2-{4-[6-(4-trifluoromethoxyphenyl)pyridin-2-yl]piperazin-1-yl}ethyl)carbamate
• 2-(methylamino)-2-oxoethyl (2-{4-[6-(4-trifluoromethoxyphenyl)pyridin-2-yl]piperazin-1-yl}ethyl)-carbamate
• 2-amino-2-oxoethyl (2-{4-[5-(3-chlorophenyl)-pyridin-2-yl]piperazin-1-yl}ethyl)carbamate
• 2-(methylamino)-2-oxethyl (2-{4-[5-(3-chlorophenyl)pyridin-2-yl]piperazin-1-yl}ethyl)carbamate
• 2-amino-2-oxoethyl (2-{4-[5-(4-chlorophenyl)-pyridin-2-yl]piperazin-1-yl}ethyl)carbamate
• 2-(methylamino)-2-oxoethyl (2-{4-[5-(4-chlorophenyl)pyridin-2-yl]piperazin-1-yl}ethyl)carbamate
• 2-amino-2-oxoethyl (2-{4-[5-(3-cyanophenyl)-pyridin-2-yl]piperazin-1-yl}ethyl)carbamate
• 2-(methylamino)-2-oxoethyl (2-{4-[5-(3-cyanophenyl)pyridin-2-yl]piperazin-1-yl}ethyl)carbamate
• 2-amino-2-oxoethyl (2-{4-[5-(4-cyanophenyl)-pyridin-2-yl]piperazin-1-yl}ethyl)carbamate
• 2-(methylamino)-2-oxoethyl (2-{4-[5-(4-cyanophenyl)pyridin-2-yl]piperazin-1-yl}ethyl)carbamate
• 2-amino-2-oxoethyl (2-{4-(5-(3-methoxyphenyl)-pyridin-2-yl]piperazin-1-yl}ethyl)carbamate
• 2-(methylamino)-2-oxoethyl (2-{4-[5-(3-methoxyphenyl)pyridin-2-yl]piperazin-1-yl}ethyl)carbamate
• 2-amino-2-oxoethyl (2-{4-[5-(4-methoxyphenyl)-pyridin-2-yl]piperazin-1-yl}ethyl)carbamate
• 2-(methylamino)-2-oxoethyl (2-{4-[5-(4-methoxyphenyl)pyridin-2-yl]piperazin-1-yl}ethyl)carbamate
• 2-amino-2-oxoethyl (2-{4-[5-(3-trifluoromethoxyphenyl)pyridin-2-yl]piperazin-1-yl}ethyl)carbamate
• 2-(methylamino)-2-oxoethyl (2-{4-[5-(3-trifluoromethoxyphenyl)pyridin-2-yl]piperazin-1-yl}ethyl)-carbamate
• 2-amino-2-oxoethyl (2-{4-[5-(4-trifluoromethoxyphenyl)pyridin-2-yl]piperazin-1-yl}ethyl)carbamate
• 2-(methylamino)-2-oxoethyl (2-{4-[5-(4-trifluoromethoxyphenyl)pyridin-2-yl]piperazin-1-yl}ethyl)-carbamate
• 2-(methylamino)-2-oxoethyl {3-[4-(5-nitropyridin-2-yl)-[1,4]diazepan-1-yl]propyl}carbamate
• 2-(methylamino)-2-oxoethyl {3-[4-(3-chloro-5-trifluoromethylpyridin-2-yl)-[1,4]diazepan-1-yl]propyl}carbamate
• 2-(methylamino)-2-oxoethyl {3-[4-(4-trifluoromethylpyrimidin-2-yl)-[1,4]diazepan-1-yl]propyl}-carbamate
• 2-(methylamino)-2-oxoethyl {3-[4-(3-phenyl-[1,2,4]thiadiazol-5-yl)-[1,4]diazepan-1-yl]propyl}carbamate
• 2-(methylamino)-2-oxoethyl [2-(4-naphthalen-1-yl-piperazin-1-yl)ethyl]carbamate
• 2-(methylamino)-2-oxoethyl [3-(4-naphthalen-1-yl-piperazin-1-yl)propyl]carbamate
• 2-amino-2-oxoethyl [1-(3-trifluoromethylphenyl)-azetidin-3-ylmethyl]carbamate
• 2-(methylamino)-2-oxoethyl [1-(3-trifluoromethylphenyl)azetidin-3-ylmethyl]carbamate
• 2-(cyclopropylmethylamino)-2-oxoethyl [1-(3-trifluoromethylphenyl)azetidin-3-ylmethyl]carbamate
• 2-(methylamino)-2-oxoethyl (2-[1-(3-trifluoromethylphenyl)piperidin-4-yl]ethyl)carbamate
• 2-amino-2-oxoethyl (2-[1-(3-trifluoromethylphenyl)piperidin-4-yl]ethyl)carbamate
• 2-(methylamino)-2-oxoethyl (2-[4-fluoro-1-(3-trifluoromethylphenyl)piperidin-4-yl]ethyl)carbamate
• 2-(methylamino)-2-oxoethyl [2-(3,4,5,6-tetrahydro-2H-[1,2']bipyridinyl-4-yl)ethyl]carbamate
• 2-(methylamino)-2-oxoethyl [2-(6'-methyl-3,4,5,6-tetrahydro-2H-[1,2']bipyridinyl-4-yl)ethyl]-carbamate
• 2-(methylamino)-2-oxoethyl [2-(6'-propyl-3,4,5,6-tetrahydro-2H-[1,2']bipyridinyl-4-yl)ethyl]-carbamate
• 2-(methylamino)-2-oxoethyl [2-(6'-isobutyl-3,4,5,6-tetrahydro-2H-[1,2']bipyridinyl-4-yl)-ethyl]carbamate
• 2-amino-2-oxoethyl [2-(6'-isobutyl-3,4,5,6-tetrahydro-2H-[1,2']bipyridinyl-4-yl)ethyl]carbamate
• 2-(methylamino)-2-oxoethyl [2-(6'-cyclopropyl-3,4,5,6-tetrahydro-2H-[1,2']bipyridinyl-4-yl)ethyl]carbamate
• 2-(methylamino)-2-oxothyl [2-(6'-cyclopentyl-3,4,5,6-tetrahydro-2H-[1,2']bipyridinyl-4-yl)-ethyl] carbamate
• 2-(methylamino)-2-oxoethyl [2-(6'-pyrrolidin-1-yl-3,4,5,6-tetrahydro-2H-[1,2']bipyridinyl-4-yl)-ethyl]carbamate
• 2-amino-2-oxoethyl (6'-bromo-3,4,5,6-tetrahydro-2H-[1,2']bipyridinyl-4-yl)carbamate
• 2-amino-2-oxoethyl (6'-bromo-3,4,5,6-tetrahydro-2H-[1,2']bipyridinyl-4-ylmethyl)carbamate
• 2-amino-2-oxoethyl [2-(6'-bromo-3,4,5,6-tetrahydro-2H-[1,2']bipyridinyl-4-yl)ethyl]carbamate
• 2-amino-2-oxoethyl (6'-phenyl-3,4,5,6-tetrahydro-2H-[1,2']bipyridinyl-4-yl)carbamate
• 2-amino-2-oxoethyl (6'-phenyl-3,4,5,6-tetrahydro-2H-[1,2']bipyridinyl-4-ylmethyl)carbamate
• 2-amino-2-oxoethyl [2-(6'-phenyl-3,4,5,6-tetrahydro-2H-[1,2']bipyridinyl-4-yl)ethyl]carbamate
• 2-amino-2-oxoethyl [6'-(4-fluorophenyl)-3,4,5,6-tetrahydro-2H-[1,2']bipyridinyl-4-yl]carbamate
• 2-amino-2-oxoethyl [6'-(4-fluorophenyl)-3,4,5,6-tetrahydro-2H-[1,2']bipyridinyl-4-ylmethyl]-carbamate
• 2-amino-2-oxoethyl {2-[6'-(4-fluorophenyl)-3,4,5,6-tetrahydro-2H-[1,2']bipyridinyl-4-yl]ethyl}carbamate
• 2-amino-2-oxoethyl (3'-bromo-3,4,5,6-tetrahydro-2H-[1,2']bipyridinyl-4-yl)carbamate
• 2-amino-2-oxoethyl (3'-bromo-3,4,5,6-tetrahydro-2H-[1,2']bipyridinyl-4-ylmethyl)carbamate
• 2-amino-2-oxoethyl [2-(3'-bromo-3,4,5,6-tetrahydro-2H-[1,2']bipyridinyl-4-yl)ethyl]carbamate
• 2-amino-2-oxoethyl (3'-cyano-3,4,5,6-tetrahydro-2H-[1,2']bipyridinyl-4-ylmethyl]carbamate
• 2-amino-2-oxoethyl [2-(3'-cyano-3,4,5,6-tetrahydro-2H-[1,2']bipyridinyl-4-yl)ethyl]carbamate
• 2-amino-2-oxoethyl (3'-phenyl-3,4,5,6-tetrahydro-2H-[1,2']bipyridinyl-4-yl)carbamate
• 2-amino-2-oxoethyl (3'-phenyl-3,4,5,6-tetrahydro-2H-[1,2']bipyridinyl-4-ylmethyl)carbamate
• 2-amino-2-oxoethyl [2-(3'-phenyl-3,4,5,6-tetrahydro-2H-[1,2']bipyridinyl-4-yl)ethyl]carbamate
• 2-amino-2-oxoethyl [3'-(4-fluorophenyl)-3,4,5,6-tetrahydr-2H-[1,2']bipyridinyl-4-yl]carbamate
• 2-amino-2-oxoethyl [3'-(4-fluorophenyl)-3,4,5,6-tetrahydro-2H-[1,2']bipyridinyl-4-ylmethyl]-carbamate
• 2-amino-2-oxoethyl (2-[3'-(4-fluorophenyl)-3,4,5,6-tetrahydro-2H-[1,2']bipyridinyl-4-yl]-ethyl)carbamate
• 2-amino-2-oxoethyl (4'-trifluoromethyl-3,4,5,6-tetrahydro-2H-[1,2']bipyridinyl-4-ylmethyl) carbamate
• 2-amino-2-oxoethyl [2-(4'-trifluoromethyl-3,4,5,6-tetrahydro-2H-[1,2']bipyridinyl-4-yl)ethyl]-carbamate
• 2-(methylamino)-2-oxoethyl [2-(4'phenyl-3,4,5,6-tetrahydro-2H-[1,2']bipyridinyl-4-yl)ethyl]-carbamate
• 2-amino-2-oxoethyl [2-(4'-phenyl-3,4,5,6-tetrahydro-2H-[1,2']bipyridinyl-4-yl)ethyl]carbamate
• 2-amino-2-oxoethyl (5'-chloro-3,4,5,6-tetrahydro-2H-[1,2']bipyridinyl-4-ylmethyl)carbamate
• 2-amino-2-oxoethyl [2-(5'-chloro-3,4,5,6-tetrahydro-2H-[1,2']bipyridinyl-4-yl)ethyl]carbamate
• 2-amino-2-oxoethyl (5'-bromo-3,4,5,6-tetrahydro-2H-[1,2']bipyridinyl-4-yl)carbamate
• 2-amino-2-oxoethyl (5'-bromo-3,4,5,6-tetrahydro-2H-[1,2']bipyridinyl-4-ylmethyl)carbamate
• 2-amino-2-oxoethyl [2-(5'-bromo-3,4,5,6-tetrahydro-2H-[1,2']bipyridinyl-4-yl)ethyl]carbamate
• 2-amino-2-oxoethyl (5'-methyl-3,4,5,6-tetrahydro-2H-[1,2']bipyridinyl-4-ylmethyl)carbamate
• 2-amino-2-oxoethyl [2-(5'-methyl-3,4,5,6-tetrahydro-2H-[1,2']bipyridinyl-4-yl)ethyl]carbamate
• 2-amino-2-oxoethyl [2-(5'-isobutyl-3,4,5,6-tetrahydro-2H-[1,2']bipyridinyl-4-yl)ethyl]carbamate
• 2-(methylamino)-2-oxoethyl [2-(5'-isobutyl-3,4,5,6-tetrahydro-2H-[1,2']bipyridinyl-4-yl)ethyl]carbamate
• 2-amino-2-oxoethyl (5'-trifluoromethyl-3,4,5,6-tetrahydro-2H-[1,2']bipyridinyl-4-ylmethyl)-carbamate
• 2-amino-2-oxoethyl [2-(5'-trifluoromethyl-3,4,5,6-tetrahydro-2H-[1,2']bipyridinyl-4-yl)ethyl]-carbamate
• 2-amino-2-oxoethyl (5'-cyano-3,4,5,6-tetrahydro-2H-[1,2']bipyridinyl-4-ylmethyl)carbamate
• 2-amino-2-oxoethyl (5'-acetyl-3,4,5,6-tetrahydro-2H-[1,2']bipyridinyl-4-ylmethyl)carbamate
• 2-amino-2-oxoethyl [2-(5'-acetyl-3,4,5,6-tetrahydro-2H-[1,2']bipyridinyl-4-yl)ethyl]carbamate
• 2-amino-2-oxoethyl (5'-phenyl-3,4,5,6-tetrahydro-2H-[1,2']bipyridinyl-4-yl)carbamate
• 2-amino-2-oxoethyl (5'-phenyl-3,4,5,6-tetrahydro-2H-[2,2']bipyridinyl-4-ylmethyl)carbamate
• 2-amino-2-oxoethyl [5'-(4-fluorophenyl)-3,4,5,6-tetrahydro-2H-[1,2']bipyridinyl-4-yl]carbamate
• 2-amino-2-oxoethyl [5'-(4-fluorophenyl)-3,4,5,6-tetrahydro-2H-[1,2']bipyridinyl-4-ylmethyl]-carbamate
• 2-amino-2-oxoethyl {2-[5'-(4-fluorophenyl)-3,4,5,6-tetrahydro-2H-[1,2']bipyridinyl-4-yl]-ethyl}carbamate
• 2-amino-2-oxoethyl {2-[5'-(4-chlorophenyl) 3,4,5,6-tetrahydro-2H-[1,2']bipyridinyl-4-yl]-ethyl}carbamate
• 2-(methylamino)-2-oxoethyl {2-[5'-(4-chlorophenyl)-3,4,5,6-tetrahydro-2H-[1,2']bipyridinyl-4-yl]ethyl}carbamate
• 2-amino-2-oxoethyl [1-(5-ethylpyrimidin-2-yl)-piperidin-4-ylmethyl]carbamate
• 2-amino-2-oxoethyl {2-[1-(5-ethylpyrimidin-2-yl)-piperidin-4-yl]ethyl}carbamate
• 2-amino-2-oxoethyl [1-(5-propylpyrimidin-2-yl)-piperidin-4-ylmethyl]carbamate
• 2-amino-2-oxoethyl (2-{1-[5-(4-chlorophenyl)-pyrimidin-2-yl)piperidin-4-yl}ethyl)carbamate
• 2-amino-2-oxoethyl [1-(4-trifluoromethylpyrimidin-2-yl)piperidin-4-ylmethyl]carbamate
• 2-amino-2-oxoethyl {2-[1-(4-dimethylamino-6-methylpyrimidin-2-yl)piperidin-4-yl]ethyl}-carbamate
• 2-amino-2-oxoethyl (1-(2-amino-6-methylpyrimidin-4-yl)piperidin-4-ylmethyl]carbamate
• 2-amino-2-oxoethyl {2-[1-(2-amino-6-methylpyrimidin-4-yl)piperidin-4-yl]ethyl}carbamate
• 2-amino-2-oxoethyl [1-(6-methyl-2-pyridin-2-yl-pyrimidin-4-yl)piperidin-4-ylmethyl]carbamate
• 2-amino-2-oxoethyl [1-(6-phenylpyrimidin-4-yl)-piperidin-4-ylmethyl]carbamate
• 2-amino-2-oxoethyl [1-(4-phenylpyrimidin-2-yl)-piperidin-4-ylmethyl]carbamate
• 2-amino-2-oxoethyl [1-(6-imidazol-1-ylpyrimidin-4-yl)piperidin-4-ylmethyl]carbamate
• 2-amino-2-oxoethyl {2-[1-(6-imidazol-1-yl-pyrimidin-4-yl)piperidin-4-yl]ethyl}carbamate
• 2-amino-2-oxoethyl (pyrazin-2-ylpiperidin-4-yl-methyl)carbamate
• 2-amino-2-oxoethyl [2-(1-pyrazin-2-yl-piperidin-4-yl)ethyl]carbamate
• 2-amino-2-oxoethyl [1-(6-aminopyrazin-2-yl)-piperidin-4-ylmethyl]carbamate
• 2-amino-2-oxoethyl {2-[1-(6-aminopyrazin-2-yl)-piperidin-4-yl]ethyl}carbamate
• 2-amino-2-oxoethyl [1-(3,6-dimethylpyrazin-2-yl)-piperidin-4-ylmethyl]carbamate
• 2-amino-2-oxoethyl {2-[1-(3,6-dimethylpyrazin-2-yl)-piperidin-4-yl]ethyl}carbamate
• 2-amino-2-oxoethyl [1-(6-methylpyridazin-3-yl)-piperidin-4-ylmethyl]carbamate
• 2-amino-2-oxoethyl {2-[1-(6-methylpyridazin-3-yl)-piperidin-4-yl]ethyl}carbamate
• 2-amino-2-oxoethyl {2-[1-(6-aminopyridazin-3-yl)-piperidin-4-yl]ethyl}carbamate
• 2-amino-2-oxoethyl [1-(6-phenylpyridazin-3-yl)-piperidin-4-ylmethyl]carbamate
• 2-amino-2-oxoethyl {2-[1-(6-phenylpyridazin-3-yl)-piperidin-4-yl]ethyl}carbamate
• 2-(methylamino)-2-oxoethyl (quinolin-2-yl-piperidin-4-ylmethyl)carbamate
• 2-amino-2-oxoethyl (quinolin-2-ylpiperidin-4-yl-methyl)carbamate
• 1-methyl-2-(methylamino)-2-oxoethyl (quinolin-2-ylpiperidin-4-ylmethyl)carbamate
• 2-(methylamino)-2-oxoethyl [2-(1-quinolin-2-yl-piperidin-4-yl)ethyl]carbamate
• 2-amino-2-oxoethyl [2-(1-quinolin-2-ylpiperidin-4-yl)ethyl]carbamate
• 2-(methylamino)-2-oxoethyl [2-(4-fluoro-1-quinolin-2-ylpiperidin-4-yl)ethyl]carbamate
• 2-(methylamino)-2-oxoethyl (2-(4-hydroxy-1-quinolin-2-ylpiperidin-4-yl)ethyl]carbamate
• 2- (methylamino) -2-oxoethyl {2-[1-(5-chloroquinolin-2-yl)piperidin-4-yl]ethyl}carbamate
• 2-amino-2-oxoethyl {2-[1-(5-chloroquinolin-2-yl)-piperidin-4-yl]ethyl}carbamate
• 2-(methylamino)-2-oxoethyl {2-[1-(5-fluoroquinolin-2-yl)piperidin-4-yl]ethyl}carbamate
• 2- (methylamino) -2-oxoethyl {2-[1-(5-methoxyquinolin-2-yl)piperidin-4-yl]ethyl}carbamate
• 2-(methylamino)-2-oxoethyl [1-(6-chloroquinolin-2-yl)piperidin-4-ylmethyl]carbamate
• 2-amino-2-oxoethyl [1-(6-chloroquinolin-2-yl)-piperidin-4-ylmethyl]carbamate
• 2-(methylamino)-2-oxoethyl {2-[1-(6-chloroquinolin-2-yl)piperidin-4-yl]ethyl}carbamate
• 2-amino-2-oxoethyl {2-[1-(6-chloroquinolin-2-yl)-piperidin-4-yl]ethyl}carbamate
• 2-amino-2-oxoethyl {2-[1-(6-methoxyquinolin-2-yl)-piperidin-4-yl]ethyl}carbamate
• 2-(methylamino)-2-oxoethyl {2-[1-(6-methoxyquinolin-2-yl)piperidin-4-yl]ethyl}carbamate
• 2-(ethylamino)-2-oxoethyl {2-[1-(6-methoxyquinolin-2-yl)piperidin-4-yl]ethyl}carbamate
• 2-(cyclopropylmethylamino)-2-oxoethyl {2-[1-(6-methoxyquinolin-2-yl)piperidin-4-yl]-ethyl}carbamate
• 2-amino-2-oxoethyl {2-[1-(7-chloroquinolin-2-yl)-piperidin-4-yl]ethyl}carbamate
• 2-(methylamino)-2-oxoethyl {2-[1-(7-chloroquinolin-2-yl)piperidin-4-yl]ethyl}carbamate
• 2-(methylamino)-2-oxoethyl {2-[1-(8-chloroquinolin-2-yl)piperidin-4-yl]ethyl}carbamate
• 2-amino-2-oxoethyl {2-[1-(8-chloroquinolin-2-yl)-piperidin-4-yl]ethy}carbamate
• 2-(methylamino)-2-oxoethyl [2-(1-quinolin-3-ylpiperidin-4-yl)ethy]carbamate
• 2-amino-2-oxoethyl [2-(1-quinolin-3-ylpiperidin-4-yl)ethyl]carbamate
• 2-(methylamino)-2-oxoethyl [2-(1-quinolin-4-yl-piperidin-4-yl)ethyl]carbamate
• 2-amino-2-oxoethyl [2-(1-quinolin-4-ylpiperidin-4-yl)ethyl]carbamate
• 2-(methylamino)-2-oxoethyl [2-(1-[1,5]-naphthyridin-2-ylpiperidin-4-yl)ethyl]carbamate
• 2-amino-2-oxoethyl (isoquinolin-1-yl-pyrrolidin-3-ylmethyl)carbamate
• 2-(methylamino)-2-oxoethyl (isoquinolin-1-yl-pyrrolidin-3-ylmethyl)carbamate
• 2-(methylamino)-2-oxoethyl (isoquinolin-1-yl-piperidin-4-yl)carbamate
• 2-(methylamino)-2-oxoethyl (isoquinolin-1-yl-piperidin-4-ylmethyl)carbamate
• 2-(methylamino)-2-oxoethyl [2-(1-isoquinolin-1-yl-piperidin-4-yl)ethyl]carbamate
• 2-amino-2-oxoethyl [2-(1-isoquinolin-1-yl-piperidin-4-yl)ethyl]carbamate
• 2-(methylamino)-2-oxoethyl [3-(1-isoquinolin-1-yl-piperidin-4-yl)propyl]carbamate
• 2-amino-2-oxoethyl {2-[1-(6-fluoroisoquinolin-1-yl)piperidin-4-yl]ethyl}carbamate
• 2-(methylamino)-2-oxoethyl {2-[1-(6-fluoroisoquinolin-1-yl)piperidin-4-yl]ethyl}carbamate
• 2-(methylamino-2-oxoethyl {2-[1-(6-chloroisoquinolin-1-yl)piperidin-4-yl]ethyl}carbamate
• 2-(methylamino)-2-oxoethyl [1-(6-methoxy-isoquinolin-1-yl)piperidin-4-ylmethyl]carbamate
• 2-(methylamino)-2-oxoethyl {2-[1-(6-methoxy-isoinolin-1-yl)piperidin-4-yl]ethyl}carbamate
• 2-amino-2-oxoethyl {2-[1-(6-methoxyisoquinolin-1-yl)piperidin-4-yl]ethyl}carbamate
• 2-(methylamino)-2-oxoethyl {2-[1-(7-chloroisoquinolin-1-yl)piperidin-4-yl]ethyl}carbamate
• 2-(methylamino)-2-oxoethyl {2-[1-(7-methoxy-isoquinolin-1-yl)piperidin-4-yl]ethyl}carbamate
• 2-(methylamino)-2-oxoethyl [2-(1-isoquinolin-3-yl-piperidin-4-yl)ethyl]carbamate
• 2-amino-2-oxoethyl [2-(1-isoquinolin-3-yl-piperidin-4-yl)ethyl]carbamate
• 2-(methylamino)-2-oxoethyl {2-[1-(6-chloroisoquinolin-3-yl)piperidin-4-yl]ethyl}carbamate
• 2-(methylamino)-2-oxoethyl {2-[1-(6-methoxy-isoquinolin-3-yl)piperidin-4-yl]ethyl}carbamate
• 2-amino-2-oxoethyl {2-[1-(3-chloroisoquinolin-6-yl)piperidin-4-yl]ethyl}carbamate
• 2-(methylamino)-2-oxoethyl {2-[1-(3-chloroisoquinolin-6-yl)piperidin-4-yl]ethyl}carbamate
• 2-(methylamino)-2-oxoethyl [2-(1-isoquinolin-4-yl-piperidin-4-yl)ethyl]carbamate
• 2-amino-2-oxoethyl [1-(4-amino-6,7-diemthoxy-quinazolin-2-yl)piperidin-4-ylmethyl]carbamate
• 2-amino-2-oxoethyl {2-[1-(4-amino-6,7-diemthoxy-quinazolin-2-yl)piperidin-4-yl]ethyl}carbamate
• 2-amino-2-oxoethyl (furo[3,2-c]pyridin-4-yl-piperidin-4-ylmethyl)carbamate
• 2-amino-2-oxoethyl [2-(1-furo[3,2-c]pyridin-4-yl-piperidin-4-yl)ethyl]carbamate
• 2-amino-2-oxoethyl (thieno[3,2-d]pyridin-4-yl piperidin-4-ylmethyl)carbamate
• 2-amino-2-oxoethyl (quinoxalin-2-ylpiperidin-4-yl-methyl)carbamate
• 2-(methylamino)-2-oxoethyl [2-(1-quinoxalin-2-ylpiperidin-4-yl)ethyl]carbamate
• 2-amino-2-oxoethyl [2-(1-quinoxalin-2-ylpiperidin-4-yl)ethyl]carbamate
• 2-amino-2-oxoethyl thiazol-2-ylpiperidin-4-yl-methyl)carbamate
• 2-amino-2-oxoethyl [2-(1-thiazol-2-ylpiperidin-4-yl)ethyl]carbamate
• 2-amino-2-oxoethyl (benzothiazol-2-ylpiperidin-4-ylmethyl)carbamate
• 2-amino-2-oxoethyl [2-(1-benzothiazol-2-yl-piperidin-4-yl)ethyl]carbamate
• 2-amino-2-oxoethyl [1-(6-chlorobenzothiazol-2-yl)piperidin-4-ylmethyl]carbamate
• 2-amino-2-oxoethyl {2-[1-(5-chlorobenzothiazol-2-yl)piperidin-4-yl]ethyl}carbamate
• 2-amino-2-oxoethyl [1-(6-methoxybenzothiazol-2-yl)piperidin-4-ylmethyl]carbamate
• 2-amino-2-oxoethyl (benzoxazol-2-ylpiperidin-4-ylmethyl)carbamate
• 2-amino-2-oxoethyl [2-(1-benzooxazol-2-yl-piperidin-4-yl)ethyl]carbamate
• 2-amino-2-oxoethyl [1-(1H-benzoimidazol-2-yl)-piperidin-4-ylmethyl]carbamate
• 2-amino-2-oxoethyl {2-[1-(1H-benzoimidazol-2-yl)-piperidin-4-yl]ethyl}carbamate.

6. Process for preparing a compound of formula (I) according to any one of Claims 1 to 5, comprising the step consisting in
converting the carbamate ester of general formula (Ia) 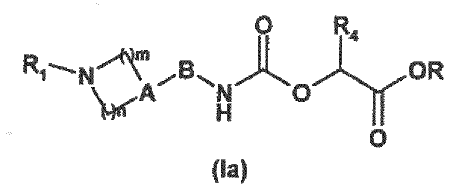 in which n, m, A, B, R₁ and R₄ are as defined in the general formula (I) according to Claim 1 and R represents a methyl or ethyl group,
by aminolysis using an amine of general formula R₅NH₂ in which R₅ is as defined in the general formula (I) according to Claim 1.

7. Process for preparing a compound of formula (I) according to any one of Claims 1 to 5, comprising the step consisting in
converting the oxazolidinedione derivative of general formula (V) 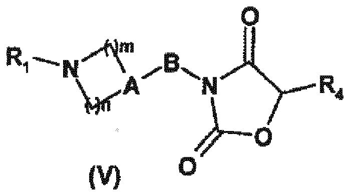 in which n, m, A, B, R₁ and R₄ are as defined in the general formula (I) according to Claim 1,
by aminolysis using an amine of general formula R₅NH₂ in which R₅ is as defined in the general formula (I) according to Claim 1.

8. Compound corresponding to the general formula (Ia) 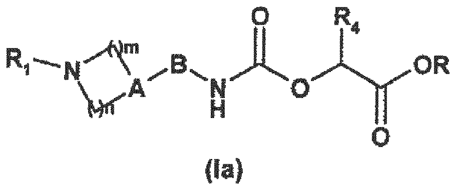 in which
A represents a nitrogen atom or a group CR₂ in which R₂ represents a hydrogen or fluorine atom or a hydroxyl, cyano, trifluoromethyl, C₁-₆-alkyl or C₁-₆-alkoxy group; n represents an integer equal to 2 or 3 and m represents an integer equal to 2 when A represents a nitrogen atom;
n represents an integer equal to 1, 2 or 3 and m represents an integer equal to 1 or 2 when A represents a group CR₂ ;
B represents a covalent bond or a C₁-₈-alkylene group; R₁ represents a group chosen from phenyl, pyridyl, pyridazinyl, pyrimidinyl, pyrazinyl, triazinyl, oxazolyl, isoxazolyl, thiazolyl, isothiazolyl, imidazolyl, oxadiazolyl, thiadiazolyl, triazolyl, naphthyl, quinolyl, tetrahydroquinolyl, isoquinolyl, tetrahydroisoquinolyl, phthalazinyl, quinazolinyl, quinoxalinyl, naphthyridinyl, cinnolyl, imidazopyrimidinyl, thienopyrimidinyl, benzofuranyl, benzothienyl, benzimidazolyl, benzoxazolyl, benzisoxazolyl, benzothiazolyl, benzisothiazolyl, indazolyl, pyrrolopyridyl, furopyridyl, dihydrofuropyridyl, thienopyridyl, dihydrothienopyridyl, imidazopyridyl, pyrazolopyridyl, oxazolopyridyl, isoxazolopyridyl and thiazolopyridyl; the group R₁ optionally being substituted with one or more groups R' and/or R";
R' represents a halogen atom or a cyano, nitro, hydroxyl, C₁-₆-alkyl, C₁-₆-alkoxy, C₁-₆-thioalkyl, C₁-₆-fluoroalkyl, C₁-₆-fluoroalkoxy, C₁-₆-fluorothioalkyl, C₃-₇-cycloalkyl, C₃-₇-cycloalkyl-C₁-₆-alkylene, azetidinyl, piperidyl, pyrrolidinyl, morpholinyl, piperazinyl, azepinyl, NH₂, NHR₆, NR₆R₇, NR₆COR₇, NR₆SO₂R₇, COR₆, CO₂R₆, SO₂R₆, SO₂NR₆R₇ or -O-(C₁-₆-alkylene)-O- group;
R" represents a phenyl, imidazolyl, pyridyl, pyrazinyl, pyridazinyl or pyrimidinyl;
the group(s) R" being optionally substituted with one or more groups R', which may be identical to or different from each other;
R₄ represents a hydrogen atom or a C₁-₆-alkyl group;
R₆ and R₇ represent, independently of each other, a C₁-₆-alkyl group;
R represents a methyl or ethyl group.

9. Compound corresponding to the general formula (V) 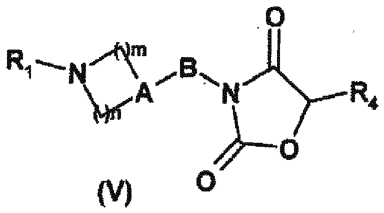 in which
A represents a nitrogen atom or a group CR₂ in which R₂ represents a hydrogen or fluorine atom or a hydroxyl, cyano, trifluoromethyl, C₁-₆-alkyl or C₁-₆-alkoxy group; n represents an integer equal to 2 or 3 and m represents an integer equal to 2 when A represents a nitrogen atom;
n represents an integer equal to 1, 2 or 3 and m represents an integer equal to 1 or 2 when A represents a group CR₂;
B represents a covalent bond or a C₁-₈-alkylene group; R₁ represents a group chosen from phenyl, pyridyl, pyridazinyl, pyrimidinyl, pyrazinyl, triazinyl, oxazolyl, isoxazolyl, thiazolyl, isothiazolyl, imidazolyl, oxadiazolyl, thiadiazolyl, triazolyl, naphthyl, quinolyl, tetrahydroquinolyl, isoquinolyl, tetrahydroisoquinolyl, phthalazinyl, quinazolinyl, inoxalinyl, naphthyridinyl, cinnolyl, imidazopyrimidinyl, thienopyrimidinyl, benzofuranyl, benzothienyl, benzimidazolyl, benzoxazolyl, benzisoxazolyl, benzothiazolyl, benzisothiazolyl, indazolyl, pyrrolopyridyl, furopyridyl,
dihydrofuropyridyl, thienopyridyl, dihydrothienopyridyl, imidazopyridyl, pyrazolopyridyl, oxazolopyridyl, isoxazolopyridyl and thiazolopyridyl; the group R₁ optionally being substituted with one or more groups R' and/or R";
R' represents a halogen atom or a cyano, nitro, hydroxyl, C₁-₆-alkyl, C₁-₆-alkoxy, C₁-₆-thioalkyl, C₁-₆-fluoroalkyl, C₁-₆-fluoroalkoxy; C₁-₆-fluorothioalkyl,
C₃-₇-cycloalkyl, C₃-₇-cycloalkyl-C₁-₆-alkylene, azetidinyl, piperidyl, pyrrolidinyl, morpholinyl, piperazinyl, azepinyl, NH₂, NHR₆, NR₆R₇, NR₆COR₇, NR₆SO₂R₇, COR₆, CO₂R₆, SO₂R₆, SO₂NR₆R₇ or -O- (C₁-₆-alkylene)-O- group;
R" represents a phenyl, imidazolyl, pyridyl, pyrazinyl, pyridazinyl or pyrimidinyl;
the group(s) R" being optionally substituted with one or more groups R', which may be identical to or different from each other;
R₄ represents a hydrogen atom or a C₁-₆-alkyl group;
R₆ and R₇ represent, independently of each other, a C₁-₆-alkyl group;
the following compounds being excluded:
* 3-[1-(4-amino-6,7-dimethoxy-2-quinazolinyl)-4-piperidyl]-2,4-oxazolidinedione
* 3-[1-(4-amino-6,7-dimethoxy-2-quinazolinyl)-4-piperidyl]-5-methyl-2,4-oxazolidinedione
* 3-[1-(4-amino-6,7-dimethoxy-2-quinazolinyl)-4-piperidyl]-5-ethyl-2,4-oxazolidinedione
* 3-[1-(4-amino-6,7-dimethoxy-2-quinazolinyl)-4-piperidyl]-5-propyl-2,4-oxazolidinedione
* 3-[1-(4-amino-6,7-dimethoxy-2-quinazolinyl)-4-piperidyl]-5-(1-methylethyl)-2,4-oxazolidinedione.

10. Compound of formula (I) according to any one of Claims 1 to 5, in the form of the base or of a pharmaceutically acceptable acid-addition salt, hydrate or solvate, for its use as a medicament.

11. Pharmaceutical composition containing at least one compound of formula (I) according to any one of Claims 1 to 5, in the form of the base or of a pharmaceutically acceptable acid-addition salt, hydrate or solvate, and optionally one or more pharmaceutically acceptable excipients.

12. Use of a compound of formula (I) according to any one of Claims 1 to 5, in the form of the base or of a pharmaceutically acceptable acid-addition salt, hydrate or solvate, for the preparation of a medicament for preventing or treating a pathology in which the endogenous cannabinoids and/or any other substrate metabolized by the enzyme FAAH are involved.

13. Use of a compound of formula (I) according to any one of Claims 1 to 5, in the form of the base or of a pharmaceutically acceptable acid-addition salt, hydrate or solvate, for the preparation of a medicament for preventing or treating acute or chronic pain, vertigo, vomiting, nausea, eating disorders, neurological and psychiatric pathologies, acute or chronic neurodegenerative diseases, epilepsy, sleeping disorders, cardiovascular diseases, renal ischaemia, cancers, immune system disorders, allergic diseases, parasitic, viral or bacterial infectious diseases, inflammatory diseases, osteoporosis, ocular complaints, pulmonary complaints, gastrointestinal diseases or urinary incontinence.

## Patentansprüche

1. Verbindung der Formel (I), 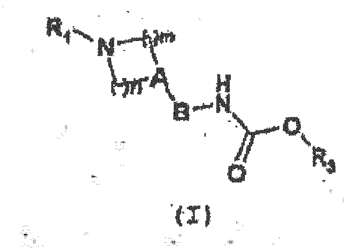 in der
A ein Stickstoffatom oder eine CR₂-Gruppe bedeutet, in der R₂ ein Wasserstoffatom, ein Fluoratom oder eine Hydroxyl-, Cyano-, Trifluormethyl-, C₁-C₆-Alkyl-, C₁-C₆-Alkoxygruppe bedeutet;
n eine ganze Zahl gleich 2 oder 3 bedeutet und m eine ganze Zahl gleich 2 bedeutet, wenn A ein Stickstoffatom bedeutet;
n eine ganze Zahl gleich 1, 2 oder 3 bedeutet und m eine ganze Zahl gleich 1 oder 2 bedeutet, wenn A eine CR₂-Gruppe bedeutet;
B eine kovalente Bindung oder eine C₁-C₈-Alkylengruppe bedeutet;
R₁ eine Gruppe ausgewählt aus Phenyl, Pyridinyl, Pyridazinyl, Pyrimidinyl, Pyrazinyl, Triazinyl, oxazolyl, Isoxazolyl, Thiazolyl, Isothiazolyl, Imidazolyl, Oxadiazolyl, Thiadiazolyl, Triazolyl, Naphthyl, Chinolinyl, Tetrahydrochinolinyl, Isochinolinyl, Tetrahydroisochinolinyl, Phthalazinyl, Chinazolinyl, Chinoxalinyl, Naphthyridinyl, Cinnolinyl, Imidazopyrimidinyl, Thienopyrimidinyl, Benzofuranyl, Benzothienyl, Benzimidazolyl, Benzoxazolyl, Benzisoxazolyl, Benzothiazolyl, Benzisothiazolyl, Indazolyl, Pyrrolopyridinyl, Furopyridinyl, Dihydrofuropyridinyl, Thienopyridinyl, Dihydrothienopyridinyl, Imidazopyridinyl, Pyrazolopyridinyl, Oxazolopyridinyl, Isoxazolopyridinyl, Thiazolopyridinyl bedeutet;
wobei die R₁-Gruppe gegebenenfalls durch einen oder mehrere Gruppen R' und/oder R" substituiert ist;
R' ein Halogenatom oder eine Cyano-, Nitro-, Hydroxyl-, C₁-C₆-Alkyl-, C₁-C₆-Alkoxy-, C₁-C₆-Thioalkyl-, C₁-C₆-Fluoralkyl-, C₁-C₆-Fluoralkoxy-, C₁-C₆-Fluorthioalkyl-, C₃-C₇-Cycloalkyl-, C₃-C₇-Cycloalkyl-C₁-C₆-alkylen-, Azetidinyl-, Piperidinyl-, Pyrrolidinyl-, Morpholinyl-, Piperazinyl-, Azepinyl-, NH₂-, NHR₆-, NR₆R₇-, NR₆COR₇-, NR₆SO₂R₇-, COR₆-, CO₂R₆-, SO₂R₆-, SO₂NR₆R₇- oder -O-(C₁-C₆-Alkyen)-O-Gruppe bedeutet;
R" Phenyl, Imidazolyl, Pyridinyl oder Pyrimidinyl bedeutet;
wobei die Gruppe(n) R" gegebenenfalls durch eine oder mehrere gleiche oder voneinander verschiedene Gruppen R' substituiert ist/sind;
R₃ eine Gruppe der allgemeinen Formel CHR₄CONHR₅ bedeutet, in der
R₄ ein Wasserstoffatom oder eine C₁-C₆-Alkylgruppe bedeutet und
R₅ ein Wasserstoffatom oder eine C₁-C₆-Alkyl-, C₃-C₇-Cycloalkyl-, C₃-C₇-Cycloalkyl-C₁-C₆-alkylengruppe bedeutet;
R₆ und R₇ unabhängig voneinander eine C₁-C₆-Alkylgruppe bedeuten;
als Base, Säureadditionssalz, Hydrat oder Solvat.

2. Verbindung der Formel (I) nach Anspruch 1, **dadurch gekennzeichnet, dass**:
A ein Stickstoffatom bedeutet;
n eine ganze Zahl gleich 2 oder 3 bedeutet und m eine ganze Zahl gleich 2 bedeutet;
B eine C₁-C₈-Alkylengruppe bedeutet;
R₁ eine Gruppe ausgewählt aus Phenyl, Pyridinyl, Pyrimidinyl, Thiadiazolyl, Naphthalenyl bedeutet, wobei die Gruppe R₁ gegebenenfalls durch eine oder mehrere Gruppen R' und/oder R" substituiert ist;
R' ein Halogenatom oder eine Nitro- oder C₁-C₆-Fluoralkylgruppe bedeutet;
R" Phenyl bedeutet, das gegebenenfalls durch eine oder mehrere identische oder voneinander verschiedene Gruppen ausgewählt aus der Reihe Halogenatom oder Cyano-, C₁-C₆-Alkoxy-, C₁-C₆-Fluoralkoxygruppe substituiert ist;
R₃ eine Gruppe der allgemeinen Formel CHR₄CONHR₅ bedeutet, in der
R₄ ein Wasserstoffatom bedeutet und
R₅ ein Wasserstoffatom oder eine C₁-C₆-Alkyl-, C₃-C₇-Cycloalkyl-, C₃-C₇-Cycloalkyl-C₁-C₆-alkylengruppe bedeutet;
als Base, Säureadditionssalz, Hydrat oder Solvat.

3. Verbindung der Formel (I) nach Anspruch 1, **dadurch gekennzeichnet, dass**:
A eine Gruppe CR₂ bedeutet, in der R₂ ein Wasserstoffatom, ein Fluoratom oder eine Hydroxylgruppe bedeutet;
m eine ganze Zahl gleich 1 oder 2 bedeutet und n eine ganze Zahl gleich 1 oder 2 bedeutet;
B eine kovalente Bindung oder eine C₁-C₄-Alkylengruppe bedeutet;
R₁ eine Gruppe ausgewählt aus Phenyl, Pyridinyl, Pyridazinyl, Pyrimidinyl, Pyrazinyl, Thiazolyl, Chinolinyl, Isochinolinyl, Phthalazinyl, Chinazolinyl, Chinoxalinyl, Naphthyridinyl, Furopyridinyl, Thienopyrimidinyl, Imidazopyrimidinyl, Benzothiazolyl, Benzimidazolyl, Benzoxazolyl bedeutet;
wobei die R₁-Gruppe gegebenenfalls durch eine oder mehrere Gruppen R' und/oder R" substituiert ist;
R' ein Halogenatom oder eine Cyano- C₁-C₆-Alkyl-, C₁-C₆-Alkoxy-, C₁-C₆-Fluoralkyl-, C₁-C₆-Fluoralkoxy-, C₃-C₇-Cycloalkyl-, Pyrrolidinyl-, NH₂-, NR₆R₇-, COR₆-Gruppe bedeutet;
R" Phenyl, Imidazolyl oder Pyridinyl bedeutet;
wobei die Gruppe(n) R" gegebenenfalls durch eine oder mehrere Gruppen R', die gleich oder voneinander verschieden sind, substituiert ist/sind;
R₃ eine Gruppe der allgemeinen Formel CHR₄CONHR₅ bedeutet, in der.
R₄ ein Wasserstoffatom oder eine C₁-C₆-Alkylgruppe bedeutet, und
R₅ ein Wasserstoffatom oder eine C₁-C₆-Alkyl-, C₃-C₇-Cycloalkyl-, C₃-C₇-Cycloalkyl-C₁-C₆-alkylengruppe bedeutet;
R₆ und R₇ unabhängig voneinander eine C₁-C₆-Alkylgruppe bedeuten;
als Base, Säureadditionssalz, Hydrat oder Solvat.

4. Verbindung der Formel (I) nach Anspruch 1 oder 3, **dadurch gekennzeichnet, dass**:
A eine Gruppe CR₂ bedeutet, in der R₂ ein Wasserstoffatom bedeutet;
m gleich 2 ist und n gleich 2 ist;
B eine Ethylgruppe bedeutet;
R₁ eine Gruppe ausgewählt aus der Reihe Phenyl, Pyridinyl, Pyridazinyl, Pyrimidinyl, Pyrazinyl, Thiazolyl, Chinolinyl, Isochinolinyl, Phthalazinyl, Chinazolinyl, Chinoxalinyl, Naphthyridinyl, Furopyridinyl, Thienopyrimidinyl, Imidazopyrimidinyl, Benzothiazolyl, Benzimidazolyl, Benzoxazolyl bedeutet;
wobei die R₁-Gruppe gegebenenfalls durch eine oder mehrere Gruppe (n) R' und/oder R" substituiert ist;
R' ein Halogenatom oder eine Cyano-, C₁-C₆-Alkyl-, C₁-C₆-Alkoxy-, C₁-C₆-Fluoralkyl-, C₁-C₆-Fluoralkoxy-, C₃-C₇-Cycloalkyl-, Pyrrolidinyl-, NH₂-, NR₆R₇-, COR₆-Gruppe bedeutet;
R" Phenyl, Imidazolyl oder Pyridinyl bedeutet;
wobei die Gruppe(n) R" gegebenenfalls durch eine oder mehrere Gruppen R', die gleich oder voneinander verschieden sind, substituiert ist/sind;
R₃ eine Gruppe der allgemeinen Formel CHR₄CONHR₅ bedeutet, in der
R₄ ein Wasserstoffatom bedeutet und
R₅ ein Wasserstoffatom oder eine C₁-C₆-Alkylgruppe bedeutet;
R₆ und R₇ unabhängig voneinander eine C₁-C₆-Alkylgruppe bedeuten;
als Base, Säureadditionssalz, Hydrat oder Solvat.

5. Verbindung der Formel (I) nach einem der Ansprüche 1 bis 4, **dadurch gekennzeichnet, dass** sie aus der folgenden Reihe ausgewählt ist:
• [3-(4-Phenylpiperazin-1-yl)propyl]carbaminsäure-2-(methylamino)-2-oxoethylester
• (2-{4-[6-(3-Chlorphenyl)pyridin-2-yl]piperazin-1-yl}ethyl)carbaminsäure-2-amino-2-oxoethylester
• (2-{4-[6-(3-Chlorphenyl)pyridin-2-yl]piperazin-1-yl}ethyl)carbaminsäure-2-(methylamino)-2-oxoethylester
• (2-{4-[6-(4-Chlorphenyl)pyridin-2-yl]piperazin-1-yl}ethyl)carbaminsäure-2-amino-2-oxoethylester
• (2-{4-[6-(4-Chlorphenyl)pyridin-2-yl]piperazin-1-yl}ethyl)carbaminsäure-2-(methylamino)-2-oxoethylester
• (2-{4-[6-(3-Cyanophenyl)pyridin-2-yl]piperazin-1-yl}ethyl)carbaminsäure-2-amino-2-oxoethylester
• (2-{4-[6-(3-Cyanophenyl)pyridin-2-yl]piperazin-1-yl}ethyl)carbaminsäure-2-(methylamino)-2-oxoethylester
• (2-{4-[6-(4-Cyanophenyl)pyridin-2-yl]piperazin-1-yl}ethyl)carbaminsäure-2-amino-2-oxoethylester
• (2-{4-[-6-(4-Cyanophenyl)pyridin-2-yl]piperazin-1-yl}ethyl)carbaminsäure-2-(methylamino)-2-oxoethylester
• (2-{4-[6-(3-Methoxyphenyl)pyridin-2-yl]piperazin-1-yl}ethyl)carbaminsäure-2-amino-2-oxoethylester
• (2-{4-[6-(3-Methoxyphenyl)pyridin-2-yl]piperazin-1-yl}ethyl)carbaminsäure-2-(methylamino)-2-oxoethylester
• (2-{4-[6-(4-Methoxyphenyl)pyridin-2-yl]piperazin-1-yl}ethyl)carbaminsäure-2-amino-2-oxoethylester
• (2-{4-[6-(4-Methoxyphenyl)pyridin-2-yl]piperazin-1-yl}ethyl)carbaminsäure-2-(methylamino)-2-oxoethylester
• (2-{4-[6-(3-Trifluormethoxyphenyl)pyridin-2-yl]piperazin-1-yl}ethyl)carbaminsäure-2-amino-2-oxoethylester
• (2-{4-[6-(3-Trifluormethoxyphenyl)pyridin-2-yl]piperazin-1-yl}ethyl)carbaminsäure-2-(methylamino)-2-oxoethylester
• (2-{4-[6-(4-Trifluormethoxyphenyl)pyridin-2-yl]piperazin-1-yl}ethyl)carbaminsäure-2-amino-2-oxoethylester
• (2-{4-[6-(4-Trifluormethoxyphenyl)pyridin-2-yl]piperazin-1-yl}ethyl)carbaminsäure-2-(methylamino)-2-oxoethylester
• (2-{4-[5-(3-Chlorphenyl)pyridin-2-yl]piperazin-1-yl}ethyl)carbaminsäure-2-amino-2-oxoethylester
• (2-{4-[5-(3-Chlorphenyl)pyridin-2-yl]piperazin-1-yl}ethyl)carbaminsäure-2-(methylamino)-2-oxoethylester
• (2-{4-[5-(4-Chlorphenyl)pyridin-2-yl]piperazin-1-yl}ethyl)carbaminsäure-2-amino-2-oxoethylester
• (2-{4-[5-(4-Chlorphenyl)pyridin-2-yl]piperazin-1-yl}ethyl)carbaminsäure-2-(methylamino)-2-oxoethylester
• (2-{4-[5-(3-Cyanophenyl)pyridin-2-yl]piperazin-1-yl}ethyl)carbaminsäure-2-amino-2-oxoethylester
• (2-{4-[5-(3-Cyanophenyl)pyridin-2-yl]piperazin-1-yl}ethyl)carbaminsäure-2-(methylamino)-2-oxoethylester
• (2-{4-[5-(4-Cyanophenyl)pyridin-2-yl]piperazin-1-yl}ethyl)carbaminsäure-2-amino-2-oxoethylester
• (2-{4-[5-(4-Cyanophenyl)pyridin-2-yl]piperazin-1-yl}ethyl)carbaminsäure-2-(methylamino)-2-oxoethylester
• (2-{4-[5-(3-Methoxyphenyl)pyridin-2-yl]piperazin-1-yl}ethyl)carbaminsäure-2-amino-2-oxoethylester
• (2-{4-[5-(3-Methoxyphenyl)pyridin-2-yl]piperazin-1-yl}ethyl)carbaminsäure-2-(methylamino)-2-oxoethylester
• (2-{4-[5-(4-Methoxyphenyl)pyridin-2-yl]piperazin-1-yl}ethyl)carbaminsäure-2-amino-2-oxoethylester
• (2-{4-[5-(4-Methoxyphenyl)pyridin-2-yl]piperazin-1-yl}ethyl)carbaminsäure-2-(methylamino)-2-oxoethylester
• (2-{4-[5-(3-Trifluormethoxyphenyl)pyridin-2-yl]piperazin-1-yl}ethyl)carbaminsäure-2-amino-2-oxoethylester
• (2-{4-[5-3-Trifluormethoxyphenyl)pyridin-2-yl]piperazin-1-yl}ethyl)carbaminsäure-2-(methylamino)-2-oxoethylester
• (2-{4-[5-(4-Trifluormethoxyphenyl)pyridin-2-yl]piperazin-1-yl}ethyl)carbaminsäure-2-amino-2-oxoethylester
• (2-{4-[5-(4-Trifluormethoxyphenyl)pyridin-2-yl]piperazin-1-yl}ethyl)carbaminsäure-2-(methylamino)-2-oxoethylester
• {3-[4-(5-Nitropyridin-2-yl)[1,4]diazepan-1-yl]propyl}carbaminsäure-2-(methylamino)-2-oxoethylester
• {3-[4-(3-Chlor-5-trifluormethylpyridin-2-yl)[1,4]diazepan-1-yl]propyl}carbaminsäure-2-(methylamino)-2-oxoethylester
• {3-[4-(4-Trifluormethylpyrimidin-2-yl)[1,4]diazepan-1-yl]propyl}carbaminsäure-2-(methylamino)-2-oxoethylester
• {3-[4-(3-Phenyl[1,2,4]thiadiazol-5-yl)[1,4]diazepan-1-yl]propyl}carbaminsäure-2-(methylamino)-2-oxoethylester
• [2-(4-Naphthalen-1-ylpiperazin-1-yl)ethyl}carbaminsäure-2-(methylamino)-2-oxoethylester
• [3-(4-Naphthalen-1-ylpiperazin-1-yl)propyl}carbaminsäure-2-(methylamino)-2-oxoethylester
• [1-(3-Trifluormethylphenyl)azetidin-3-ylmethyl]carbaminsäure-2-amino-2-oxoethylester
• [1-(3-Trifluormethylphenyl)azetidin-3-ylmethyl]carbaminsäure-2-(methylamino)-2-oxoethylester
• [1-(3-Trifluormethylphenyl)azetidin-3-ylmethyl]carbaminsäure-2-(cyclopropylmethylamino)-2-oxoethylester
• (3-[1-(3-Trifluormethylphenyl)piperidin-4-yl]ethyl)carbaminsäure-2-(methylamino)-2-oxoethylester
• (2-[1-(3-Trifluormethylphenyl)piperidin-4-yl]ethyl)carbaminsäure-2-amino-2-oxoethylester
• (2-[4-Fluor-1-(3-trifluormethylphenyl)piperidin-4-yl]ethyl)carbaminsäure-2-(methylamino)-2-oxoethylester
• [2-(3,4,5,6-Tetrahydro-2H-[1,2']bipyridinyl-4-yl)ethyl]carbaminsäure-2-(methylamino)-2-oxoethylester
• [2-(6'-Methyl-3,4,5,6-tetrahydro-2H-[1,2']bipyridinyl-4-yl)ethyl]carbaminsäure-2-(methylamino)-2-oxoethylester
• [2-(6'-Propyl-3,4,5,6-tetrahydro-2H-[1,2']bipyridinyl-4-yl)ethyl]carbaminsäure-2-(methylamino)-2-oxoethylester
• [2-(6'-Isobutyl-3,4,5,6-tetrahydro-2H-[2,2']bipyridinyl-4-yl)ethyl]carbaminsäure-2-(methylamino)-2-oxoethylester
• [2-(6'-Isobutyl-3,4,5,6-tetrahydro-2H-[1,2']bipyridinyl-4-yl)ethyl]carbaminsäure-2-amino-2-oxoethylester
• [2-(6'-Cyclopropyl-3,4,5,6-tetrahydro-2H-[1,2']bipyridinyl-4-yl)ethyl]carbaminsäure-2-(methylamino)-2-oxoethylester
• [2-(6'-Cyclopentyl-3,4,5,6-tetrahydro-2H-[1,2']bipyridinyl-4-yl)ethyl]carbaminsäure-2-(methylamino)-2-oxoethylester
• [2-(6'-Pyrrolidin-1-yl-3,4,5,6-tetrahydro-2H-[1,2']bipyridinyl-4-yl)ethyl]carbaminsäure-2-(methylamino)-2-oxoethylester
• (6'-Brom-3,4,5,6-tetrahydro-2H-[1,2']bipyridinyl-4-yl)ethyl]carbaminsäure-2-amino-2-oxoethylester
• (6'-Brom-3,4,5,6-tetrahydro-2H-[1,2']bipyridinyl-4-ylmethyl)carbaminsäure-2-amino-2-oxoethylester
• [2-(6'-Brom-3,4,5,6-tetrahydro-2H-[1,2']bipyridinyl-4-y)ethyl]carbaminsäure-2-amino-2-oxoethylester
• (6'-Phenyl-3,4,5,6-tetrahydro-2H-[1,2']bipyridinyl-4-yl)carbaminsäure-2-amino-2-oxoethylester
• (6'-Phenyl-3,4,5,6-tetrahydro-2H-[1,2']bipyridinyl-4-ylmethyl)carbaminsäure-2-amino-2-oxoethylester
• [2-(6'-Phenyl-3,4,5,6-tetrahydro-2H-[1,2']bipyridinyl-4-y)ethyl]carbaminsäure-2-amino-2-oxoethylester
• [6'-(4-Fluorphenyl)-3,4,5,6-tetrahydro-2H-[1,2']bipyridinyl-4-yl]carbaminsäure-2-amino-2-oxoethylester
• [6'-(4-Fluorphenyl)-3,4,5,6-tetrahydro-2H-[1,2']bipyridinyl-4-ylmethyl]carbaminsäure-2-amino-2-oxoethylester
• {2-[6'-(4-Fluorphenyl)-3,4,5,6-tetrahydro-2H-[1,2']bipyridinyl-4-y]ethyl}carbaminsäure-2-amino-2-oxoethylester
• (3'-Brom-3,4,5,5-tetrahydro-2H-[1,2']bipyridinyl-4-yl)carbaminsäure-2-amino-2-oxoethylester
• (3'-Brom-3,4,5,6-tetrahydro-2H-[1,2']bipyridinyl-4-ylmethyl)carbaminsäure-2-amino-2-oxoethylester
• [2-(3'-Brom-3,4',5,6-tetrahydro-2H-[1,2']bipyridinyl-4-yl)ethyl]carbaminsäure-2-amino-2-oxoethylester
• (3'-Cyano-3,4,5,6-tetrahydro-2H-[1,2']bipyridinyl-4-ylmethyl)carbaminsäure-2-amino-2-oxoethylester
• [2-(3'-cyano-3,4,5,6-tetrahydro-2H-[1,2']bipyridinyl-4-yl)ethyl]carbaminsäure-2-amino-2-oxoethylester
• (3'-Phenyl-3,4,5,6-tetrahydro-2H-[1,2']bipyridinyl-4-yl)carbaminsäure-2-amino-2-oxoethylester
• (3'-Phenyl-3,4,5,6-tetrahydro-2H-[1,2']bipyridinyl-4-ylmethyl)carbaminsäure-2-amino-2-oxoethylester
• [2-(3'-Phenyl-3,4,5,6-tetrahydro-2H-[1,2']bipyridinyl-4-yl)ethyl]carbaminsäure-2-amino-2-oxoethylester
• [3'-(4-Fluorphenyl)-3,4,5,6-tetrahydro-2H-[1,2']bipyridinyl-4-yl]carbaminsäure-2-amino-2-oxoethylester
• [3'-(4-Fluorphenyl)-3,4,5,6-tetrahydro-2H-[1,2']bipyridinyl-4-ylmethyl]carbaminsäure-2-amino-2-oxoethylester
• {2-[3'-(4-Fluorphenyl)-3,4,5,6-tetrahydro-2H-[1,2']bipyridinyl-4-yl]ethyl}carbaminsäure-2-amino-2-oxoethylester
• (4'-Trifluormethyl-3,4,5,6-tetrahydro-2H-[1,2']bipyridinyl-4-ylmethyl]carbaminsäure-2-amino-2-oxoethylester
• [2-(4'-Trifluormethyl-3,4,5,6-tetrahydro-2H-[1,2']bipyridinyl-4-yl)ethyl]carbaminsäure-2-amino-2-oxoethylester
• [2-(4'-Phenyl-3,4,5,6-tetrahydro-2H-[1,2']bipyridinyl-4-yl)ethyl]carbaminsäure-2-(methylamino)-2-oxoethylester
• [2-(4'-Phenyl-3,4,5,6-tetrahydro-2H-[1,2']bipyridinyl-4-yl)ethyl]carbaminsäure-2-amino-2-oxoethylester
• (5'-Chlor-3,4,5,6-tetrahydro-2H-[1,2']bipyridinyl-4-ylmethyl)carbaminsäure-2-amino-2-oxoethylester
• [2-(5'-Chlor-3,4,5,6-tetrahydro-2H-[1,2']bipyridinyl-4-yl)ethyl]carbaminsäure-2-amino-2-oxoethylester
• (5'-Brom-3,4,5,6-tetrahydro-2H-[1,2']bipyridinyl-4-yl)carbaminsäure-2-amino-2-oxoethylester
• (5'-Brom-3,4,5,6-tetrahydro-2H-[1,2']bipyridinyl-4-ylmethyl)carbaminsäure-2-amino-2-oxoethylester
• [2-(5'-Brom-3,4,5,6-tetrahydro-2H-[1,2']bipyridinyl-4-yl)ethyl]carbaminsäure-2-amino-2-oxoethylester
• (5'-Methyl-3,4,5,6-tetrahydro-2H-[1,2']bipyridinyl-4-ylmethyl)carbaminsäure-2-amino-2-oxoethylester
• [2-(5'-Methyl-3,4,5,6-tetrahydro-2H-[1,2']bipyridinyl-4-yl)ethyl]carbaminsäure-2-amino-2-oxoethylester
• [2-(5'-Isobutyl-3,4,5,6-tetrahydro-2H-[1,2']bipyridinyl-4-yl)ethyl]carbaminsäure-2-amino-2-oxoethylester
• [2-(5'-Isobutyl-3,4,5,6-tetrahydro-2H-[1,2']bipyridinyl-4-yl)ethyl]carbaminsäure-2-(methylamino)-2-oxoethylester
• (5'-Trifluormethyl-3,4,5,6-tetrahydro-2H-[1,2']bipyridinyl-4-ylmethyl)carbaminsäure-2-amino-2-oxoethylester
• [2-(5'-Trifluormethyl-3,4,5,6-tetrahydro-2H-[1,2,]bipyridinyl-4-yl)ethyl]carbaminsäure-2-amino-2-oxoethylester
• (5'-Cyano-3,4,5,6-tetrahydro-2H-[1,2']bipyridinyl-4-ylmethyl)carbaminsäure-2-amino-2-oxoethylester
• (5'-Acetyl-3,4,5,6-tetrahydro-2H-[1,2']bipyridinyl-4-ylmethyl)carbaminsäure-2-amino-2-oxoethylester
• [2-(5'-Acetyl-3,4,5,6-tetrahydro-2H-[1,2']bipyridinyl-4-yl}ethyl]carbaminsäure-2-amino-2-oxoethylester
• (5'-Phenyl-3,4,5,6-tetrahydro-2H-[1,2]bipyridinyl-4-yl)carbaminsäure-2-amino-2-oxoethylester
• (5'-Phenyl-3,4,5,6-tetrahydro-2H-[1,2']bipyridinyl-4-ylmethyl)carbaminsäure-2-amino-2-oxoethylester
• [5'-(4-Fluorphenyl}-3,4,5,5-tetrahydro-2H-[1,2']bipyridinyl-4-yl]carbaminsäure-2-amino-2-oxoethylester
• [5'-(4-Fluorphenyl)-3,4,5,6-tetrahydro-2H-[1,2']bipyridinyl-4-ylmethyl]carbaminsäure-2-amino-2-oxoethylester
• {2-[5'-(4-Fluorphenyl)-3,4,5,6-tetrahydro-2H-[1,2']bipyridinyl-4-yl]ethyl}carbaminsäure-2-amino-2-oxoethylester
• {2-[5'-(4-Chlorphenyl}-3,4,5,6-tetrahydro-2H-[1,2']bipyridinyl-4-yl]ethyl}carbaminsäure-2-amino-2-oxoethylester
• {2-[5'-(4-Chlorphenyl)-3,4,5,6-tetrahydro-2H-[1,2']bipyridinyl-4-yl]ethyl}carbaminsäure-2-(methylamino)-2-oxoethylester
• [1-(5-Ethylpyrimidin-2-yl)piperidin-4-ylmethyl]carbaminsäure-2-amino-2-oxoethylester
• {2-[1-(5-Ethylpyrimidin-2-yl)piperidin-4-yl]ethyl}carbaminsäure-2-amino-2-oxoethylester
• [1-(5-Propylpyrimidin-2-yl)piperidin-4-ylmethyl]carbaminsäure-2-amino-2-oxoethylester
• (2-{1-[5-(4-Chlorphenyl)pyrimidin-2-yl]piperidin-4-yl}ethyl)carbaminsäure-2-amino-2-oxoethylester
• [1-(4-Trifluormethylpyrimidin-2-yl)piperidin-4-ylmethyl]carbaminsäure-2-amino-2-oxoethylester
• {2-[1-(4-Dimethylamino-6-methylpyrimidin-2-yl)piperidin-4-yl]ethyl}carbaminsäure-2-amino-2-oxoethylester
• [1-(2-Amino-6-methylpyrimidin-4-yl)piperidin-4-ylmethyl]carbaminsäure-2-amino-2-oxoethylester
• {2-[1-(2-Amino-6-methylpyrimidin-4-yl)piperidin-4-yl]ethyl}carbaminsäure-2-amino-2-oxoethylester
• [1-(6-Methyl-2-pyridin-2-ylpyrimidin-4-yl)piperidin-4-ylmethyl]carbamänsäure-2-amino-2-oxoethylester
• [1-(6-Phenylpyrimidin-4-yl)piperidin-4-ylmethyl]carbaminsäure-2-amino-2-oxoethylester
• [1-(4-Phenylpyrimidin-2-yl)piperidin-4-ylmethyl]carbaminsäure-2-amino-2-oxoethylester
• [1-(6-Imidazol-1-ylpyrimidin-4-yl)piperidin-4-ylmethyl]carbaminsäure-2-amino-2-oxoethylester
• {2-[1-{6-Imidazol-1-ylpyrimidin-4-yl)piperidin-4-yl]ethyl}carbaminsäure-2-amino-2-oxoethylester
• Pyrazin-2-ylpiperidin-4-ylmethyl]carbaminsäure-2-amino-2-oxoethylester
• [2-(1-Pyrazin-2-ylpiperidin-4-yl)ethyl]carbaminsäure-2-amino-2-oxoethylester
• [1-(6-Aminopyrazin-2-yl)piperidin-4-ylmethyl]carbaminsäure-2-amino-2-oxoethylester
• {2-[1-(6-Aminopyrazin-2-yl)piperidin-4-yl]ethyl}carbaminsäure-2-amino-2-oxoethylester
• [1-(3,6-Dimethylpyrazin-2-yl)piperidin-4-ylmethyl]carbaminsäure-2-amino-2-oxoethylester
• {2-[1-(3,6-Dimethylpyrazin-2-yl)piperidin-4-yl]ethyl}carbaminsäure-2-amino-2-oxoethylester
• [1-(6-Methylpyridazin-3-yl)piperidin-4-ylmethyl]carbaminsäure-2-amino-2-oxoethylester
• {2-[1-(6-Methylpyridazin-3-yl)piperidin-4-yl]ethyl}carbaminsäure-2-amino-2-oxoethylester
• {2-[1-(6-Aminopyridazin-3-yl)piperidin-4-yl]ethyl}carbaminsäure-2-amino-2-oxoethylester
• [1-(6-Phenylpyridazin-3-yl)piperidin-4-ylmethyl]carbaminsäure-2-amino-2-oxoethylester
• {2-[1-(6-Phenylpyridazin-3-yl)piperidin-4-yl]ethyl}carbaminsäure-2-amino-2-oxoethylester
• Chinolin-2-ylpiperidin-4-ylmethyl)carbaminsäure-2-(methylamino)-2-oxoethylester
• Chinolin-2-ylpiperidin-4-ylmethyl)carbaminsäure-2-amino-2-oxoethylester
• Chinolin-2-ylpiperidin-4-ylmethyl)carbaminsäure-1-methyl-2-(methylamino)-2-oxoethylester
• [2-(1-Chinolin-2-ylpiperidin-4-yl)ethyl]carbaminsäure-2-(methylamino)-2-oxoethylester
• [2-(1-Chinolin-2-ylpiperidin-4-yl)ethyl]carbaminsäure-2-amino-2-oxoethylester
• [2-(4-Fluor-1-chinolin-2-ylpiperidin-4-yl)ethyl]carbaminsäure-2-(methylamino)-2-oxoethylester
• [2-(4-Hydroxy-1-chinolin-2-ylpiperidin-4-yl)ethyl]carbaminsäure-2-(methylamino)-2-oxoethylester
• {2-[1-(5-Chlorchinolin-2-yl)piperidin-4-yl]ethyl}carbaminsäure-2-(methylamino)-2-oxoethylester
• {2-[1-(5-Chlorchinolin-2-yl)piperidin-4-yl]ethyl)carbaminsäure-2-amino-2-oxoethylester
• {2-[1-(5-Fluorchinolin-2-yl)piperidin-4-yl]ethyl}carbaminsäure-2-(methylamino)-2-oxoethylester
• {2-[1-(5-Methoxychinolin-2-yl)piperidin-4-yl]ethyl}carbaminsäure-2-(methylamino)-2-oxoethylester
• [1-(6-Chlorchinolin-2-yl)piperidin-4-ylmethyl]carbaminsäure-2-(methylamino)-2-oxoethylester
• [1-(6-Chlorchinolin-2-yl)piperidin-4-ylmethyl]carbaminsäure-2-amino-2-oxoethylester
• {2-[1-(6-Chlorchinolin-2-yl)piperidin-4-yl]ethyl}carbaminsäure-2-(methylamino)-2-oxoethylester
• {2-[1-(6-Chlorchinolin-2-yl}piperidin-4-yl]ethyl}carbaminsäure-2-amino-2-oxoethylester
• {2-[1-(6-Methoxychinolin-2-yl)piperidin-4-yl]ethyl}carbaminsäure-2-amino-2-oxoethylester
• {2-[1-(6-Methoxychinolin-2-yl)piperidin-4-yl]ethyl}carbaminsäure-2-(methylamino)-2-oxoethylester
• {2-[1-(6-Methoxychinolin-2-yl)piperidin-4-yl]ethyl}carbaminsäure-2-(ethylamino)-2-oxoethylester
• {2-[1-(6-Methoxychinolin-2-yl)piperidin-4-yl]ethyl}carbaminsäure-2-(cyclopropylmethylamino)-2-oxoethylester
• {2-[1-(7-Chlorchinolin-2-yl)piperidin-4-yl]ethyl}carbaminsäure-2-amino-2-oxoethylester
• {2-[1-(7-Chlorchinolin-2-yl)piperidin-4-yl]ethyl}carbaminsäure-2-(methylamino)-2-oxoethylester
• {2-[1-(8-Chlorchinolin-2-yl)piperidin-4-yl]ethyl}carbaminsäure-2-(methylamino)-2-oxoethylester
• {2-[1-(8-Chlorchinolin-2-yl)piperidin-4-yl]ethyl}carbaminsäure-2-amino-2-oxoethylester
• [2-(1-Chinolin-3-yl)piperidin-4-yl)ethyl]carbaminsäure-2-(methylamino)-2-oxoethylester
• [2-(1-Chinolin-3-yl)piperidin-4-yl)ethyl]carbaminsäure-2-amino-2-oxoethylester
• [2-(1-Chinolin-4-ylpiperidin-4-yl)ethyl]carbaminsäure-2-(methylamino)-2-oxoethylester
• [2-(1-Chinolin-4-ylpiperidin-4-yl)ethyl]carbaminsäure-2-amino-2-oxoethylester
• [2-(1-[1,5]Naphthyridin-2-ylpiperidin-4-yl)ethyl]carbaminsäure-2-(methylamino)-2-oxoethylester
• Isochinolin-1-ylpyrrolidin-3-ylmethyl)carbaminsäure-2-amino-2-oxoethylester
• Isochinolin-1-ylpyrrolidin-3-ylmethyl)carbaminsäure-2-(methylamino)-2-oxoethylester
• Isochinolin-1-ylpiperidin-4-yl)carbaminsäure-2-(methylamino)-2-oxoethylester
• Isochinolin-1-ylpiperidin-4-ylmethyl)carbaminsäure-2-(methylamino)-2-oxoethylester
• [2-(1-Isochinolin-1-ylpiperidin-4-yl)ethyl]earbaminsäure-2-(methylamino)-2-oxoethylester
• [2-(1-Isochinolin-1-ylpiperidin-4-yl)ethyl]carbaminsäure-2-amino-2-oxoethylester
• [3-(1-Isochinolin-1-ylpiperidin-4-yl)propyl]carbaminsäure-2-(methylamino)-2-oxoethylester
• {2-[1-(6-Fluorisochinolin-1-yl)piperidin-4-yl]ethyl}carbaminsäure-2-amino-2-oxoethylester
• {2-[1-(6-Fluorisochinolin-1-yl}piperidin-4-yl]ethyl}carbaminsäure-2-(methylamino)-2-oxoethylester
• {2-[1-(6-Chlorisochinolin-1-yl)piperidin-4-yl]ethyl}carbaminsäure-2-(methylamino)-2-oxoethylester
• [1-(6-Methoxyisochinolin-1-yl)piperidin-4-ylmethyl]carbaminsäure-2-(methylamino)-2-oxoethylester
• {2-[1-(6-Methoxyisochinolin-1-yl)piperidin-4-yl]ethyl}carbaminsäure-2-(methylamino)-2-oxoethylester
• {2-[2-(6-Methoxyisochinolin-1-yl)piperidin-4-yl]ethyl}carbaminsäure-2-amino-2-oxoethylester
• {2-[1-(7-Chlorisochinolin-1-yl)piperidin-4-yl]ethyl}carbaminsäure-2-(methylamino)-2-oxoethylester
• {2-[1-(7-Methoxyisochinolin-1-yl)piperidin-4-yl]ethyl}carbaminsäure-2-(methylamino)-2-oxoethylester
• [2-(1-Isochinolin-3-ylpiperidin-4-yl)ethyl]carbaminsäure-2-(methylamino)-2-oxoethylester
• [2-(1-Isochinolin-3-ylpiperidin-4-yl}ethyl]carbaminsäure-2-amino-2-oxoethylester
• {2-[1-(6-Chlorisochinolin-3-yl)piperidin-4-yl]ethyl}carbaminsäure-2-(methylamino)-2-oxoethylester
• {2-[1-(6-Methoxyisochinolin-3-yl)piperidin-4-yl]ethyl}carbaminsäure-2-(methylamino)-2-oxoethylester
• {2-[1-(3-Chlorisochinolin-6-yl}piperidin-4-yl]ethyl}carbaminsäure-2-amino-2-oxoethylester
• {2-[1-(3-Chlorisochinolin-6-yl)piperidin-4-yl]ethyl}carbaminsäure-2-(methylamino)-2-oxoethylester
• [2-(1-Isochinolin-4-ylpiperidin-4-yl)ethyl]carbaminsäure-2-(methylamino)-2-oxoethylester
• [1-(4-Amino-6,7-dimethoxychinazolin-2-yl)piperidin-4-ylmethyl]carbaminsäure-2-amino-2-oxoethylester
• {2-[1-(4-Amino-6,7-dimethoxychinazolin-2-yl)piperidin-4-yl]ethyl}carbaminsäure-2-amino-2-oxoethylester
• Furo[3,2-c]pyridin-4-ylpiperzdin-4-ylmethyl]carbaminsäure-2-amino-2-oxoethylester
• [2-(1-Furo[3,2-c]pyridin-4-ylpiperidin-4-yl)ethyl]carbaminsäure-2-amino-2-oxoethylester
• Thieno[3,2-d]pyrimidin-4-ylpiperidin-4-ylmethyl)carbaminsäure-2-amino-2-oxoethylester
• Chinoxalin-2-ylpiperidin-4-ylmethyl)carbaminsäure-2-amino-2-oxoethylester
• [2-(1-Chinoxalin-2-ylpiperidin-4-yl)ethyl]carbaminsäure-2-(methylamino)-2-oxoethylester
• [2-(1-Chinoxalin-2-ylpiperidin-4-yl)ethyl]carbaminsäure-2-amino-2-oxoethylester
• Thiazol-2-ylpiperidin-4-ylmethyl)carbaminsäure-2-amino-2-oxoethylester
• [2-(1-Thiazol-2-ylpiperidin-4-yl)ethyl]carbaminsäure-2-amino-2-oxoethylester
• Benzothiazol-2-ylpiperidin-4-ylmethyl)carbaminsäure-2-amino-2-oxoethylester
• [2-(1-Benzothiazol-2-ylpiperidin-4-yl)ethyl]carbaminsäure-2-amino-2-oxoethylester
• [1-(6-Chlorbenzothiazol-2-yl)piperidin-4-ylmethyl)carbaminsäure-2-amino-2-oxoethylester
• {2-[1-(6-Chlorbenzothiazol-2-yl)piperidin-4-yl]ethyl}carbaminsäure-2-amino-2-oxoethylester
• [1-(6-Methoxybenzothiazol-2-yl)piperidin-4-ylmethyl)carbaminsäure-2-amino-2-oxoethylester
• Benzooxazol-2-ylpiperidin-4-ylmethyl)carbaminsäure-2-amino-2-oxoethylester
• [2-(1-Benzooxazol-2-ylpiperidin-4-yl)ethyl]carbaminsäure-2-amino-2-oxoethylester
• [1-(1H-Benzoimidazol-2-yl)piperidin-4-ylmethyl)carbaminsäure-2-amino-2-oxoethylester
• {2-[1-(1H-Benzoimidazol-2-yl)piperidin-4-yl]ethyl}carbaminsäure-2-amino-2-oxoethylester.

6. Verfahren zur Herstellung einer Verbindung der Formel (I) nach einem der Ansprüche 1 bis 5, umfassend den Schritt, dass man
den Carbaminsäureester der allgemeinen Formel (Ia) 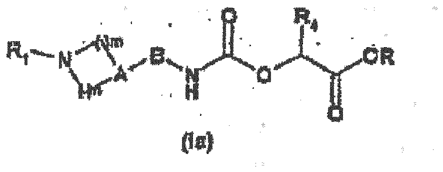 , in der n, m, A, B, R₁ und R₄ wie in der allgemeinen Formel (I) nach Anspruch 1 definiert sind und R eine Methyl- oder Ethylgruppe bedeutet,
durch Aminolyse mit einem Amin der allgemeinen Formel R₅NH₂, in der R₅ wie in der allgemeinen Formel (I) nach Anspruch 1 definiert ist, umwandelt.

7. Verfahren zur Herstellung einer Verbindung der Formel (I) nach einem der Ansprüche 1 bis 5, umfassend den Schritt, dass man
das Oxazolidindionderivat der allgemeinen Formel (V) 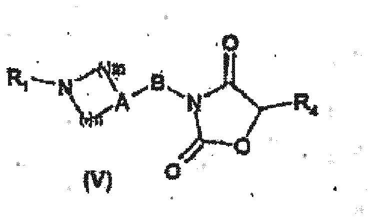 , in der n, m, A, B, R₁ und R₄ wie in der allgemeinen Formel (I) nach Anspruch 1 definiert sind,
durch Aminolyse mit einem Amin der allgemeinen Formel R₅NH₂, in der R₅ wie in der allgemeinen Formel (I) nach Anspruch 1 definiert ist, umwandelt.

8. Verbindung der allgemeinen Formel (Ia), 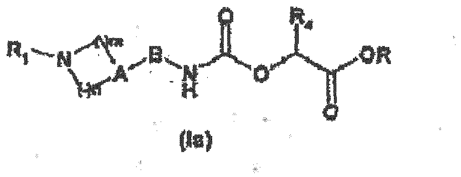 in der
A ein Stickstoffatom oder eine CR₂-Gruppe bedeutet, in der R₂ ein Wasserstoffatom, ein Fluoratom oder eine Hydroxyl-, Cyano-, Trifluormethyl-, C₁-C₆-Alkyl-, C₁-C₆-Alkoxygruppe bedeutet;
n eine ganze Zahl gleich 2 oder 3 bedeutet und m eine ganze Zahl gleich 2 bedeutet, wenn A ein Stickstoffatom bedeutet;
n eine ganze Zahl gleich 1, 2 oder 3 bedeutet und m eine ganze Zahl gleich 1 oder 2 bedeutet, wenn A eine CR₂-Gruppe bedeutet;
B eine kovalente Bindung oder eine C₁-C₈-Alkylengruppe bedeutet;
R₁ eine Gruppe ausgewählt aus Phenyl, Pyridinyl, Pyridazinyl, Pyrimidinyl, Pyrazinyl, Triazinyl, Oxazolyl, Isoxazolyl, Thiazolyl, Isothiazolyl, Imidazolyl, Oxadiazolyl, Thiadiazolyl, Triazolyl, Naphthyl, Chinolinyl, Tetrahydrochinolinyl, Isochinolinyl, Tetrahydroisochinolinyl, Phthalazinyl, Chinazolinyl, Chinoxalinyl, Naphthyridinyl, Cinnolinyl, Imidazopyrimidinyl, Thienopyrimidinyl, Benzofuranyl, Benzothienyl, Benzimidazolyl, Benzoxazolyl, Benzisoxazolyl, Benzothiazolyl, Benzisothiazolyl, Indazolyl, Pyrrolopyridinyl, Furopyridinyl, Dihydrofuropyridinyl, Thienopyridinyl, Dihydrothienopyridinyl, Imidazopyridinyl, Pyrazolopyridinyl, Oxazolopyridinyl, Isoxazolopyridinyl, Thiazolopyridinyl bedeutet;
wobei die R₁-Gruppe gegebenenfalls durch eine oder mehrere Gruppen R' und/oder R'' substituiert ist;
R' ein Halogenatom oder eine Cyano-, Nitro-, Hydroxyl-, C₁-C₆-Alkyl-, C₁-C₆-Alkoxy-, C₁-C₆-Thioalkyl-, C₁-C₆-Fluoralkyl-, C₁-C₆-Fluoralkoxy-, C₁-C₆-Fluorthioalkyl-, C₃-C₇-Cycloalkyl-, C₃-C₇-Cycloalkyl-C₁-C₆-alkylen-, Azetidinyl-, Piperidinyl-, Pyrrolidinyl-, Morpholinyl-, Piperazinyl-, Azepinyl-, NH₂-, NHR₆-, NR₆R₇-, NR₆COR₇-, NR₆SO₂R₇-, COR₆-, CO₂R₆-, SO₂R₆-, SO₂NR₆R₇- oder -O-(C₁-C₆-Alkyen)-O-Gruppe bedeutet;
R'' phenyl, Imidazolyl, Pyridinyl, Pyrazinyl, Pyridazinyl oder Pyrimidinyl bedeutet;
wobei die Gruppe(n) R" gegebenenfalls durch eine oder mehrere gleiche oder voneinander verschiedene Gruppen R' substituiert ist/sind;
R₄ ein Wasserstoffatom oder eine C₁-C₆-Alkylgruppe bedeutet und
R₆ und R₇ unabhängig voneinander eine C₁-C₆-Alkylgruppe bedeuten;
R eine Methyl- oder Ethylgruppe bedeutet.

9. Verbindung der allgemeinen Formel (V), 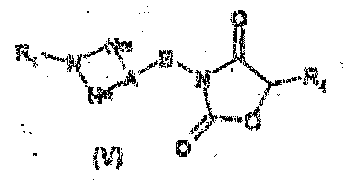 in der
A ein Stickstoffatom oder eine CR₂-Gruppe bedeutet, in der R₂ ein Wasserstoffatom, ein Fluoratom oder eine Hydroxyl-, Cyano-, Trifluormethyl-, C₁-C₆-Alkyl-, C₁-C₆-Alkoxygruppe bedeutet;
n eine ganze Zahl gleich 2 oder 3 bedeutet und m eine ganze Zahl gleich 2 bedeutet, wenn A ein Stickstoffatom bedeutet;
n eine ganze Zahl gleich 1, 2 oder 3 bedeutet und m eine ganze Zahl gleich 1 oder 2 bedeutet, wenn A eine CR₂-Gruppe bedeutet;
B eine kovalente Bindung oder eine C₁-C₈-Alkylengruppe bedeutet;
R₁ eine Gruppe ausgewählt aus Phenyl, Pyridinyl, Pyridazinyl, Pyrimidinyl, Pyrazinyl, Triazinyl, Oxazolyl, Isoxazolyl, Thiazolyl, Isothiazolyl, Imidazolyl, Oxadiazolyl, Thiadiazolyl, Triazolyl, Naphthyl, Chinolinyl, Tetrahydrochinolinyl, Isochinolinyl, Tetrahydroisochinolinyl, Phthalazinyl, Chinazolinyl, Chinoxalinyl, Naphthyridinyl, Cinnolinyl, Imidazopyrimidinyl, Thienopyrimidinyl, Benzofuranyl, Benzothienyl, Benzimidazolyl, Benzoxazolyl, Benzisoxazolyl, Benzothiazolyl, Benzisothiazolyl, Indazolyl, Pyrrolopyridinyl, Furopyridinyl, Dihydrofuropyridinyl, Thienopyridinyl, Dihydrothienopyridinyl, Imidazopyridinyl, Pyrazolopyridinyl, Oxazolopyridinyl, Isoxazolopyridinyl, Thiazolopyridinyl bedeutet;
wobei die R₁-Gruppe gegebenenfalls durch einen oder mehrere Gruppen R' und/oder R'' substituiert ist;
R' ein Halogenatom oder eine Cyano-, Nitro-, Hydroxyl-, C₁-C₆-Alkyl-, C₁-C₆-Alkoxy-, C₁-C₆-Thioalkyl-, C₁-C₆-Fluoralkyl-, C₁-C₆-Fluoralkoxy-, C₁-C₆-Fluorthioalkyl-, C₃-C₇-Cycloalkyl-, C₃-C₇-Cycloalkyl-C₁-C₆-alkylen-, Azetidinyl-, Piperidinyl-, Pyrrolidinyl-, Morpholinyl-, Piperazinyl-, Azepinyl-, NH₂-, NHR₆-, NR₆R₇-, NR₆COR₇-, NR₆SO₂R₇-, COR₆-, CO₂R₆-, SO₂R₆-, SO₂NR₆R₇- oder -O-(C₁-C₆-Alkyen)-O-Gruppe bedeutet;
R'' Phenyl, Imidazolyl, Pyridinyl, Pyrazinyl, Pyridazinyl oder Pyrimidinyl bedeutet;
wobei die Gruppe(n) R" gegebenenfalls durch eine oder mehrere gleiche oder voneinander verschiedene Gruppen R' substituiert ist/sind;
R₄ ein Wasserstoffatom oder eine C₁-C₆-Alkylgruppe bedeutet und
R₆ und R₇ unabhängig voneinander eine C₁-C₆-Alkylgruppe bedeuten;
mit Ausnahme der folgenden Verbindungen:
* 3-[1-(4-Amino-6,7-dimethoxy-2-chinazolinyl)-4-piperidinyl]-2,4-oxazolidindion
* 3-[1-(4-Amino-6,7-dimethoxy-2-chinazolinyl)-4-piperidinyl]-5-methyl-2,4-oxazolidindion
* 3-[1-(4-Amino-6,7-dimethoxy-2-chinazolinyl)-4-piperidinyl]-5-ethyl-2,4-oxazolidindion
* 3-[1-(4-Amino-6,7-dimethoxy-2-chinazolinyl)-4-piperidinyl]-5-propyl-2,4-oxazolidindion
* 3-[1-(4-Amino-6,7-dimethoxy-2-chinazolinyl)-4-piperidinyl]-5-(1-methylethyl)-2,4-oxazolidindion.

10. Verbindung der Formel (I) nach einem der Ansprüche 1 bis 5 als pharmazeutisch unbedenkliche(s) Base, Säureadditionssalz, Hydrat oder Solvat zur Verwendung als Arzneimittel.

11. Pharmazeutische Zusammensetzung, enthaltend mindestens eine Verbindung der Formel (I) nach einem der Ansprüche 1 bis 5 als pharmazeutisch unbedenkliche(s) Base, Säureadditionssalz, Hydrat oder Solvat sowie gegebenenfalls einen oder mehrere pharmazeutisch unbedenkliche Grundstoffe.

12. Verwendung einer Verbindung der Formel (I) nach einem der Ansprüche 1 bis 5 als pharmazeutisch unbedenkliche(s) Base, Säureadditionssalz, Hydrat oder Solvat für die Herstellung eines Arzneimittels zur Vorbeugung oder Behandlung einer Pathologie, an der die endogenen Cannabinoide und/oder alle anderen von dem Enzym FAAH metabolisierten Substrate beteiligt sind.

13. Verwendung einer Verbindung der Formel (I) nach einem der Ansprüche 1 bis 5 als pharmazeutisch unbedenkliche(s) Base, Säureadditionssalz, Hydrat oder Solvat für die Herstellung eines Arzneimittels für die Vorbeugung oder Behandlung von akuten oder chronischen Schmerzen, Schwindelgefühl, Erbrechen, Übelkeit, Problemen mit dem Ernährungsverhalten, neurologischen und psychiatrischen Pathologien, akuten oder chronischen neurodegenerativen Erkrankungen, Epilepsie, Schlafstörungen, Herz-Kreislauf-Krankheiten, renaler Ischämie, Krebs, Störungen des Immunsystems, Allergien, parasitären, viralen oder bakteriellen Infektionskrankheiten, Entzündungen, Osteoporose, Augenbeschwerden, Lungenbeschwerden, Magen-Darm-Krankheiten oder Harninkontinenz.
